(19) Europäisches Patentamt
European Patent Office
Office européen des brevets

(11) EP 1 082 127 B1

(12) **EUROPEAN PATENT SPECIFICATION**

(45) Date of publication and mention of the grant of the patent:
**22.06.2005 Bulletin 2005/25**

(21) Application number: **99918930.1**

(22) Date of filing: **30.04.1999**

(51) Int Cl.[7]: **A61K 38/05**, C07D 211/14, C07D 265/28, A61P 29/00

(86) International application number:
**PCT/US1999/009463**

(87) International publication number:
**WO 1999/056765 (11.11.1999 Gazette 1999/45)**

# (54) SUCCINAMIDE INHIBITORS OF INTERLEUKIN-1BETA CONVERTING ENZYME

SUCCINAMID HEMMSTOFFE DES INTERLEUKIN-1 BETA KONVERTIERENDEN ENZYMS

INHIBITEURS SUCCINAMIDES DE L'ENZYME DE CONVERSION DE L'INTERLEUKINE 1BETA

(84) Designated Contracting States:
**AT BE CH CY DE DK ES FI FR GB GR IE IT LI LU MC NL PT SE**
Designated Extension States:
**AL LT LV MK RO SI**

(30) Priority: **05.05.1998 US 84320 P**

(43) Date of publication of application:
**14.03.2001 Bulletin 2001/11**

(73) Proprietor: **WARNER-LAMBERT COMPANY LLC**
**Morris Plains, NJ 07950 (US)**

(72) Inventors:
- **CAPRATHE, Bradley, William**
  **Livonia, MI 48154 (US)**
- **GILMORE, John, Lodge**
  **Wilmington, DE 19809 (US)**
- **HARTER, William, Glen**
  **Chelsea, MI 48118 (US)**
- **HAYS, Sheryl, Jeanne**
  **F-75006 Paris (FR)**
- **KNAPP, Kristen, Michele**
  **Ypsilanti, MI 48197 (US)**
- **KOSTLAN, Catherine, Rose**
  **Saline, MI 48176 (US)**
- **LUNNEY, Elizabeth, Ann**
  **Ann Arbor, MI 48103 (US)**
- **PARA, Kimberly, Suzanne**
  **Ann Arbor, MI 48103 (US)**
- **GALATSIS, Paul**
  **Ann Arbor, MI 48103 (US)**
- **THOMAS, Anthony, Jerome**
  **Ann Arbor, MI 48104 (US)**

(74) Representative: **Kinzebach, Werner, Dr. et al**
**Patentanwälte**
**Reitstötter, Kinzebach und Partner**
**Postfach 86 06 49**
**81633 München (DE)**

(56) References cited:
**US-A- 5 559 232**

- **DANG et al., "Preparation of an Autolysis-Resistant Interleukin-1BETA Converting Enzyme Mutant", BIOCHEMISTRY, 1996, Vol. 35, No. 47, pages 14910-14916, XP002921664.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 082 127 B1

## Description

FIELD OF THE INVENTION

This invention relates to a series of succinamide compounds that inhibit the interleukin-1β converting enzyme. This invention also relates to pharmaceutically acceptable compositions that contain a compound that is an inhibitor of interleukin-1β converting enzyme and a method of using the compositions for the treatment of stroke, inflammatory diseases, septic shock, reperfusion injury, Alzheimer's disease, and shigellosis.

BACKGROUND OF THE INVENTION

Interleukin 1β-protease (also known as interleukin-1β converting enzyme, ICE or Caspase 1) acts on pro-interleukin-1β (pro-IL-1β) to produce interleukin-1β (IL-1β), which is an inflammatory cytokine (Kostura M.J. et al., *Proc. Nat. Acad. Sci*. 1989;86:5227-5231 and Black R.A. et al., *FEBS Let*. 1989;247:386-391). Several diseases are associated with interleukin-1 activity. Examples of diseases in which interleukin-1 is involved include inflammatory diseases such as rheumatoid arthritis and inflammatory bowel disease, and neuroinflammatory disorders such as stroke, multiple sclerosis, and Alzheimer's disease (Dinarello C.A., *Eur. Cytokine Netw.,* 1994;5:517). Other diseases include septic shock, reperfusion injury, and shigellosis.

Agents that modulate IL-1β activity have been shown to have beneficial *in vivo* effects. For example, compounds that are interleukin-1 receptor antagonists have been shown to inhibit ischaemic and excitotoxic damage in rat brains (e.g., Relton J.K. et al., *Brain Research Bulletin* 1992;29:243-246). Additionally, ICE inhibitors were shown to reduce inflammation and pyrexia in rats (Elford P.R. et al., *British Journal of Pharmacology* 1995;115:601-606).

Inhibitors of ICE can also inhibit other cysteine proteases in the ICE family. Recently, the nomenclature of these ICE family cysteine proteases (also known as Caspases with ICE being known as Caspase-1) has been further defined. The following proteases are representative members of this class of enzymes using the nomenclature described in Alnemri et al., *Cell* 1996;87:171: Caspase-2 (also known as Ich-1); Caspase-3 (also known as CPP32, Yama, and apopain); Caspase-4 (also known as TX, Ich-2, and ICE rel-II); Caspase-5 (also known as ICE rel-III); Caspase-6 (also known as Mch2); Caspase-7 (also known as Mch3); Caspase-8 (also known as FLICE and Mch5); Caspase-9 (also known as ICE-LAP6 and Mch6); Caspase-10 (also known as Mch4). It is recognized that members of this enzyme family play key biological roles in both inflammation and apoptosis (programmed cell death) (Thornberry N.A. et al., *Perspectives in Drug Discovery and Design* 1994;2:389-399).

In addition to its effects on IL-1β production, ICE has been shown to play a role in the production of the inflammatory mediator interferon-γ (Ghayur et al., *Nature* 1997;386(6625):619-623). ICE processes the inactive proform of interferon-γ inducing factor (IGIF; Interleukin-18) to active IGIF, a protein that induces production of interferon-γ by T-cells and natural killer cells. Interferon-γ has been implicated in the pathogenesis of diseases such as inflammatory disorders and septic shock. Therefore, ICE inhibitors would be expected to have beneficial effects in such disease states by effects on interferon-γ.

The majority of the ICE inhibitors described in the art are peptide-based (e.g., Dolle R. et al., *J. Med. Chem*. 1994;37:563. However, the use of pyridone or pyrimidone based peptidomimetic inhibitors has recently been reported (Dolle R. et al., WO 9526958, 1995; Dolle R. et al., *J. Med. Chem.* 1996;39:2438; and Semple G. et al., *Bioorg. Med. Chem. Lett.* 1997;7:1337). X-ray crystallography and molecular modeling have shown that pyridone based inhibitors are suitable replacements for $P_2$-$P_3$ found in peptide based inhibitors (Golec J. et al., *Bioorg. Med. Chem. Lett.* 1997; 7:2181-2186). US 5 559 232 discloses carboxamides with a similar but different Markush formula, for use as platelet aggregation inhibitors.

WO 9526958 discloses the use of pyrimidone based inhibitors of ICE. As well as possessing some activity in *in vitro* models, the compounds taught in WO 9526958 are reported to have an *in vivo* $IC_{50}$ range of from 0.1 to 10 μm, reflecting the percent inhibition of release of IL-1β. While these values reflect some activity, the search for better ICE inhibitors for the treatment of such diseases as inflammation, Alzheimer's disease, stroke, and septic shock is desired.

SUMMARY OF THE INVENTION

The present invention provides compounds of the Formula I

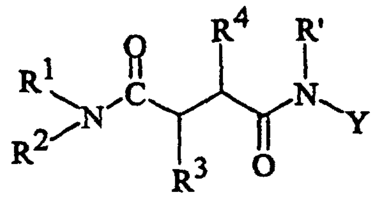

I

wherein
Y is

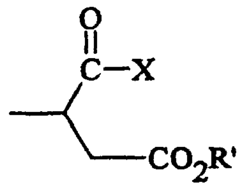

,

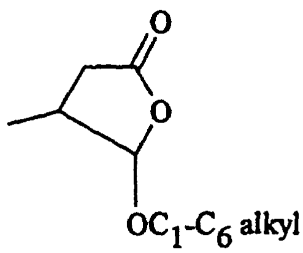

,

or

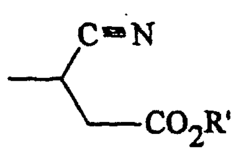

;

each R' is independently hydrogen or $C_1$-$C_6$ alkyl;
$R^1$ and $R^2$ are independently hydrogen, $C_1$-$C_6$ alkyl,

-OH, $-(CH_2)_n$ aryl,
$-(CH_2)_n$-substituted aryl,
$-(CH_2)_n$-O-aryl,
$-(CH_2)_n$-O-substituted aryl,
$-(CH_2)_n$-S-aryl,
$-(CH_2)_n$-S-substituted aryl,
$-(CH_2)_n$-S-heteroaryl,
$-(CH_2)_n$-S-substituted heteroaryl,
$-(CH_2)_n$-NR'-aryl,
$-(CH_2)_n$-NR'-substituted aryl,
$-(CH_2)_n$-NR'-heteroaryl,
$-(CH_2)_n$-NR'-substituted heteroaryl,
$-(CH_2)_n$-heteroaryl, or
$-(CH_2)_n$-substituted heteroaryl;

each n is independently 0 to 6;

$R^3$ is hydrogen or $C_1$-$C_6$ alkyl;
$R^4$ is $C_1$-$C_6$ alkyl or hydrogen; and
X is hydrogen,

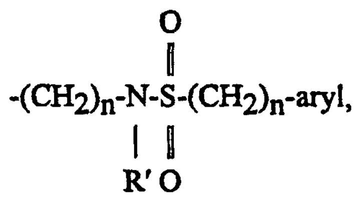

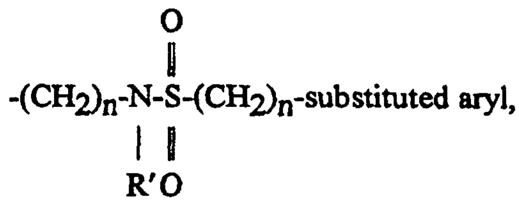

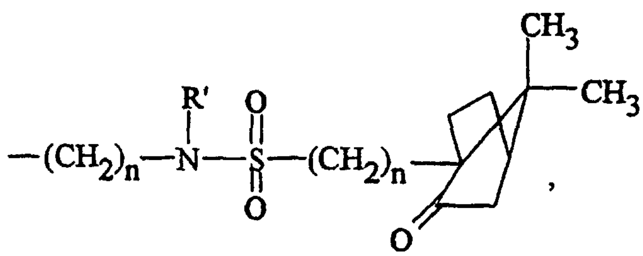

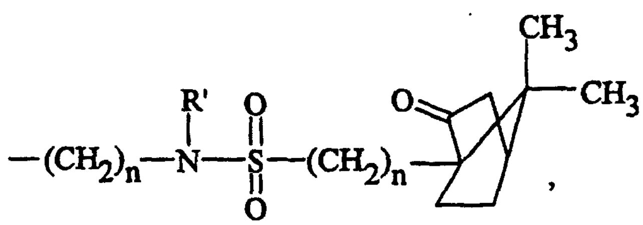

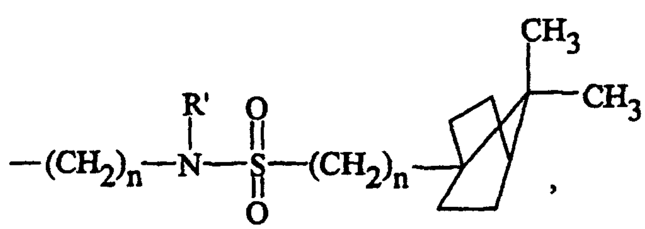

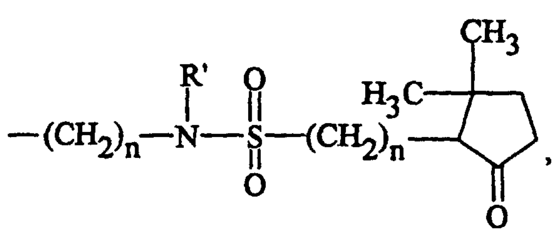

$-(CH_2)_n-S-(CH_2)_n$-aryl,

$-(CH_2)_n-S-(CH_2)_n$-substituted aryl,

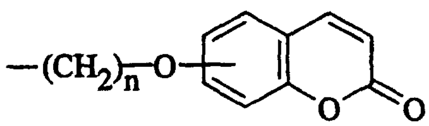

,

or

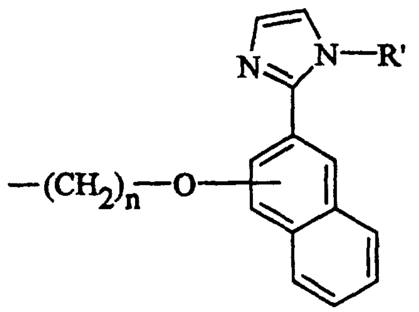

;

and the pharmaceutically acceptable salts thereof.

In a preferred embodiment of the compounds of Formula I, R' is hydrogen or methyl.

In another preferred embodiment,
each R' is hydrogen.

In another preferred embodiment,
$R^3$ is hydrogen, and
$R^4$ is methyl, ethyl, or isopropyl.

In another preferred embodiment,
$R^1$ is hydrogen or methyl; and
$R^2$ is

$-(CH_2)_n$-phenyl,
hydrogen,
$-(CH_2)_n$-O-phenyl
-OH,
$-(CH_2)_n$-benzimidazoyl,
$-(CH_2)_n$-indolyl, or
$-(CH_2)_n$-phenol.

In another preferred embodiment, Y is

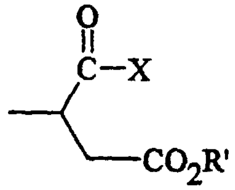

.

In still another preferred embodiment, wherein
Y is

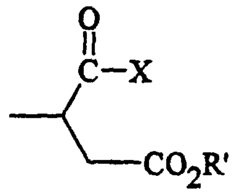,

X is hydrogen,

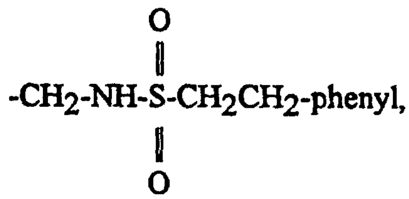,

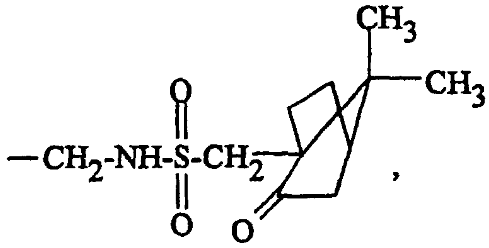,

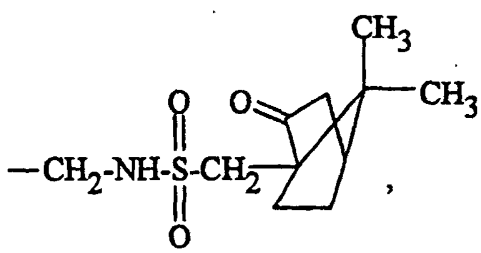,

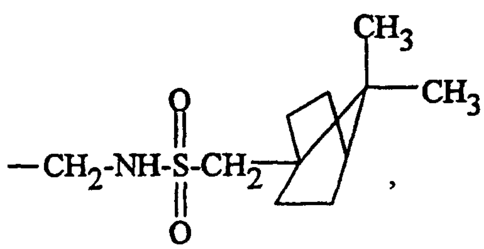,

$-CH_2-S-CH_2CH_2CH_2$-phenyl,

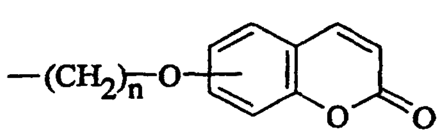,

or

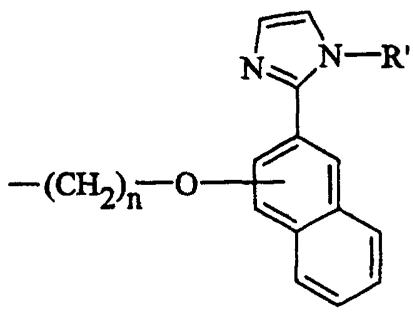
.

In a more preferred embodiment, the present invention provides compounds of the Formula I

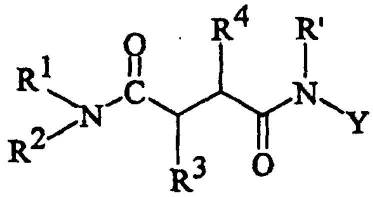
I

wherein
Y is

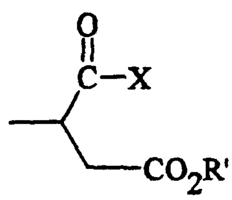
,

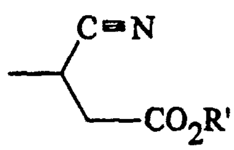
,

or

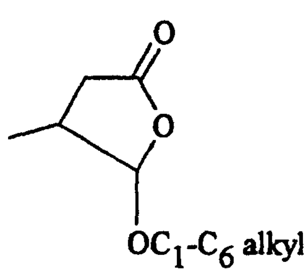
;

each R' is independently hydrogen or methyl;
each n is independently 2 to 3;
$R^1$ and $R^2$ are independently hydrogen,

$-(CH_2)_n$-phenyl,

$-(CH_2)_n$-O-phenyl,

-OH,

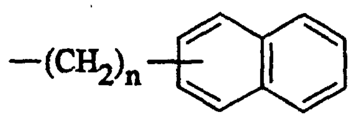
,

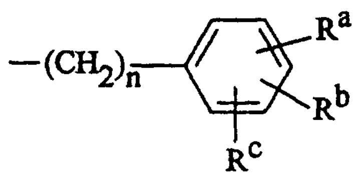
,

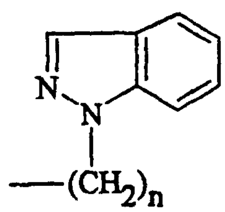
,

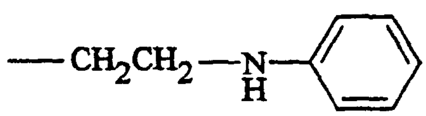
,

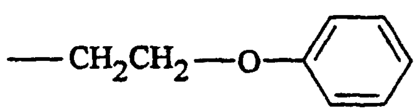
,

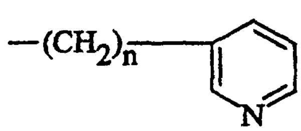
,

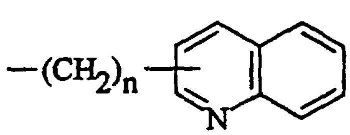
,

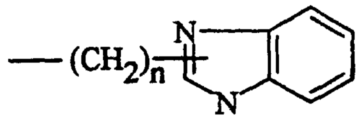,

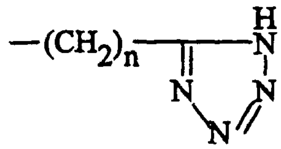,

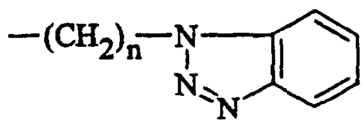,

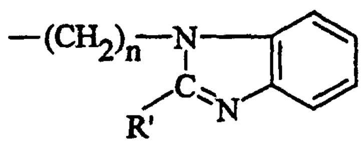,

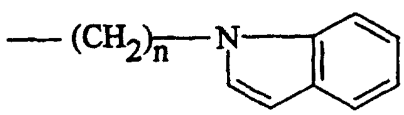,

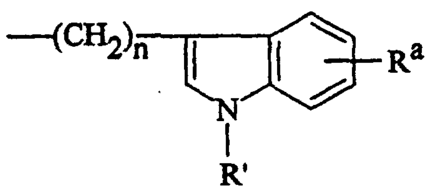,

or

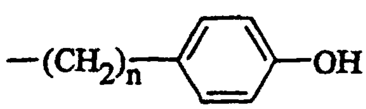;

$R^a$, $R^b$, and $R^c$ are independently halogen, $-OC_1$-$C_6$ alkyl or hydrogen;
$R^3$ is hydrogen;
$R^4$ is methyl, ethyl, or isopropyl; and
X is hydrogen,

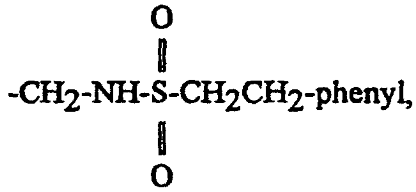

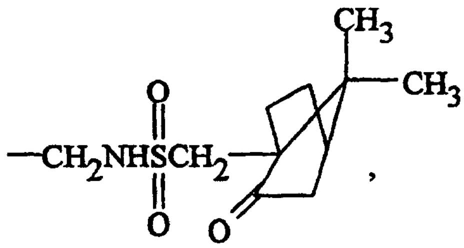
,

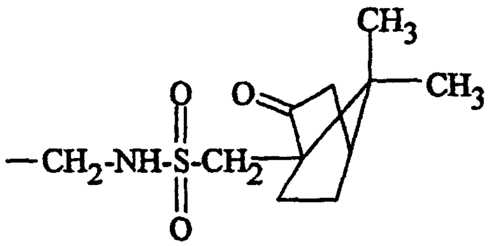
,

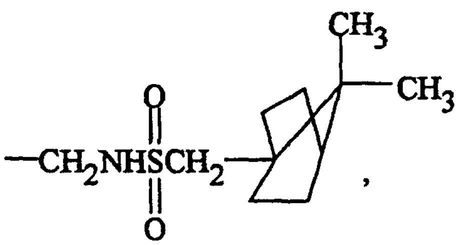
,

$-CH_2-S-CH_2CH_2CH_2$-phenyl,

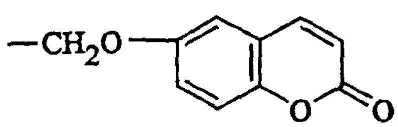
,

or

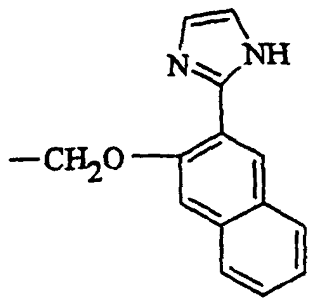

;

and the pharmaceutically acceptable salts thereof.

In a preferred embodiment of Formula I,
$R^1$ is hydrogen and
$R^2$ is $-(CH_2)_n$-indolyl,

$-(CH_2)_n$-substituted indolyl,
$-(CH_2)_n$-NH-phenyl,
$-(CH_2)_n$-O-phenyl,
$-(CH_2)_n$-tetrazolyl,
$-(CH_2)_n$-phenyl,
$-(CH_2)_n$-substituted phenyl,
$-(CH_2)_n$-substituted benzimidazolyl,
$-(CH_2)_n$-benztriazolyl,
$-(CH_2)_n$-indazolyl,
$-(CH_2)_n$-benzimidazolyl,
$-(CH_2)_n$-pyridyl,
$-(CH_2)_n$-naphthyl,
or $-(CH_2)_n$-quinolinyl.

In a most preferred embodiment, the present invention provides the compounds:

3-(2-Methyl-3-phenethylcarbamoyl-propionylamino)-4-oxo-5-(2-phenylethanesulfonylamino)-pentanoic acid;
3-(2-Carbamoylmethyl-3-methyl-butyrylamino)-5-(7,7-dimethyl-2-oxo-bicyclo[2.2.1]hept-1-ylmethanesulfonylamino)-4-oxo-pentanoic acid;
5-(7,7-Dimethyl-2-oxo-bicyclo[2.2.1]hept-1-ylmethanesulfonylamino)-3-[3-methyl-2-(phenethylcarbamoyl-methyl)-butyrylamino]-4-oxo-pentanoic acid;
3-(2-Carbamoylmethyl-3-methyl-butyrylamino)-4-oxo-5-(2-phenylethanesulfonyl-amino)-pentanoic acid;
3-[3-Methyl-2-(phenethylcarbamoyl-methyl)-butyrylamino]-4-oxo-5-(2-phenyl-ethanesulfonylamino)-pentanoic acid;
5-(7,7-Dimethyl-bicyclo[2.2.1]hept-1-ylmethanesulfonylamino)-3-[3-methyl-2-(phenethylcarbamoyl-methyl)-butyrylamino]-4-oxo-pentanoic acid;
(S,S)-3-{3-Methyl-2-[(3-phenyl-propylcarbamoyl)-methyl]-butyrylamino}-4-oxo-butyric acid;
3-{3-Methyl-2-[(3-phenoxy-ethylcarbamoyl)-methyl]-butyrylamino}-4-oxo-butyric acid;
5-(7,7-Dimethyl-2-oxo-bicyclo[2.2.1]hept-1-ylmethanesulfonylamino)-3-{3-methyl-2-[(2-phenoxy-ethylcarbamoyl)-methyl]-butyrylamino}-4-oxo-pentanoic acid;
3-[3-Methyl-2-(phenethylcarbamoyl-methyl)-butyrylamino]-4-oxo-5-(3-phenyl-propylsulfanyl)-pentanoic acid;
5-(7,7-Dimethyl-2-oxo-bicyclo[2.2.1]hept-1-ylmethanesulfonylamino)-3-{3-methyl-2-[(methyl-phenethyl-carbamoyl)-methyl]-butyrylamino}-4-oxopentanoic acid;
5-(7,7-Dimethyl-2-oxo-bicyclo[2.2.1]hept-1-ylmethanesulfonylamino)-3-{3-methyl-2-[(3-phenyl-propylcarbamoyl)-methyl]-butyrylamino}-4-oxo-pentanoic acid;
3-{3-Methyl-2-[(3-phenyl-propylcarbamoyl)-methyl]-butyrylamino}-4-oxo-5-(2-oxo-2H-chromen-6-yloxy)-pentanoic acid;
5-[3-(1H-Imidazol-2-yl)-naphthalen-2-yloxy]-3-{3-methyl-2-[(3-phenyl-propylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-(2-hydroxycarbamoylmethyl-3-methyl-butyrylamino)-4-oxo-pentanoic acid;

5-(7,7-Dimethyl-2-oxo-bicyclo[2.2.1]hept-1-ylmethanesulfonylamino)-3-(2-{[2-(1H-indol-3-yl)-ethylcarbamoyl]-methyl}-3-methyl-butyrylamino)-4-oxo-pentanoic acid;
5-(7,7-Dimethyl-2-oxo-bicyclo[2.2.1]hept-1-ylmethanesulfonylamino)-3-{3-methyl-2-[[3-(4-hydroxyphenyl)-propylcarbamoyl]-methyl]-butyrylamino}-4-oxo-pentanoic acid;
5-(7,7-Dimethyl-2-oxo-bicyclo[2.2.1]hept-1-ylmethanesulfonylamino)-3-(2-{[2-(1H-indol-3-yl)-ethylcarbamoyl]-methyl}-3-methyl-butyrylamino)-4-oxo-pentanoic acid;
5-(7,7-Dimethyl-2-oxo-bicyclo[2.2.1]hept-1-ylmethanesulfonylamino)-3-(3-methyl-2-{[2-(1-methyl-1H-indol-3-yl)-ethylcarbamoyl]-methyl}-butyrylamino)-4-oxo-pentanoic acid;
5-(7,7-Dimethyl-2-oxo-bicyclo[2.2.1]hept-1-ylmethanesulfonylamino)-3-(3-methyl-2-{[2-(7-methyl-1H-indol-3-yl)-ethylcarbamoyl]-methyl}-butyrylamino)-4-oxo-pentanoic acid;
5-(7,7-Dimethyl-2-oxo-bicyclo[2.2.1]hept-1-ylmethanesulfonylamino)-3-(2-{[2-(6-fluoro-1H-indol-3-yl)-ethylcarbamoyl]-methyl}-3-methyl-butyrylamino)-4-oxo-pentanoic acid;
5-(7,7-Dimethyl-2-oxo-bicyclo[2.2.1]hept-1-ylmethanesulfonylamino)-3-[3-methyl-2-({methyl-[2-(1-methyl-1H-indol-3-yl)-ethyl]-carbamoyl}-methyl)-butyrylamino]-4-oxo-pentanoic acid;
3-{2-[(2-Benzoimidazol-1-yl-ethylcarbamoyl)-methyl]-3-methyl-butyrylamino}-5-(7,7-dimethyl-2-oxo-bicyclo [2.2.1]hept-1-ylmethanesulfonylamino)-4-oxo-pentanoic acid;
5-(7,7-Dimethyl-2-oxo-bicyclo[2.2.1]hept-1-ylmethanesulfonylamino)-3-(3-methyl-2-{[2-(1H-tetrazol-5-yl)-ethylcarbamoyl]-methyl}-butyrylamino)-4-oxo-pentanoic acid;
5-(7,7-Dimethyl-2-oxo-bicyclo[2.2.1]hept-1-ylmethanesulfonylamino)-4-oxo-3-{2-[(3-phenyl-propylearbamoyl)-methyl]-butyrylamino}-pentanoic acid;
5-(7,7-Dimethyl-2-oxo-bicyclo[2.2.1]hept-1-ylmethanesulfonylamino)-3-(2-{[2-(1-methyl-1H-indol-3-yl)-ethylcarbamoyl]-methyl}-butyrylamino)-4-oxo-pentanoic acid;
3-(3-Methyl-2-{[2-(1-methyl-1H-indol-3-yl)-ethylcarbamoyl]methyl}-butyrylamino)-4-oxo-butyric acid;
3-[3-Methyl-2-({methyl-[2-(1-methyl-1H-indol-3-yl)-ethyl]-carbamoyl}-methyl)-butyrylamino]-4-oxo-butyric acid;
3-{2-[(2-Benzoimidazol-1-yl-ethylcarbamoyl)-methyl]-3-methyl-butyrylamino}-4-oxo-butyric acid;
5-(7,7-Dimethyl-2-oxo-bicyclo[2.2.1]hept-1-ylmethanesulfonylamino)-3-(2-{[3-(4-hydroxy-phenyl)-propylcarbamoyl]-methyl}-3-methyl-butyrylamino)-4-oxo-pentanoic acid;
5-(7,7-Dimethyl-2-oxo-bicyclo[2.2.1]hept-1-ylmethanesulfonylamino)-3-{3-methyl-2-[(2-pyridin-4-yl-ethylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(2-naphthalen-2-yl-ethylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(3-pyridin-4-yl-propylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(3-quinolin-2-yl-propylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(3-naphthalen-2-yl-propylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(3-pyridin-3-yl-propylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(2-naphthalen-2-yl-ethylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-(3-methyl-2-{[2-(7-methyl-1H-indol-3-yl)-ethylcarbamoyl]-methyl}-butyrylamino)-4-oxo-pentanoic acid;
5-(7,7-Dimethyl-2-oxo-bicyclo[2.2.1]hept-1-ylmethanesulfonylamino)-3-(2-{[2-(6-fluoro-1H-indol-3-yl)-ethylcarbamoyl]-methyl}-3-methyl-butyrylamino)-4-oxo-pentanoic acid;
3-[3-Methyl-2-({methyl-[2-(1-methyl-1H-indol-3-yl)-ethyl]-carbamoyl}-methyl)-butyrylamino]-4-oxo-butyric acid;
3-(3-Methyl-2-{[methyl-(2-phenoxy-ethyl)-carbamoyl]-methyl}-butyrylamino)-4-oxo-butyric acid;
3-(2-{[2-(5,6-Dimethyl-benzoimidazol-1-yl)-ethylcarbamoyl]-methyl}-3-methyl-butyrylamino)-4-oxo-butyric acid, trifluoroacetate salt;
5-(7,7-Dimethyl-bicyclo[2.2.1]hept-1-ylmethanesulfonylamino)-3-{3-methyl-2-[(3-phenyl-propylcarbamoyl)-methyl]-butyrylamino}-4-oxo-pentanoic acid;
5-(7,7-Dimethyl-bicyclo[2.2.1]hept-1-ylmethanesulfonylamino)-3-(2-{[2-(1H-indol-3-yl)-ethylcarbamoyl]-methyl}-3-methyl-butyrylamino)-4-oxo-pentanoic acid;
5-(7,7-Dimethyl-2-oxo-bicyclo[2.2.1]hept-1-ylmethanesulfonylamino)-3-{3-methyl-2-[(3-pyridin-4-yl-propylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(3-quinolin-2-yl-propylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(3-naphthalen-1-yl-propylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(3-pyridin-3-yl-propylcarbamoyl)-methyl]-butyrylamino}-4-oxo-pentanoic acid;
3-{2-[(2-Benzoimidazol-1-yl-ethylcarbamoyl)-methyl]-3-methyl-butyrylamino}-5-(7,7-dimethyl-2-oxo-bicyclo[2.2.1]hept-1-ylmethanesulfonylamino)-4-oxo-pentanoic acid;
5-(7,7-Dimethyl-2-oxo-bicyclo[2.2.1]hept-1-ylmethanesulfonylamino)-3-(3-methyl-2-{[2-(1-methyl-1H-indol-3-yl)-ethylcarbamoyl]-methyl}-butyrylamino)-4-oxo-pentanoic acid;
5-(7,7-Dimethyl-2-oxo-bicyclo[2.2.1]hept-1-ylmethanesulfonylamino)-3-{3-methyl-2-[(2-pyridin-4-yl-ethylcarbamoyl)-methyl]-butyrylamino}-4-oxo-pentanoic acid;
3-(2-{[2-(5-Acetyl-1H-indol-3-yl)-ethylcarbamoyl]-methyl}-3-methyl-butyrylamino)-5-(7,7-dimethyl-2-oxo-bicyclo[2.2.1]hept-1-ylmethanesulfonylamino)-4-oxo-pentanoic acid;
5-(7,7-Dimethyl-2-oxo-bicyclo[2.2.1]hept-1-ylmethanesulfonylamino)-3-(3-methyl-2-{[2-(1H-tetrazol-5-yl)-ethylcarbamoyl]-methyl}-butyrylamino)-4-oxo-pentanoic acid;
$N^4$-(2-Benzoimidazol-1-yl-ethyl)-$N^1$-(2-ethoxy-5-oxo-tetrahydro-furan-3-yl)-2-isopropyl-succinamide;
$N^1$-(2-Ethoxy-5-oxo-tetrahydro-furan-3-yl)-$N^4$-[2-(1H-indol-3-yl)-ethyl]-2-isopropyl-succinamide;
$N^1$-(2-Ethoxy-5-oxo-tetrahydro-furan-3-yl)-2-isopropyl-$N^4$-[2-(1-methyl-1H-indol-3-yl)-ethyl]-succinamide;
$N^4$-[2-(5,6-Dichloro-benzoimidazol-1-yl)-ethyl]-$N^1$-(2-ethoxy-5-oxo-tetrahydro-furan-3-yl)-2-isopropyl-succinamide;
$N^1$-(2-Ethoxy-5-oxo-tetrahydro-furan-3-yl)-2-isopropyl-$N^4$-(2-phenoxy-ethyl)-succinamide;
$N^1$-(2-Ethoxy-5-oxo-tetrahydro-furan-3-yl)-2-isopropyl-$N^4$-[2-(6-methoxy-1H-indol-3-yl)-ethyl]-succinamide;
$N^4$-(2-Benzotriazol-1-yl-ethyl)-$N^1$-(2-ethoxy-5-oxo-tetrahydro-furan-3-yl)-2-isopropyl-succinamide;
$N^1$-(2-Ethoxy-5-oxo-tetrahydro-furan-3-yl)-$N^4$-(2-indazol-1-yl-ethyl)-2-isopropyl-succinamide;
$N^1$-(2-Ethoxy-5-oxo-tetrahydro-furan-3-yl)-2-isopropyl-$N^4$-(2-phenylamino-ethyl)-succinamide;
$N^1$-(2-Ethoxy-5-oxo-tetrahydro-furan-3-yl)-$N^4$-[2-(6-fluoro-1H-indol-3-yl)-ethyl]-2-isopropyl-succinamide;
$N^1$-(2-Ethoxy-5-oxo-tetrahydro-furan-3-yl)-$N^4$-[2-(7-methyl-1H-indol-3-yl)-ethyl]-2-isopropyl-succinamide;
$N^1$-(2-ethoxy-5-oxotetrahydro-furan-3-yl)-2-isopropyl-$N^4$-[2-(2-methyl-benzoimidazol-1-yl)-ethyl]-succinamide;
$N^1$-(2-Ethoxy-5-oxo-tetrahydro-furan-3-yl)-2-isopropyl-$N^4$-[3-(3,4,5-trimethoxy-phenyl)-propyl]-succinamide;
$N^1$-(2-Ethoxy-5-oxo-tetrahydro-furan-3-yl)-2-isopropyl-$N^4$-[2-(phenyl)-ethyl]-succinamide;
$N^1$-(2-Ethoxy-5-oxo-tetrahydro-furan-3-yl)-2-isopropyl-$N^4$-[4-(phenyl)-butyl]-succinamide;
$N^1$-(2-Ethoxy-5-oxo-tetrahydro-furan-3-yl)-$N^4$-(2-indol-1-yl-ethyl)-2-isopropyl-succinamide;
$N^1$-(2-Ethoxy-5-oxo-tetrahydro-furan-3-yl)-2-isopropyl-$N^4$-[4-(phenyl)-propyl]-succinamide;
$N^1$-(2-Ethoxy-5-oxo-tetrahydro-furan-3-yl)-$N^4$-[2-(5-fluoro-1H-indol-3-yl)-ethyl]-2-isopropyl-succinamide;
3-{2-[(2-Benzoimidazol-1-yl-ethylcarbamoyl)-methyl]-3-methyl-butyrylamino}-4-oxo-butyric acid;
3-(3-Methyl-2-{[2-(1-methyl-1H-indol-3-yl)-ethylcarbamoyl]-methyl}-butyrylamino)-4-oxo-butyric acid;
3-(2-{[2-(5-Fluoro-1-methyl-1H-indol-3-yl)-ethylcarbamoyl]-methyl}-3-methyl-butyrylamino)-4-oxo-butyric acid;
3-(2-{[2-(5,6-Dichloro-benzoimidazol-1-yl)-ethylcarbamoyl]-methyl}-3-methyl-butyrylamino)-4-oxo-butyric acid;
3-(2-{[(2-Benzoimidazol-1-yl-ethyl)-methyl-carbamoyl]-methyl}-3-methyl-butyrylamino)-4-oxo-butyric acid;
3-{2-[(2-Benzotriazol-1-yl-ethylcarbamoyl)-methyl]-3-methyl-butyrylamino}-4-oxo-butyric acid;
3-{2-[(2-Indazol-1-yl-ethylcarbamoyl)-methyl]-3-methyl-butyrylamino}-4-oxo-butyric acid;
3-[2-({[2-(5,6-Dimethyl-benzoimidazol-1-yl)-ethyl]-methylcarbamoyl}-methyl)-3-methyl-butyrylamino]4-oxo-butyric acid;
3-[2-({[2-(2-Methyl-benzoimidazol-1-yl)-ethyl]-methylcarbamoyl}-methyl)-3-methyl-butyrylamino]4-oxo-butyric acid;
3-(3-methyl-2-{[3-(3,4,5-trimethoxy-pheny)-propylcarbamoyl]-methyl}-butyrylamino)-4-oxo-butyric acid;
3-{3-Methyl-2-[(2-phenoxy-ethylcarbamoyl)-methyl]-butyrylamino}-4-oxo-butyric acid ethyl ester;
3-Cyano-3-{3-methyl-2-[(2-phenoxy-ethylcarbamoyl)-methyl]-butyrylamino}-propionic acid ethyl ester; and
3-Cyano-3-{3-methyl-2-[(2-phenoxy-ethylcarbamoyl)-methyl]-butyrylamino}-propionic acid.

Also provided is a pharmaceutical composition that comprises a compound of Formula I.
Also provided is a method of manufacture of a medicament for treating or preventing stroke, where the composition is to be administered to a patient having, having had, or at risk of having a stroke a therapeutically effective amount of a compound of Formula I.
Also provided is a method of manufacture of a medicament for treating inflammatory diseases, where the composition is to be administered to a patient having an inflammatory disease a therapeutically effective amount of a compound of Formula I.
In a preferred embodiment of the method of manufacture of a medicament for treating inflammatory diseases, the inflammatory disease is rheumatoid arthritis or inflammatory bowel disease.
Also provided is a method of manufacture of a medicament for treating septic shock, where the composition

is to be administered to a patient having septic shock a therapeutically effective amount of a compound of Formula I.

Also provided is a method of manufacture of a medicament for treating reperfusion injury, where the composition is to be administered to a patient having reperfusion injury a therapeutically effective amount of a compound of Formula I.

Also provided is a method of manufacture of a medicament for treating Alzheimer's disease, where the composition is to be administered to a patient having Alzheimer's disease a therapeutically effective amount of a compound of Formula I.

Also provided is a method of manufacture of a medicament for treating shigellosis, where the composition is to be administered to a patient having shigellosis a therapeutically effective amount of a compound of Formula I.

Also provided is a method of manufacture of a medicament for treating multiple sclerosis, where the composition is to be administered to to a patient having multiple sclerosis a therapeutically effective amount of a compound of Formula I.

Also provided is a method of manufacture of a medicament for inhibiting interleukin-1β converting enzyme, where the composition is to be administered to a patient in need of interleukin-1β converting enzyme inhibition a therapeutically effective amount of a compound of Formula I.

DETAILED DESCRIPTION OF THE INVENTION

The term "alkyl" means a straight or branched chain hydrocarbon. Representative examples of alkyl groups are methyl, ethyl, propyl, isopropyl, isobutyl, butyl, tert-butyl, sec-butyl, pentyl, and hexyl. Preferred alkyls are $C_1$-$C_6$ alkyl.

The term "alkoxy" means an alkyl group attached to an oxygen atom. Representative examples of alkoxy groups include methoxy, ethoxy, tert-butoxy, propoxy, and isobutoxy.

The term "halogen" includes chlorine, fluorine, bromine, and iodine.

The term "alkenyl" means a branched or straight chain hydrocarbon having one or more carbon-carbon double bond.

The term "alkynyl" means a branched or straight chain hydrocarbon having one or more carbon-carbon triple bond.

The term "aryl" means an aromatic hydrocarbon. Representative examples of aryl groups include phenyl and naphthyl.

The term "heteroatom" includes oxygen, nitrogen, and sulfur.

The term "heteroaryl" means an aryl group wherein one or more carbon atom of the aromatic hydrocarbon has been replaced with a heteroatom. Examples of heteroaryl radicals include, but are not limited to, pyridyl, imidazolyl, pyrrolyl, benzimidazolyl, tetrazolyl, benzotriazolyl, indazolyl, thienyl, furyl, pyranyl, pyrimidinyl, pyridazinyl, indolyl, quinolyl, naphthyridinyl, and isoxazolyl.

The term "cycloalkyl" means a cyclic hydrocarbon. Examples of cycloalkyl groups include cyclopropyl, cyclobutyl, cyclopentyl, and cyclohexyl.

The symbol "-" means a bond.

The term "patient" means all animals including humans. Examples of patients include humans, cows, dogs, cats, goats, sheep, and pigs.

The term "substituted" means that the base organic radical has one or more substituents. For example, substituted cyclohexyl means a cyclohexyl radical that has one or more substituents. Substituents include, but are not limited to, halogen, $-CF_3$, $C_1$-$C_8$ alkyl, -CN, $CF_3$, $-NO_2$,

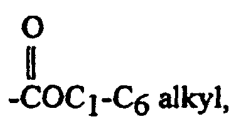 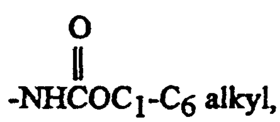

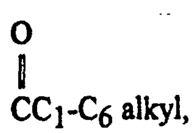

$-NH_2$, -O-phenyl, $-NHC_1$-$C_8$ alkyl, $-N(C_1$-$C_8$ alkyl$)_2$, $-SC_1$-$C_6$ alkyl, $-OC_1$-$C_8$ alkyl, and -OH. Particularly preferred substituents include, but are not limited to tert-butyl, methyl, -OH, $-NH_2$, $-SCH_3$, -CN, $-OCH_3$,

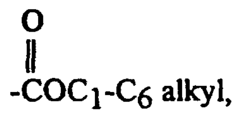

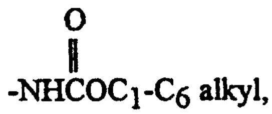

bromine, fluorine, and chlorine.

The term "cycloalkenyl" means a cycloalkyl group having at least one carbon-carbon double bond. Examples of cycloalkenyl groups include cyclopentene, cyclobutene, and cyclohexene.

The term "heterocycle" or "heterocycloalkyl" means a cycloalkyl group wherein one or more carbon atom is replaced with a heteroatom. Examples of heterocycles include, but are not limited to, pyrrolidinyl, piperidinyl, and piperazinyl.

The compounds of Formula I can be administered to a patient either alone or as part of a pharmaceutically acceptable composition. The compositions can be administered to patients such as humans and animals either orally, rectally, parenterally (intravenously, intramuscularly, or subcutaneously), intracisternally, intravaginally, intraperitoneally, intravesically, locally (powders, ointments, or drops), or as a buccal or nasal spray.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents, or vehicles include water, ethanol, polyols (propyleneglycol, polyethyleneglycol, glycerol, and the like), suitable mixtures thereof, vegetable oils (such as olive oil), and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example, sugars, sodium chloride, and the like. Prolonged absorption of the injectable pharmaceutical form can be brought about by the use of agents delaying absorption, for example, aluminum monostearate and gelatin.

Solid dosage forms for oral administration include capsules, tablets, pills, powders, and granules. In such solid dosage forms, the active compound is admixed with at least one inert customary excipient (or carrier) such as sodium citrate or dicalcium phosphate or,

(a) fillers or extenders, as for example, starches, lactose, sucrose, glucose, mannitol, and silicic acid;
(b) binders, as for example, carboxymethylcellulose, alignates, gelatin, polyvinylpyrrolidone, sucrose, and acacia;
(c) humectants, as for example, glycerol;
(d) disintegrating agents, as for example, agar-agar, calcium carbonate, potato or tapioca starch, alginic acid, certain complex silicates, and sodium carbonate;
(e) solution retarders, as for example paraffin;
(f) absorption accelerators, as for example, quaternary ammonium compounds;
(g) wetting agents, as for example, cetyl alcohol, and glycerol monostearate;
(h) adsorbents, as for example, kaolin and bentonite; and
(i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents.

Solid compositions of a similar type may also be employed as fillers in soft and hard-filled gelatin capsules using such excipients as lactose or milk sugar as well as high molecular weight polyethyleneglycols, and the like.

Solid dosage forms such as tablets, dragees, capsules, pills, and granules can be prepared with coatings and shells, such as enteric coatings and others well-known in the art. They may contain opacifying agents, and can also be of such composition that they release the active compound or compounds in a certain part of the intestinal tract in a delayed manner. Examples of embedding compositions which can be used are polymeric substances and waxes. The active compounds can also be in micro-encapsulated form, if appropriate, with one or more of the above-mentioned

excipients.

Liquid dosage forms for oral administration include pharmaceutically acceptable emulsions, solutions, suspensions, syrups, and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil, and sesame oil, glycerol, tetrahydrofurfuryl alcohol, polyethyleneglycols, and fatty acid esters of sorbitan or mixtures of these substances, and the like.

Besides such inert diluents, the composition can also include adjuvants, such as wetting agents, emulsifying and suspending agents, sweetening, flavoring, and perfuming agents.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like.

Compositions for rectal administrations are preferably suppositories which can be prepared by mixing the compounds of the present invention with suitable non-irritating excipients or carriers such as cocoa butter, polyethyleneglycol, or a suppository wax, which are solid at ordinary temperatures but liquid at body temperature and, therefore, melt in the rectum or vaginal cavity and release the active component.

Dosage forms for topical administration of a compound of this invention include ointments, powders, sprays, and inhalants. The active component is admixed under sterile conditions with a physiologically acceptable carrier and any preservatives, buffers, or propellants as may be required. Ophthalmic formulations, eye ointments, powders, and solutions are also contemplated as being within the scope of this invention.

The compounds of the present invention can be administered to a patient at dosage levels in the range of about 0.1 to about 1,000 mg per day. For a normal human adult having a body weight of about 70 kg, a dosage in the range of about 0.01 to about 100 mg/kg of body weight per day is preferable. The specific dosage used, however, can vary. For example, the dosage can depend on a numbers of factors including the requirements of the patient, the severity of the condition being treated, and the pharmacological activity of the compound being used. The determination of optimum dosages for a particular patient is well-known to those skilled in the art.

The terms pharmaceutically acceptable salts, esters, amides, and prodrugs as used herein refers to those carboxylate salts, amino acid addition salts, esters, amides, and prodrugs of the compounds of the present invention which are within the scope of sound medical judgement, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention. The term "salts" refers to the relatively nontoxic, inorganic and organic acid addition salts of compounds of the present invention. These salts can be prepared *in situ* during the final isolation and purification of the compounds or by separately reacting the purified compound in its free base form with a suitable organic or inorganic acid and isolating the salt thus formed. Representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate mesylate, glucoheptonate, lactobionate, and laurylsulphonate salts and the like. These may include cations based on the alkali and alkaline earth metals, such as sodium, lithium, potassium, calcium, magnesium, and the like, as well as nontoxic ammonium, quaternary ammonium, and amine cations including, but not limited to ammonium, tetramethylammonium, tetraethylammonium, methylamine, dimethylamine, trimethylamine, triethylamine, ethylamine and the like. (See, for example, Berge S.M. et al., "Pharmaceutical Salts," *J. Pharm. Sci.* 1977;66:1-19.

Examples of pharmaceutically acceptable, nontoxic esters of the compounds of this invention include $C_1$-$C_6$ alkyl esters wherein the alkyl group is a straight or branched chain. Acceptable esters also include $C_5$-$C_7$ cycloalkyl esters as well as arylalkyl esters such as, but not limited to, benzyl. $C_1$-$C_4$ alkyl esters are preferred. Esters of the compounds of the present invention may be prepared according to conventional methods.

Examples of pharmaceutically acceptable, nontoxic amides of the compounds of this invention include amides derived from ammonia, primary $C_1$-$C_6$ alkyl amines, and secondary $C_1$-$C_6$ dialkyl amines wherein the alkyl groups are straight or branched chain. In the case of secondary amines, the amine may also be in the form of a 5- or 6-membered heterocycle containing one nitrogen atom. Amides derived from ammonia, $C_1$-$C_3$ alkyl primary amines and $C_1$-$C_2$ dialkyl secondary amines, are preferred. Amides of the compounds of the invention may be prepared according to conventional methods.

The term "prodrug" refers to compounds that are rapidly transformed *in vivo* to yield the parent compound of the above formulae, for example, by hydrolysis in blood. A thorough discussion is provided in Higuchi T. and Stella V., "Pro-drugs as Novel Delivery Systems," Vol 14 of the A.C.S. Symposium Series, and in *Bioreversible Carriers in Drug* Design, ed. Edward B. Roche, American Pharmaceutical Association and Pergamon Press, 1987.

In addition, the compounds of the present invention can exist in unsolvated as well as solvated forms with pharmaceutically acceptable solvents such as water, ethanol, and the like. In general, the solvated forms are considered

equivalent to the unsolvated forms for the purposes of the present invention.

The compounds of the present invention can exist in different stereoisomeric forms by virtue of the presence of asymmetric centers in the compounds; i.e., each asymmetric carbon can have either the R or S configuration. It is contemplated that all stereoisomeric forms of the compounds as well as mixtures thereof, including racemic mixtures, form part of this invention.

The compounds of the present invention are administered to a patient in need of ICE inhibition. In general, patients in need of ICE inhibition are those patients having a disease or condition in which ICE plays a role. Examples of such diseases include, but are not limited to, inflammatory diseases such as rheumatoid arthritis and inflammatory bowel disease, neuroinflamatory disorders such as stroke, and septic shock. Other diseases include reperfusion injury, Alzheimer's disease, and shigellosis.

A "therapeutically effective amount" is an amount of a compound of Formula I that when administered to a patient having a disease that can be treated with a compound of Formula I ameliorates a symptom of the disease. A therapeutically effective amount of a compound of Formula I can be readily determined by one skilled in the art by administering a compound of Formula I to a patient and observing the results.

An illustration of the preparation of compounds of the present invention is given in Schemes I-VII.

## Scheme I

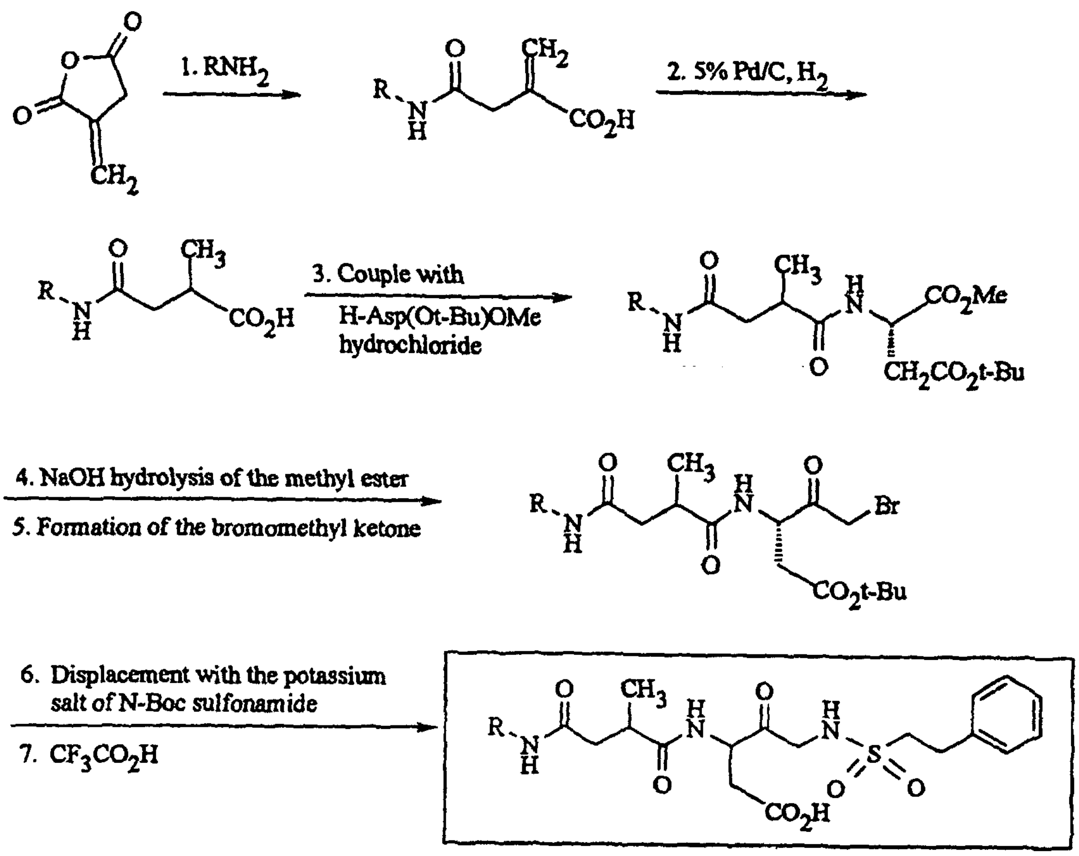

## Scheme II

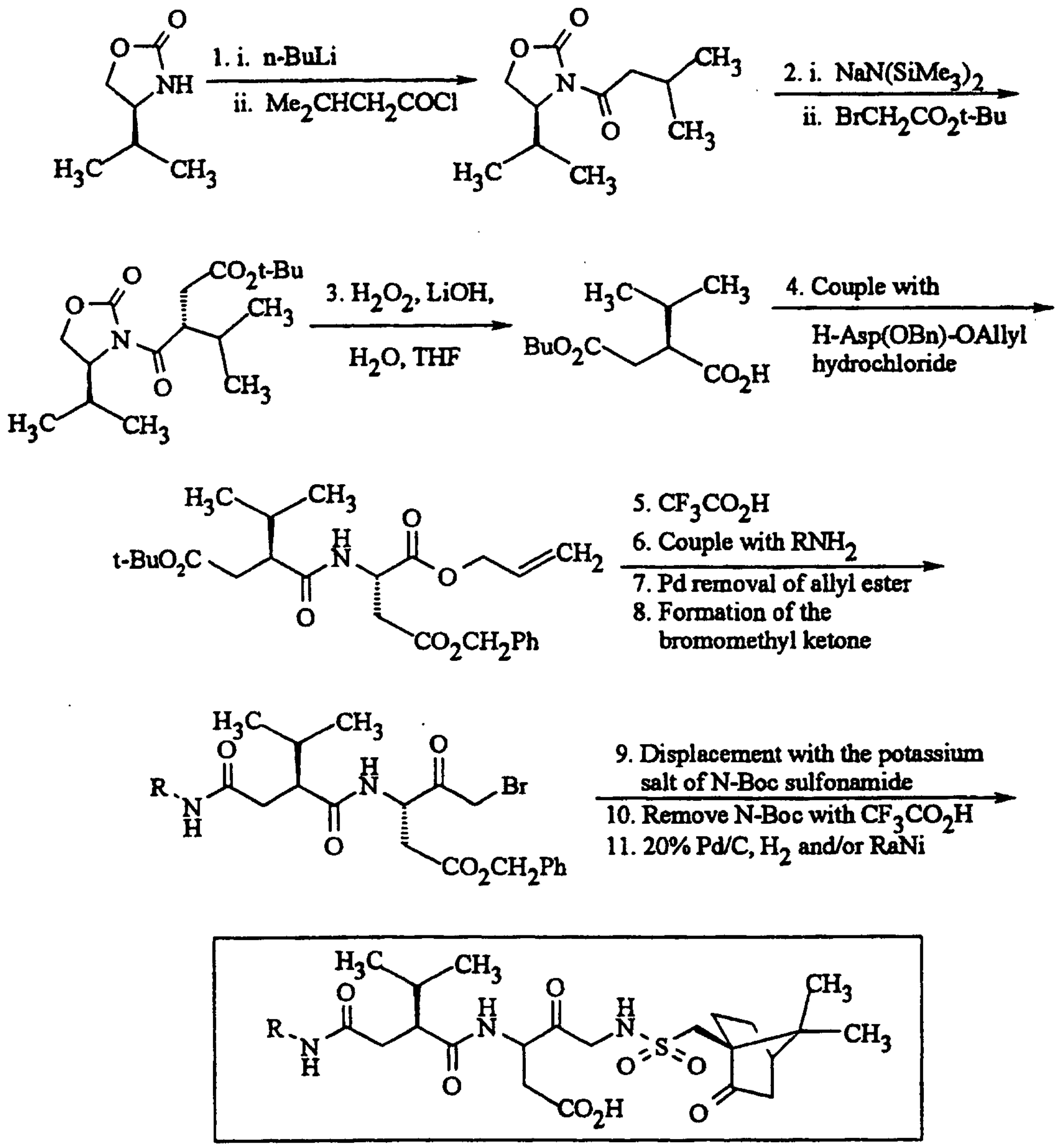
1. i. n-BuLi
ii. $Me_2CHCH_2COCl$
2. i. $NaN(SiMe_3)_2$
ii. $BrCH_2CO_2t$-Bu
3. $H_2O_2$, LiOH,
$H_2O$, THF
4. Couple with
H-Asp(OBn)-OAllyl
hydrochloride
5. $CF_3CO_2H$
6. Couple with $RNH_2$
7. Pd removal of allyl ester
8. Formation of the
bromomethyl ketone
9. Displacement with the potassium
salt of N-Boc sulfonamide
10. Remove N-Boc with $CF_3CO_2H$
11. 20% Pd/C, $H_2$ and/or RaNi

## Scheme III

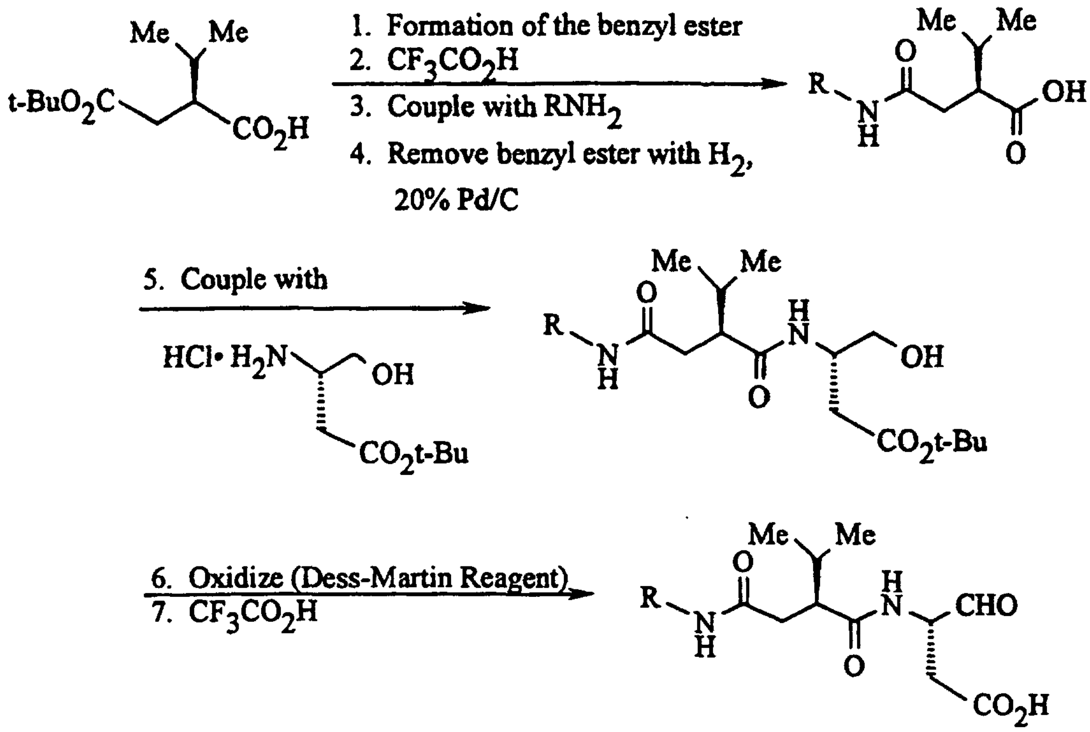
1. Formation of the benzyl ester
2. $CF_3CO_2H$
3. Couple with $RNH_2$
4. Remove benzyl ester with $H_2$,
20% Pd/C
5. Couple with
6. Oxidize (Dess-Martin Reagent)
7. $CF_3CO_2H$

## Scheme IV

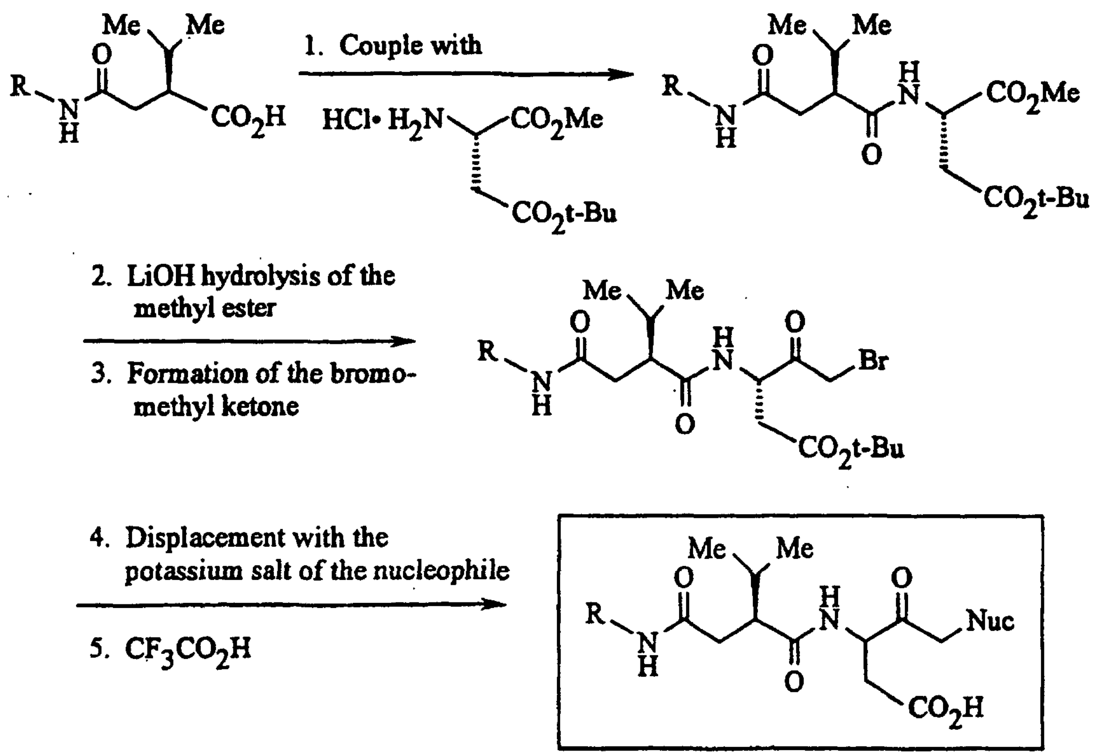
1. Couple with
2. LiOH hydrolysis of the methyl ester
3. Formation of the bromomethyl ketone
4. Displacement with the potassium salt of the nucleophile
5. $CF_3CO_2H$

Scheme V

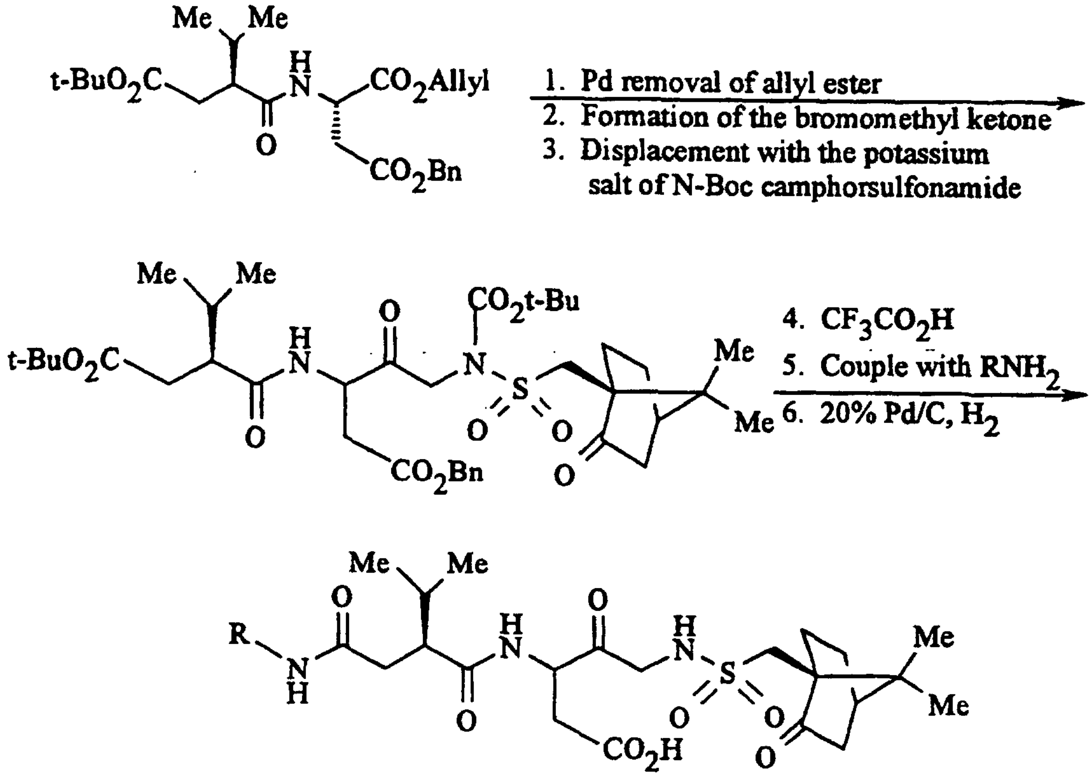
1. Pd removal of allyl ester
2. Formation of the bromomethyl ketone
3. Displacement with the potassium salt of N-Boc camphorsulfonamide
4. $CF_3CO_2H$
5. Couple with $RNH_2$
6. 20% Pd/C, $H_2$

## Scheme VI

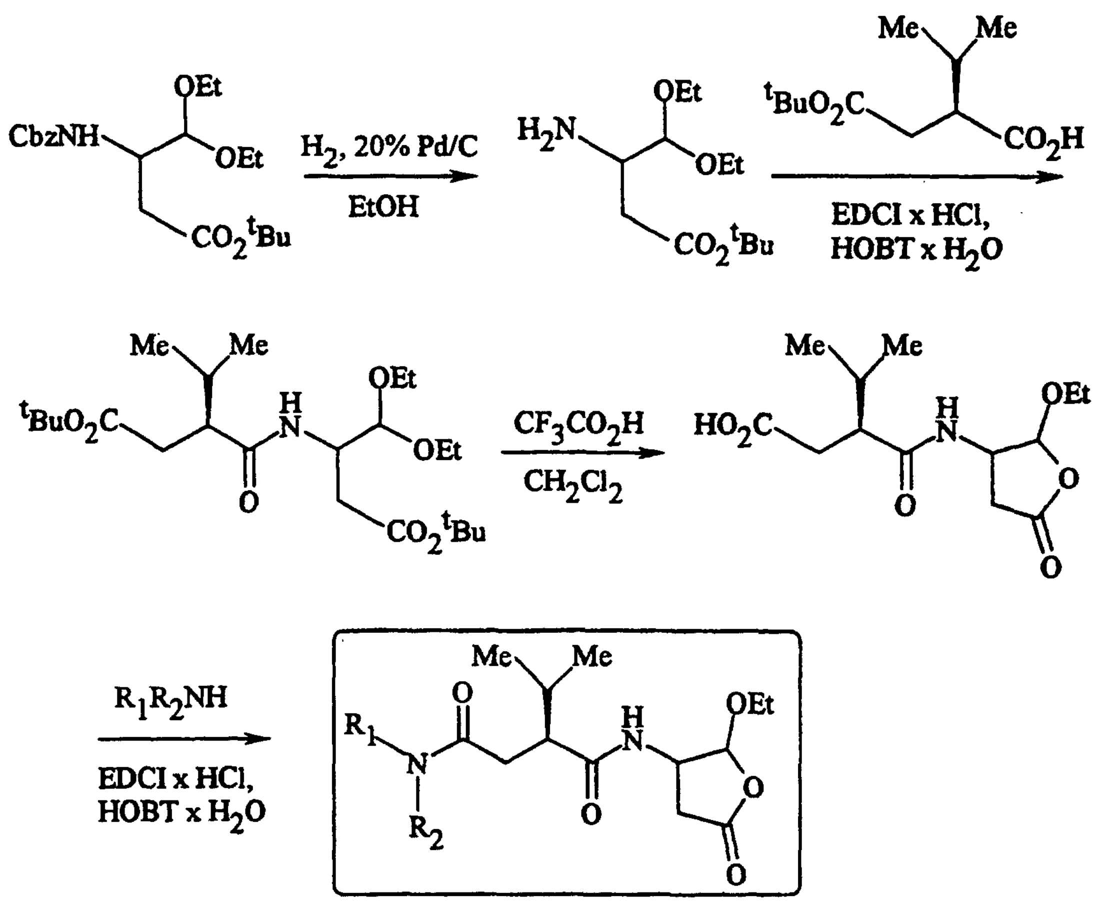


## Scheme VII

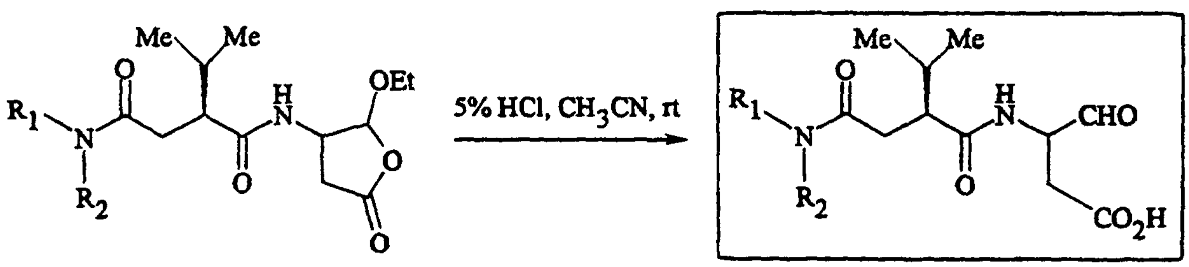


Those having skill in the art will recognize that the starting materials may be varied and additional steps employed to produce compounds encompassed by the present invention, as demonstrated by the following examples.

As shown in Scheme I, acylation of an appropriate amine with itaconic anhydride affords the desired acrylic acid, which in turn is hydrogenated to yield the respective propionic acid. The acid is then coupled to an appropriate amino acid under standard peptide coupling conditions, such as, for example, HOBT and EDCI in the presence of a base such as 4-methylmorpholine, to produce the desired succinic ester. The methyl ester is then hydrolyzed with a base such as sodium hydroxide. The resulting t-butyl ester acid is subsequently transformed to the bromomethyl ketone by, for example, forming the mixed anhydride from the acid, treating the mixed anhydride with diazomethane, and then brominating with HBr in acetic acid. The displacement of the bromide with the potassium salt of N-Boc sulfonamide results in the desired sulfonyl compound. The t-butyl ester is then hydrolyzed to the acid in the presence of an acid,

such as, for example, trifluoroacetic acid.

As shown in Scheme II, (4S)-(-)-4-isopropyl-2-oxazolidinone is acylated with an acid chloride in the presence of base to form the desired oxazolidinone, which is subsequently alkylated in the presence of base with t-butyl bromoacetate. The resulting N-acyloxazolidinone is then hydrolyzed to the succinic acid t-butyl ester essentially by the procedure described in *Tet. Lett.* 1987;28:6141-6144. The acid is then coupled to (S)-2-amino-succinic acid 1-allyl ester 4-benzyl ester under standard peptide coupling conditions as defined above in Scheme I. The t-butyl ester of this product is then cleaved and the acid is treated with O-benzylhydroxylamine under similar peptide coupling conditions to afford the desired benzyloxy carbamoyl. The allyl ester is then cleaved essentially by the procedure described in *Tet. Lett.* 1995;36:5741-5744, and the resulting acid is transformed to the bromomethyl ketone as described above in Scheme I. The bromide is displaced with the potassium salt of N-Boc sulfonamide, the Boc group is cleaved with acid, and the benzyl ester is hydrogenated over, for example, Pd/C or Raney nickel, to yield the desired pentanoic acid.

In Scheme III, the benzyl ester of the succinic acid (product 3) in Scheme II is formed from benzyl alcohol in the presence of a dehydrating agent, such as, for example, EDCI, and the t-butyl ester is then hydrolyzed with an acid. The succinic acid benzyl ester is then coupled to an appropriate amine under standard peptide coupling conditions as described in Scheme I, and the benzyl ester is then cleaved via hydrogenation as described in Scheme II to afford the butyric acid. The acid is subsequently coupled to the amino alcohol shown in Step 5, and the resulting alcohol is then oxidized to the aldehyde essential by the procedure described by Dess and Martin in *J. Org. Chem.* 1993;58:2899 and in *J. Org. Chem.* 1983;48:4156-4158. The final product is obtained upon hydrolysis of the t-butyl ester with an acid.

As shown in Scheme IV, the succinic acid of Scheme II (product 3) is coupled to the amino ester of Step 1 under peptide conditions similar to those described above in Scheme I. The ester is hydrolyzed with base, such as, for example, lithium hydroxide, and the resulting acid is converted to the bromomethyl ketone as described above in Scheme II. The bromide is displaced with the potassium salt of an appropriate nucleophile and the t-butyl ester is hydrolyzed with acid to produce the desired product.

As shown in Scheme VI, the benzyloxycarbonyl (Cbz) protecting group of the diethyl acetal of Cbz-Asp(OtBu) -H is removed by hydrogenolysis using 20% Pd/C as a catalyst. This amine is coupled to the mono-protected succinic acid of Scheme II (product 3) under standard peptide conditions such as, for example, HOBT and EDCI in the presence of a base such as 4-methylmorpholine. Removal of the t-butyl groups may be achieved with, for example, trifluoroacetic acid in dichloromethane to provide the cyclic

O-ethylacetal. Coupling of this acid under standard peptide coupling conditions, as stated above, for example, with various amines gives the desired product.

It is noted that compounds containing the cyclic structure

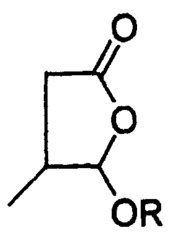

can exist in equilibrium with the open chain form

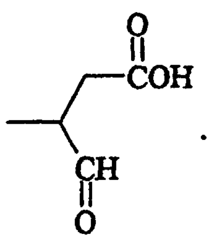

Both the cyclic and open chain forms are intended to be part of the present invention.

As shown in Scheme VII, acid hydrolysis of the cyclic O-ethylacetal of Scheme V (product 4) for example, in dilute hydrochloric acid in acetonitrile affords the aldehyde acid.

The starting materials and various intermediates may be obtained from commercial sources, prepared from commercially available organic compounds, or prepared using well-known synthetic methods.

The examples presented below are intended to illustrate particular embodiments of the invention and are not intended to limit the scope of the specification, including the claims, in any manner.

EXAMPLE 1

**3-(2-Methyl-3-phenethylcarbamoyl-propionylamino)-4-oxo-5-(2-phenylethanesulfonylamino)-pentanoic acid**

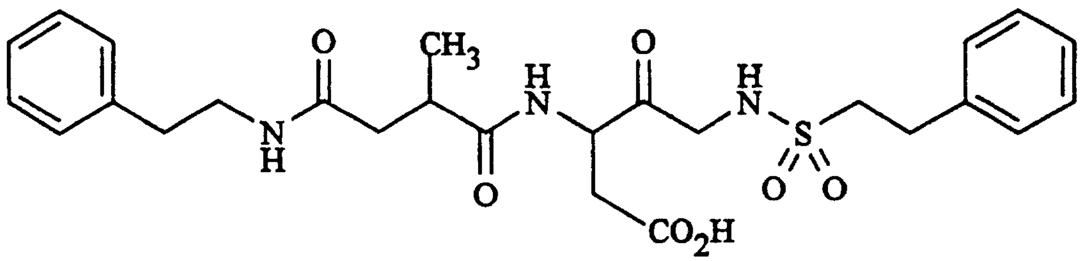

**Step A**

A solution of itaconic anhydride (5.00 g, 44.6 mmol) and phenethylamine (5.95 g, 49.1 mmol) in 100 mL of acetonitrile is stirred at room temperature under nitrogen for 72 hours. The mixture (solid forms) is concentrated, then partitioned between EtOAc and 1N HCl. The organic extract is washed with brine, dried ($MgSO_4$), concentrated, and the residue crystallized from diethyl ether to give 5.24 g (50%) of 2-(phenethylcarbamoyl-methyl)-acrylic acid as a white solid:
mp 133-140°C.
MS (APCI) m/z 234.2 (M+1, 67.4%) and 216.2 (M-17, 100%).

| Analysis Calcd for $C_{13}H_{15}NO_3$ (233.269): | | | |
|---|---|---|---|
| | C, 66.94; | H, 6.48; | N, 6.00. |
| Found | C, 66.74; | H, 6.56; | N, 6.00. |

**Step B**

A solution of 2-(phenethylcarbamoyl-methyl)-acrylic acid (2.76 g, 11.8 mmol, Example 1, Step A) in 100 mL of THF is treated with 5% Pd/C (0.2 g) and hydrogenated at room temperature and 52 psig of hydrogen for 2.5 hours. The mixture is filtered and concentrated to give 2.39 g (86%) of 2-(phenethylcarbamoyl-methyl)-propionic acid as an off-white solid.
MS (APCI) m/z 236.0 (M+1, 100%).

| Analysis Calcd for $C_{13}H_{17}NO_3$ (235.285): | | | |
|---|---|---|---|
| | C, 66.36; | H, 7.28; | N, 5.95. |
| Found | C, 65.31; | H, 7.05; | N, 5.80. |

**Step C**

A mixture of 2-(phenethylcarbamoyl-methyl)-propionic acid (1.63 g, 6.93 mmol, Example 1, Step B), H-Asp (OtBu)-OMe·HCl (1.83 g, 7.64 mmol, purchased from Bachem Bioscience Inc.), 1-hydroxybenzotriazole hydrate (HOBT·$H_2O$, 1.17 g, 7.64 mmol), N-ethyl-N'-(3-dimethylaminopropyl)-carbodiimide hydrochloride (EDCI·HCl, 1.46 g, 7.62 mmol) and 4-methylmorpholine (0.95 mL, 8.64 mmol) in 100 mL of dichloromethane is stirred at room temperature for 24 hours. The mixture is concentrated, then partitioned between EtOAc and saturated $NaHCO_3$ solution. The organic extract is washed with saturated $KH_2PO_4$ and brine solutions, dried ($MgSO_4$), filtered, concentrated and chromatographed (silica gel, 25% hexanes/75% EtOAc) to give 2.54 g (87%) of 2-(2-methyl-3-phenethylcarbamoyl-propionylamino)-succinic acid, 4-tert-butyl ester 1-methyl ester as a waxy white solid.

MS (APCI) m/z 420.9 (M, 100%).

| Analysis Calcd for $C_{22}H_{32}N_2O_6$ (420.510): | | | |
|---|---|---|---|
| | C, 62.84; | H, 7.67; | N, 6.66. |
| Found | C, 62.68; | H, 7.69; | N, 6.54. |

**Step D**

A solution of 2-(2-methyl-3-phenethylcarbamoyl-propionylamino)-succinic acid, 4-tert-butyl ester 1-methyl ester (2.11 g, 5.01 mmol, Example 1, Step C) and 0.1N sodium hydroxide solution (60.1 mL, 6.01 mmol) in 60 mL of ethanol is stirred at room temperature for 12 hours. The solution is concentrated, acidified with saturated $KH_2PO_4$ solution to a pH ~5, and extracted with chloroform (2 × 100 mL). The combined chloroform extract is dried ($MgSO_4$), filtered, and concentrated to give 2.23 g (~100%) of 2-(2-methyl-3-phenethylcarbamoyl-propionylamino)-succinic acid, 4-tert-butyl ester as a colorless oil, which is used without further purification.

A solution of 2-(2-methyl-3-phenethylcarbamoyl-propionylamino)-succinic acid, 4-tert-butyl ester (2.23 g, 5.49 mmol) and 4-methylmorpholine (0.61 mL, 5.73 mmol) in 50 mL of THF in a Clear-Seal joint 250 mL round-bottom flask is cooled to ca. -45°C (Dry Ice-acetonitrile slurry) and treated with isobutyl chloroformate (0.75 mL, 5.78 mmol). Solid immediately forms and the mixture is stirred for 15 minutes, then treated with a 0.25 to 0.5 M diazomethane in ether solution (55 mL, 27.5 mmol, generated from Diazald and freshly distilled). The cooling bath is removed, the light yellow solution is stirred at room temperature for 2 hours, cooled to 0°C, and treated dropwise with a solution of 48% hydrobromic acid (10 mL, 184 mmol) in 10 mL of acetic acid. The colorless solution is stirred at room temperature for 30 minutes, then partitioned between EtOAc and water (~200 mL of each). The organic extract is washed with water, saturated $NaHCO_3$, and brine solutions, dried ($MgSO_4$), filtered, and concentrated to give 1.40 g (53%) of 5-bromo-2-(2-methyl-3-phenethylcarbamoyl-propionylamino)-4-oxo-pentanoic acid, tert-butyl ester as a light yellow solid.

**Step E**

A solution of 1,1-dimethylethyl[(2-phenylethyl)sulfonyl]carbamate (0.47 g, 1.66 mmol) in 3.0 mL of DMF at room temperature under nitrogen is treated with potassium tert-butoxide (0.21 g, 1.66 mmol). The sample is stirred at room temperature for 1 hour, cooled to 0°C, then treated with 2-(2-methyl-3-phenethylcarbamoyl-propionylamino)-5-bromo-4-oxo-pentanoic acid, tert-butyl ester (0.67 g, 1.38 mmol, Example 1, Step D). The sample is allowed to warm to room temperature overnight. The sample is partitioned between EtOAc and saturated $NaHCO_3$ solution. The organic extract is washed with saturated $KH_2PO_4$ and brine solutions, dried ($MgSO_4$), filtered, and concentrated. The residue is chromatographed (silica gel, 50% hexanes/50% EtOAc) to give 0.43 g (52%) of 1,1-dimethylethyl 5-[[(1,1-dimethylethoxy)carbonyl][(2-phenylethyl)sulfonyl]amino]-3-[[2-methyl-1,4-dioxo-4-[(2-phenylethyl)amino]butyl]amino]-4-oxopentanoate as a foamy light yellow solid.

**Step F**

A solution of 3-(2-methyl-3-phenethylcarbamoyl-propionylamino)-4-oxo-5-[(2-phenyl-ethanesulfonyl)-N-Boc-amino]-pentanoic acid, 5-tert-butyl ester (0.40 g, 0.58 mmol, Example 1, Step E) and trifluoroacetic acid (10 mL) in 20 mL of dichloromethane is stirred at room temperature for 2 hours. The solution is concentrated to a yellow oil. Diethyl ether (~50 mL) is added and the oil solidifies. The sample is stirred at room temperature overnight, filtered, washed with fresh ether, and vacuum dried to give 0.28 g (89%) of 3-(2-methyl-3-phenethylcarbamoyl-propionylamino)-4-oxo-5-(2-phenyl-ethanesulfonylamino)-pentanoic acid as a white solid.
MS (APCI) m/z 532.1 (M+1, 100%).

| Analysis Calcd for $C_{26}H_{33}N_3O_7S$ (531.633): | | | |
|---|---|---|---|
| | C, 58.74; | H, 6.26; | N, 7.90. |
| Found | C; 58.38; | H, 6.23; | N, 7.71. |

EXAMPLE 2

**3-(2-Carbamoylmethyl-3-methyl-butyrylamino)-5-(7,7-dimethyl-2-oxo-bicyclo[2.2.1]hept-1-ylmethanesulfonylamino)-4-oxo-pentanoic acid**

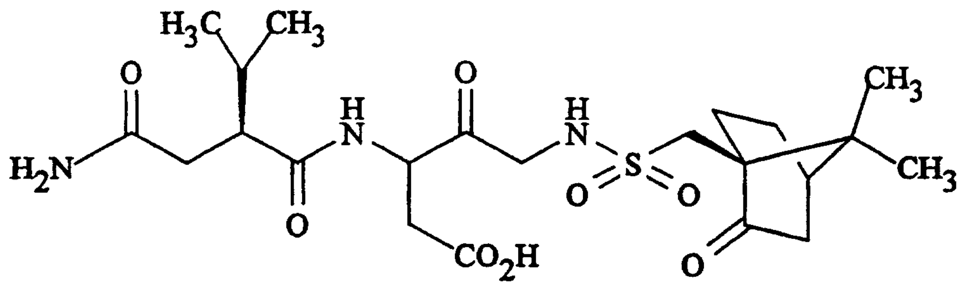

**Step A**

A solution of (4S)-(-)-4-isopropyl-2-oxazolidinone (19.85 g, 0.154 mol) in 400 mL of THF at -78°C under $N_2$ is treated dropwise with *n*-butyl lithium (64.5 mL, 0.161 mol, 2.5 M solution in hexanes) resulting in the formation of solid. The mixture is stirred at -78°C for 30 minutes, then treated with dropwise addition of *iso*-valeryl chloride (20.6 mL, 0.169 mol). The reaction is allowed to warm to room temperature slowly overnight. The sample is concentrated and then partitioned between EtOAc and saturated $KH_2PO_4$ solution. The organic extract is washed with brine, dried ($MgSO_4$), and the resultant yellow oil is chromatographed (MPLC, silica gel, 10% EtOAc in hexanes) to give 29.8 g (91%) of (S)-4-isopropyl-3-(3-methyl-butyryl)-oxazolidin-2-one as a light yellow oil.

**Step B**

A solution of (S)-4-isopropyl-3-(3-methyl-butyryl)-oxazolidin-2-one (20.8 g, 97.5 mmol, Example 2, Step A) in 500 mL of THF at -78°C under $N_2$ is treated dropwise with sodium bis(trimethylsilyl)amide (107 mL, 107 mmol, 1.0 M solution in THF). The solution is stirred at -78°C for 30 minutes, then treated dropwise with a solution of *tert*-butyl bromoacetate (18.0 mL, 121.9 mmol) in 100 mL of THF. The sample is stirred at -78°C for 1 hour, then quenched by dropwise addition of saturated $KH_2PO_4$ solution (~125 mL). The mixture is warmed to room temperature, concentrated (to remove most of the THF), then extracted with ether. The organic extract is washed with saturated $NaHCO_3$ and brine solutions, dried ($MgSO_4$), concentrated, and crystallized from ether-petroleum ether to give 21.1 g (66%) of [(S-(R*,R*)]-3-(4-isopropyl-2-oxo-oxazolidine-3-carbonyl)-4-methyl-pentanoic acid tert-butyl ester as a white solid.

| Analysis Calcd for $C_{17}H_{29}NO_5$ (327.424): | | | |
|---|---|---|---|
| | C, 62.36; | H, 8.93; | N, 4.28. |
| Found | C, 62.30; | H, 9.07; | N, 4.09. |

**Step C**

The hydrolysis of the N-acyloxazolidone is achieved using lithium hydroperoxide following the procedure of Evans D.A. et al. (*Tet. Lett.* 1987;28:6141-6144). To a stirring solution at 0°C of [(S-(R*,R*)]-3-(4-isopropyl-2-oxo-oxazolidine-3-carbonyl)-4-methyl-pentanoic acid tert-butyl ester (9.05 g, 27.64 mmol, Example 2, Step B) in 250 mL of THF is added dropwise hydrogen peroxide (14.1 mL, 138 mmol, 30 wt% solution in water) followed by 1.0 M lithium hydroxide solution (55.3 mL, 55.3 mmol). The reaction is allowed to warm to room temperature slowly overnight. The reaction is concentrated to remove most of the THF, and then the basic solution is washed with $CH_2Cl_2$ (2 × 100 mL). The aqueous phase is cooled, acidified with saturated $KH_2PO_4$ solution to a pH ~5, and extracted into EtOAc. The organic extract is washed with brine solution, dried ($MgSO_4$), and concentrated to give 5.66 g (95%) of (S)-2-isopropyl succinic acid 4-tert-butyl ester as a colorless oil, which is used without further purification.

**Step D**

A mixture of (S)-2-isopropyl succinic acid 4-tert-butyl ester (10.77 g, 49.80 mmol, Example 2, Step C), (S)-2-amino-succinic acid 1-allyl ester 4-benzyl ester hydrochloride (14.93 g, 49.81 mmol), HOBT·$H_2O$ (8.4 g, 54.8 mmol), EDCI·HCl (10.5 g, 54.8 mmol) and 4-methylmorpholine (8.2 mL, 74.6 mmol) in 250 mL of $CH_2Cl_2$ is stirred at room temperature for 12 hours. The mixture is concentrated, then partitioned between EtOAc and saturated $NaHCO_3$ solution. The EtOAc extract is washed with saturated $KH_2PO_4$ and brine solutions, dried ($MgSO_4$), filtered, concentrated, and chromatographed (MPLC, silica gel, 20% EtOAc in hexanes) to give 19.21 g (84%) of [S-(R*,R*)]-2-(2-tert-butoxycarbonylmethyl-3-methyl-butyrylamino)-succinic acid 1-allyl ester 4-benzyl ester as light yellow oil.

**Step E**

A solution of [S-(R*,R*)]-2-(2-tert-butoxycarbonylmethyl-3-methyl-butyrylamino)-succinic acid 1-allyl ester 4-benzyl ester (9.3 g, 23.0 mmol, Example 2, Step D) and trifluoroacetic acid (35 mL) in 35 mL of $CH_2Cl_2$ is stirred at room temperature under $N_2$ for 2 hours. The sample is concentrated, redissolved into $CH_2Cl_2$, then treated with EDCI·HCl (8.8 g, 46.0 mmol), HOBT·$H_2O$ (6.2 g, 46.0 mmol), and O-benaylhydroxylamine hydrochloride (7.3 g, 46.0 mmol). 4-Methyl-morpholine (11.6 g, 115 mmol) is added dropwise, and the reaction mixture is stirred at room temperature overnight. The sample is diluted with $CH_2Cl_2$ and washed successively with 5% HCl and saturated $NaHCO_3$ solutions. The organic extract is dried ($Na_2SO_4$) and concentrated to afford 10.25 g (88%) of the 2-[2-(benzyloxycarbamoyl-methyl)-3-methyl-butyrylamino]-succinic acid 1-allyl ester 4-tert-butyl ester as a white solid which is carried on to the next step without further purification.

**Step F**

The allyl ester is cleaved employing the procedure of Dessolin M. et al. (*Tet. Lett.* 1995;36:5741-5744). A solution at 0°C under $N_2$ of 2-[2-(benzyloxycarbamoyl-methyl)-3-methyl-butyrylamino]-succinic acid 1-allyl ester 4-tert-butyl ester (10.25 g, 19.7 mmol, Example 2, Step E) and tetrakis(triphenylphosphine)palladium (0) (0.462 g, 0.40 mmol) in $CH_2Cl_2$ is treated dropwise with phenylsilane (4.26 g, 39.4 mmol). The reaction mixture is allowed to warm to room temperature over a period of 1 hour, then washed with saturated $KH_2PO_4$ solution. The organic layer is extracted with 0.5 N NaOH. The basic aqueous phase is acidified with concentrated HCl and extracted with EtOAc. The organic extract is dried ($Na_2SO_4$), filtered, and concentrated to afford 6.2 g (72%) of the substituted succinic acid 4-benzyl ester as foamy white solid.

To a solution of the above acid (6.0 g, 12.8 mmol) and 4-methylmorpholine (1.3 g, 12.8 mmol) in THF (50 mL) at -42°C is added isobutyl chloroformate (1.8 g, 12.8 mmol) dropwise. After stirring 30 minutes, the reaction mixture is added to a solution of diazomethane in diethyl ether (~0.5 M, 200 mL) at 0°C. The reaction mixture is stirred for 2 hours at room temperature then cooled to 0°C. A solution of 48% HBr (20 mL) and HOAc (20 mL) is added dropwise, and the reaction is stirred for 30 minutes at 0°C. The sample is diluted with diethyl ether and washed with water (2x) and saturated $NaHCO_3$ solution (2x). The organic layer is dried ($Na_2SO_4$) and concentrated. The residue is dissolved in $CH_2Cl_2$, and the product is precipitated with hexanes. The solid is collected by filtration, washed well with hexanes, and dried to afford 1.5 g (15%) of 3-[2-(benzyloxycarbamoyl-methyl)-3-methyl-butyrylamino]-5-bromo-4-oxo-pentanoic acid benzyl ester as a white solid.

**Step G**

To a solution of (S)-1,1-dimethylethyl[[(7,7-dimethyl-2-oxobicyclo[2.2.1]hept-1-yl)methyl]sulfonyl]carbamate (0.273 g, 0.82 mmol) in anhydrous DMF (3 mL) is added potassium tert-butoxide (0.092 g, 0.82 mmol). The reaction is stirred under a nitrogen atmosphere for 1 hour. This solution is then added dropwise to a solution of 3-[2-(benzyloxycarbamoyl-methyl)-3-methyl-butyrylamino]-5-bromo-4-oxo-pentanoic acid benzyl ester (0.400 g, 0.75 mmol, Example 2, Step F) in anhydrous DMF (3 mL), and the reaction is stirred overnight. The sample is diluted with EtOAc and washed with brine (2x). The EtOAc layer is dried ($Na_2SO_4$) and concentrated. The residue is chromatographed (silica gel, 40% EtOAc/60% hexanes) to afford 0.271 g of phenylmethyl-5-[[(1,1-dimethylethoxy)carbonyl]-[[(7,7-dimethyl-2-oxobicyclo [2.2.1]hept-1-yl)methyl]sulfonyl]amino]-3-[[2-(1-methylethyl)-1,4-dioxo-4-[(phenylmethoxy)amino]butyl]amino]-4-oxo-pentanoate as a white foamy solid.

The sample is treated with 50% trifluoroacetic acid in $CH_2Cl_2$ (6 mL) for 1 hour. The sample is diluted with $CH_2Cl_2$ and washed with saturated $NaHCO_3$ solution. The organic layer is dried ($Na_2SO_4$) and concentrated to give 0.237 g of a white solid which is carried on to the next step without further purification.

**Step H**

To a solution of the above compound (0.237 g, Example 2, Step G) in 75 mL of THF is added 10% Pd/C (0.50 g), and the mixture is hydrogenated at 50 psi $H_2$, room temperature for 3 hours. The reaction mixture is filtered, and Raney Nickel (0.10 g) is added. The reaction mixture is again hydrogenated at 50 psi for 15 hours. The sample is filtered, and the filtrate is concentrated. The residue is partitioned between EtOAc and saturated $NaHCO_3$ solution. The aqueous layer is separated, acidified with HCl, and extracted with EtOAc. The organic layer is dried ($Na_2SO_4$) and concentrated to afford 0.065 g of 3-(2-carbamoylmethyl-3-methyl-butyrylamino)-5-(7,7-dimethyl-2-oxo-bicyclo[2.2.1]hept-1-ylmethanesulfonylamino)-4-oxo-pentanoic acid (Compound 2) as a pinkish solid.

| Analysis Calcd for $C_{22}H_{35}N_3O_8S \times 0.27\ C_4H_8O_2$ (525.392): | | | |
|---|---|---|---|
| | C, 52.76; | H, 7.13; | N, 8.00. |
| Found | C, 52.36; | H, 7.06; | N, 7.65. |

The following compounds can be prepared in accordance with the procedure of Example 2.

EXAMPLE 3

**5-(7,7-Dimethyl-2-oxo-bicyclo[2.2.1]hept-1-ylmethanesulfonylamino)-3-[3-methyl-2-(phenethylcarbamoyl-methyl)-butyrylamino]-4-oxo-pentanoic acid**

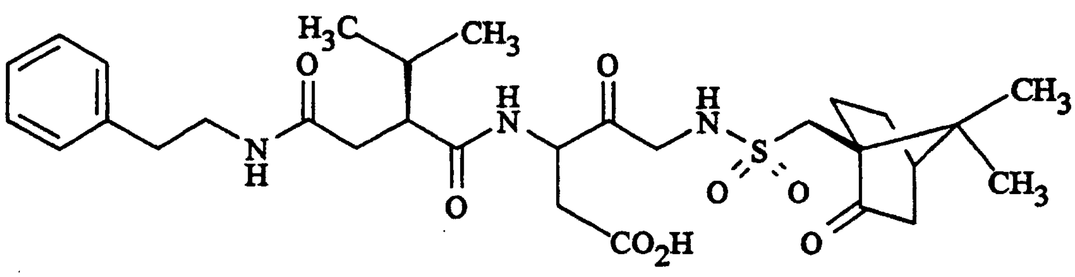

A tan foamy solid.
MS (APCI) m/z 606.4 (M+1, 25.0%), 346.3 (100%).

| Analysis Calcd for $C_{30}H_{43}N_3O_8S \times 0.50\ H_2O$ (614.764): | | | |
|---|---|---|---|
| | C, 58.61; | H, 7.21; | N, 6.84. |
| Found | C, 58.62; | H, 7.18; | N, 6.41. |

EXAMPLE 4

**3-(2-Carbamoylmethyl-3-methyl-butyrylamino)-4-oxo-5-(2-phenyl-ethanesulfonyl-amino)-pentanoic acid**

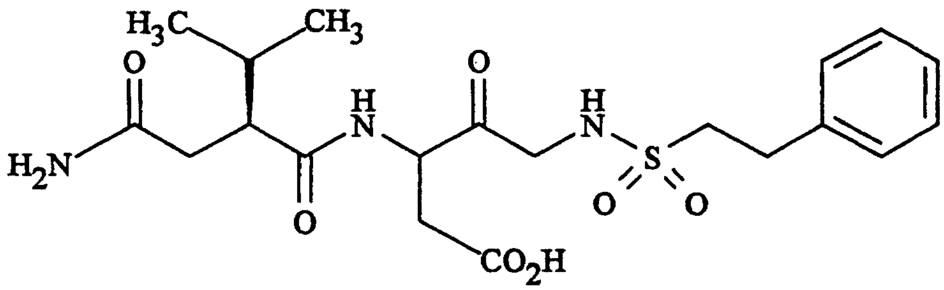

MS (APCI) m/z 456.3 (M+1, 74.7%), 242.2 (100%).

| Analysis Calcd for $C_{20}H_{29}N_3O_7S \times 0.13\ CF_3CO_2H$ (470.357): | | | |
|---|---|---|---|
| | C, 51.74; | H, 6.24; | N, 8.93. |
| Found | C, 51.70; | H, 5.97; | N, 8.61. |

EXAMPLE 5

**3-[3-Methyl-2-(phenethylcarbamoyl-methyl)-butyrylamino]-4-oxo-5-(2-phenyl-ethanesulfonylamino)-pentanoic acid**

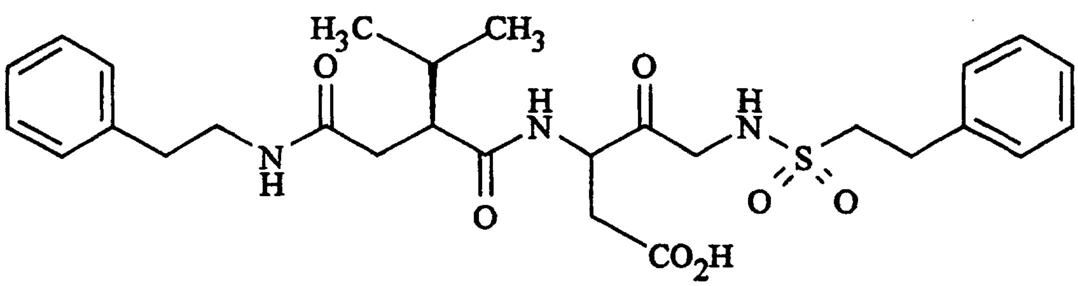

A white solid.
MS (APCI) m/z 560.4 (M+1, 50.7%), 346.3 (100%).

| Analysis Calcd for $C_{28}H_{37}N_3O_7S \times 0.27\ H_2O$ (564.551): | | | |
|---|---|---|---|
| | C, 59.57; | H, 6.70; | N, 7.44. |
| Found | C, 59.56; | H, 6.52; | N, 7.41. |

EXAMPLE 6

**5-(7,7-Dimethyl-bicyclo[2.2.1]hept-1-ylmethanesulfonylamino)-3-[3-methyl-2-(phenethylcarbamoyl-methyl)-butyrylamino]-4-oxo-pentanoic acid**

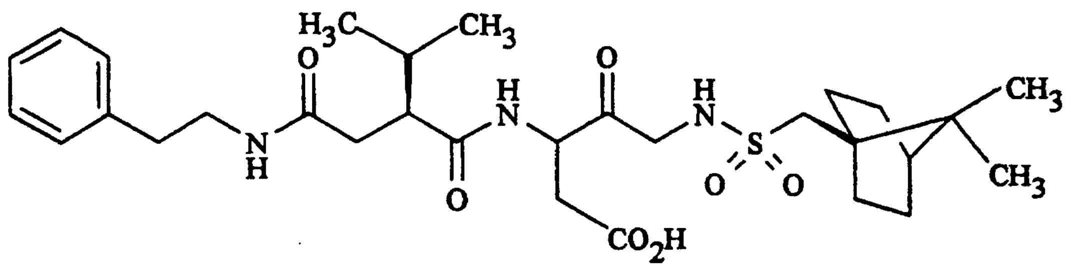

An off-white solid.

| Analysis Calcd for $C_{30}H_{45}N_3O_7S$ (591.773): | | | |
|---|---|---|---|
| | C, 60.89; | H, 7.66; | N, 7.10. |
| Found | C, 60.61; | H, 7.65; | N, 7.03. |

The starting N-Boc sulfonamide was prepared by a Wolff-Kishner reduction of (1S)-(+)-10-camphorsulfonamide followed by acylation with di-(t-butyl)-dicarbonate according to the procedure of Example 2, Step B. Neustadt R. (*Tet. Lett.* 1994;35;379-380).

EXAMPLE 7

**(S,S)-3-{3-Methyl-2-[(3-phenyl-propylcarbamoyl)-methyl]-butyrylamino}-4-oxo-butyric acid**

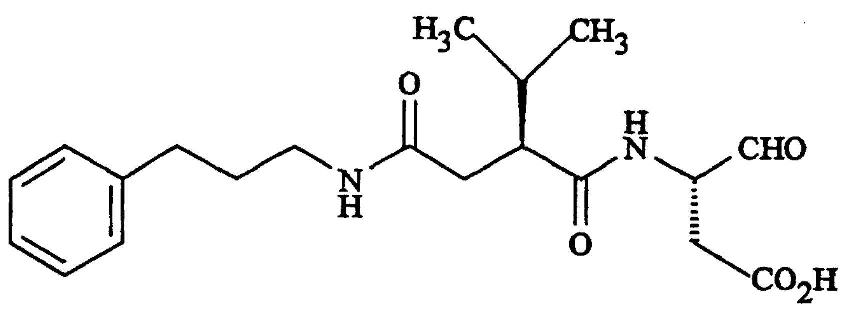

**Step A**

A mixture of (S)-2-isopropyl-succinic acid 4-tert-butyl ester (14.5 g, 67.0 mmol, Example 2, Step C), EDCI·HCl (15.4 g, 80.3 mmol), benzyl alcohol (8.7 mL, 84.1 mmol) and 4-dimethylaminopyridine (1.5 g, 12.3 mmol) in 500 mL of $CH_2Cl_2$ is stirred at room temperature for 12 hours. The sample is concentrated, then partitioned between EtOAc and saturated $NaHCO_3$ solution. The organic extract is washed with saturated $KH_2PO_4$ and brine solutions, dried ($MgSO_4$), filtered, and concentrated. The resultant yellow oil is chromatographed (MPLC, silica gel, 10% EtOAc in hexanes) yielding 15.6 g (76%) of (S)-2-isopropyl-succinic acid 1-benzyl ester 4-tert-butyl ester as a colorless oil. MS (APCI) m/z 307.2 (M+1, 92.2%), 252.2 (M-54, 90.9%) and 251.1 (M-55, 100%).

The tert-butyl ester is hydrolyzed with 20% trifluoroacetic acid in $CH_2Cl_2$ at room temperature to give (S)-2-isopropyl-succinic acid 1-benzyl ester as a light yellow oil.

MS (APCI) m/z 251.1 (M+1, 100%).

**Step B**

A mixture of (S)-2-isopropyl-succinic acid 1-benzyl ester (3.45 g, 13.78 mmol, Example 3, Step A), 3-phenyl-1-propylamine (2.15 mL, 15.12 mmol), HOB·T$H_2O$ (2.32 g, 15.15 mmol), EDCI·HCl (2.91 g, 15.18 mmol) and 4-methylmorpholine 2.3 mL (20.65 mmol) in 100 mL of $CH_2Cl_2$ is stirred at room temperature for 12 hours. The sample is concentrated and then partitioned between EtOAc and saturated $NaHCO_3$ solution. The organic extract is washed with saturated $KH_2PO_4$ and brine solutions, dried ($MgSO_4$), filtered, and concentrated to give 5.0 g (98%) of (S)-2-(phenpropylcarbamoyl-methyl)-3-methylbutyric acid benzyl ester as a light yellow oil.
MS (APCI) m/z 369.2 (M+2, 100%), 368.2 (M+1, 97,4%).
Hydrogenation of (S)-2-(phenpropylcarbamoyl-methyl)-3-methylbutyric acid benzyl ester with 20% Pd/C in EtOH at balloon pressure gave (S)-2-(phenylpropylcarbamoyl-methyl)-3-methylbutyric acid as a colorless oil.
MS (APCI) m/z 279.1 (M+2, 83.1%), 278.1 (M+1, 83.1%), and 260.1 (M-17, 100%).

**Step C**

A mixture of (S)-2-(phenyl-propylcarbamoyl-methyl)-3-methylbutyric acid (2.04 g, 7.34 mmol, Example 3, Step B), 3-amino-4-hydroxy-butyric acid tert-butyl ester monohydrochloride (1.71 g, 8.08 mmol), HOBT·$H_2O$ (1.24 g, 8.10 mmol), EDCI·HCl (1.55 g, 8.08 mmol), and 4-methylmorpholine in 50 mL of $CH_2Cl_2$ is stirred at room temperature for 12 hours. The sample is concentrated and then partitioned between EtOAc and saturated $NaHCO_3$ solution. The organic extract is washed with saturated $KH_2PO_4$ and brine solutions, dried ($MgSO_4$), filtered, and concentrated. The sample is chromatographed (MPLC, silica gel, 25% hexanes/75% EtOAc) to give 1.64 g (52%) of (S,S)-3-{3-methyl-2-[(3-phenyl-propylcarbamoyl)-methyl]-butyrylamino}-4-hydroxy-butyric acid tert-butyl ester as a foamy white solid.
MS (APCI) m/z 436.3 (M+2, 100%) and 435.3 (M+1, 83.1%).

**Step D**

A solution of (S,S)-3-{3-methyl-2-[(3-phenyl-propylcarbamoyl)-methyl]-butyrylamino}-4-hydroxy-butyric acid tert-butyl ester (1.54 g, 3.54 mmol, Example 3, Step C) and Dess-Martin reagent (2.26 g, 5.33 mmol, prepared following the procedure ofR.E. Ireland and L. Liu (*J. Org. Chem.* 1993;58:2899) in 100 mL of $CH_2Cl_2$ is stirred at room temperature for 2 hours. The sample is reductively worked-up using the procedure ofD.B. Dess and J.C. Martin (*J. Org. Chem.* 1983;48:4156-4158) to give 1.16 g (77%) of (S,S)-3-{3-methyl-2-[(3-phenyl-propylcarbamoyl)-methyl]-butyrylamino}-4-oxo-butyric acid tert-butyl ester as a waxy white solid.
MS (APCI) m/z 434.3 (M+2, 83.1%) and 433.3 (M+1, 100%).

| Analysis Calcd for $C_{24}H_{36}N_2O_5 \times 0.50\ H_2O$ (441.573): | | | |
|---|---|---|---|
| | C, 65.28; | H, 8.45; | N, 6.34. |
| Found | C, 65.31; | H, 8.13; | N, 6.16. |

**Step E**

A solution of (S,S)-3-{3-methyl-2-[(3-phenyl-propylcarbamoyl)-methyl]-butyrylamino}-4-oxo-butyric acid tert-butyl ester (1.10 g, 2.54 mmol, Example 3, Step D) and 5.0 mL of trifluoroacetic acid in 20 mL of $CH_2Cl_2$ is stirred at room temperature for 1 hour. The solution is concentrated and then partitioned between EtOAc and saturated $KH_2PO_4$ solution. The organic extract is washed with saturated $KH_2PO_4$ and brine solutions, dried ($MgSO_4$), filtered, and concentrated to give 0.83 g (86%) of (S,S)-3-{3-methyl-2-[(3-phenyl-propylcarbamoyl)-methyl]-butyrylamino}-4-oxo-butyric acid as an off-white foamy solid (Compound 7).
MS (APCI) m/z 377.2 (M+1, 100%).

| Analysis Calcd for $C_{20}H_{28}N_2O_5 \times 0.25\ H_2O$ (380.960): | | | |
|---|---|---|---|
| | C, 63.06; | H, 7.54; | N, 7.35. |
| Found: | C, 63.08; | H, 7.25; | N, 6.98. |

The following compound can be prepared according to the procedure described in Example 7.

EXAMPLE 8

**3-{3-Methyl-2-[(3-phenoxy-ethylcarbamoyl)-methyl]-butyrylamino}-4-oxo-butyric acid**

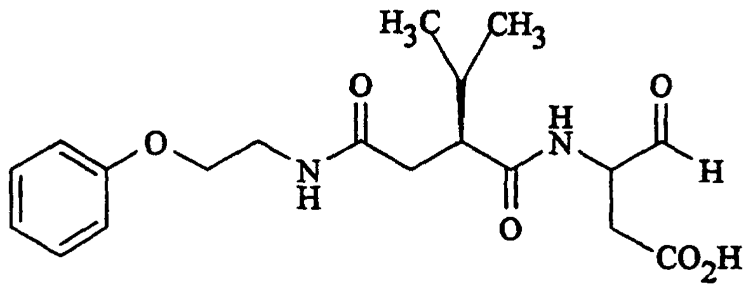

A foamy white solid.
MS (APCI) m/z 379.1 (M+1, 100%).

| Analysis Calcd for $C_{19}H_{26}N_2d_6 \times 0.50\ H_2O$ (387.437): | | | |
|---|---|---|---|
| | C, 58.90; | H, 7.02; | N, 7.23. |
| Found | C, 58.91; | H, 6.78; | N, 7.02. |

EXAMPLE 9

**5-(7,7-Dimethyl-2-oxo-bicyclo[2.2.1]hept-1-ylmethanesulfonylamino)-3-{3-methyl-2-[(2-phenoxy-ethylcarbamoyl)-methyl]-butyrylamino}-4-oxo-pentanoic acid**

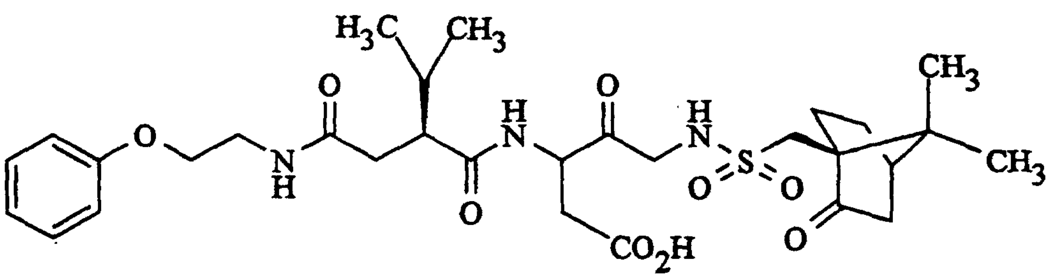

**Step A**

A mixture of (S)-2-(3-phenoxy-ethylcarbamoyl-methyl)-3-methylbutyric acid (3.51 g, 12.56 mmol, prepared using methodology described in Example 3, Steps A and B), H-Asp(OtBu)Ome × HCl (3.31 g, 13.82 mmol), HOBT × $H_2O$ (2.12 g, 13.82 mmol), EDCI·HCl (12.65 g, 13.82 mmol), and 4-methylmorpholine (2.9 mL, 26.38 mmol) in 100 mL of $CH_2Cl_2$ is stirred at room temperature for 12 hours. The sample is concentrated and then partitioned between EtOAc and saturated $NaHCO_3$ solution. The organic extract is washed with saturated $KH_2PO_4$ and brine solutions, dried ($MgSO_4$), filtered, and concentrated. The sample is chromatographed (MPLC, silica gel, 25% hexanes/75% EtOAc) to give 4.70 g (81%) of (S,S)-2-{3-methyl-2-[(2-phenoxy-ethylearbamoyl)-methyl]-butyrylamino)-succinic acid 4-tert-butyl ester 1-methyl ester as a white solid.
MS (APCI) m/z 465.2 (M+1, 100%).

| Analysis Calcd for $C_{24}H_{36}N_2O_7$ (464.564): | | | |
|---|---|---|---|
| | C, 62.05; | H, 7.81; | N, 6.03. |
| Found | C, 62.07; | H, 7.85; | N, 5.97. |

**Step B**

To a stirring solution at room temperature of (S,S)-2-{3-methyl-2-[(2-phenoxy-ethylcarbamoyl)-methyl]-butyrylamino}-succinic acid 4-tert-butyl ester 1-methyl ester (4.18 g, 9.00 mmol, Example 4, Step A) in 100 mL of THF is added dropwise 0.2 M lithium hydroxide solution (47.3 mL, 9.46 mmol). The sample is stirred for 30 minutes, acidified with saturated $KH_2PO_4$ solution, concentrated (to remove most of the THF) and extracted with EtOAc. The organic extract is washed with brine solution, dried ($MgSO_4$), filtered, and concentrated to a light yellow oil. The sample is crystallized from $CH_2Cl_2$-hexanes to give 1.96 g (48%) of (S,S)-2-{3-methyl-2-[(2-phenoxy-ethylcarbamoyl)-methyl]-butyrylamino}-succinic acid 4-tert-butyl ester as a white solid.
MS(APCI) m/z 451.2 (M+1, 100%).

| Analysis Calcd for $C_{23}H_{34}N_2O_7$ (450.537): | | | |
|---|---|---|---|
| | C, 61.32; | H, 7.61; | N, 6.22. |
| Found | C, 61.29; | H, 7.71; | N, 6.08. |

**Step C**

Using the procedure described in Example I, Step D, (S,S)-2-{3-methyl-2-[(2-phenoxy-ethylcarbamoyl)-methyl]-butyrylamino}-succinic acid 4-tert-butyl ester (2.92 g, 6.47 mmol) is converted to 3.25 g (95.2%) of (S,S)-5-bromo-3-{3-methyl-2-[(2-phenoxy-ethylcarbamoyl)-methyl]-butyrylamino}-4-oxo-pentanoic acid, tert-butyl ester as an off-white solid.
MS (APCI) m/z 529.1/527.1 (M+1, 100/98.7%).

**Step D**

To a stirring solution at room temperature of N-Boc camphorsulfonamide (1.04 g, 3.14 mmol, prepared from (1S)-(+)-10-camphorsulfonamide following the acylation procedure of Step B. Neustadt R., *Tel. Lett.* 1994;35:379-380) in 5.0 mL of DMF is added in one portion potassium *tert*-butoxide (0.35 g, 3.13 mmol). The sample is stirred at room temperature for 30 minutes, cooled to 0°C, then treated in one portion with (S,S)-5-bromo-3-{3-methyl-2-[(2-phenoxy-ethylcarbamoyl)-methyl]-butyrylamino}-4-oxo-pentanoic acid, tert-butyl ester (1.50 g, 2.84 mmol, Example 4, Step C). The sample is allowed to warm to room temperature slowly overnight. The sample is partitioned between EtOAc and saturated $KH_2PO_4$ solution. The organic extract is washed with brine solution, dried ($MgSO_4$), filtered, concentrated and chromatographed (MPLC, silica gel, 50% hexanes/50% EtOAc) to give 1.43 g (64%) of 1,1-dimethylethyl 5-[[(1,1-dimethylethoxy)carbonyl][[(7,7-dimethyl-2-oxobicyclo[2.2.1]hept-1-yl)methyl]sulfonyl]amino]-3-[[2-(1-methylethyl)-1,4-dioxo-4-[(2-phenoxyethyl)amino]butyl]amino]-4-oxopentanoate as a foamy white solid.
MS (APCI) m/z 778.3 (M+1, 100%).

**Step E**

A solution of 1,1-dimethylethyl 5-[[(1,1-dimethylethoxy)carbonyl]-[[(7,7-dimethyl-2-oxobicyclo[2.2.1]hept-1-yl)methyl]sulfonyl]amino]-3-[[2-(1-methylethyl)-1,4-dioxo-4-[(2-phenoxyethyl)amino]butyl]amino]-4-oxopentanoate (1.38 g, 1.77 mmol, Example 4, Step D) and 10 mL of trifluoroacetic acid in 20 mL of $CH_2Cl_2$ is stirred at room temperature for 1 hour. The solution is concentrated to a foamy solid. The solid is dissolved into 0.1 M sodium hydroxide solution and washed with EtOAc (25 mL). The basic aqueous phase (pH ~10-11) is acidified with saturated $KH_2PO_4$ solution to a pH ~5.0 and extracted with EtOAc. The organic extract is washed with brine solution, dried ($MgSO_4$), filtered, and concentrated to give 0.57 g (52%) of 5-(7,7-dimethyl-2-oxo-bicyclo[2.2.1]hept-1-ylmethanesulfonylamino)-3-{3-methyl-2-[(2-phenoxy-ethylcarbamoyl)-methyl]-butyrylamino}-4-oxo-pentanoic acid as a foamy white solid (Compound 9).
MS (APCI) m/z 622.1 (M+1, 40.3%) and 362.1 (100%).

| Analysis Calcd for $C_{30}H_{43}N_3O_9S$ (621.756): | | | |
|---|---|---|---|
| | C, 57.95; | H, 6.97; | N, 6.76. |
| Found | C, 57.63; | H, 7.15; | N, 6.59. |

The following compounds can be prepared according to the procedure of Example 9.

EXAMPLE 10

**3-[3-Methyl-2-(phenethylcarbamoyl-methyl)-butyrylamino]-4-oxo-5-(3-phenyl-propylsulfanyl)-pentanoic acid**

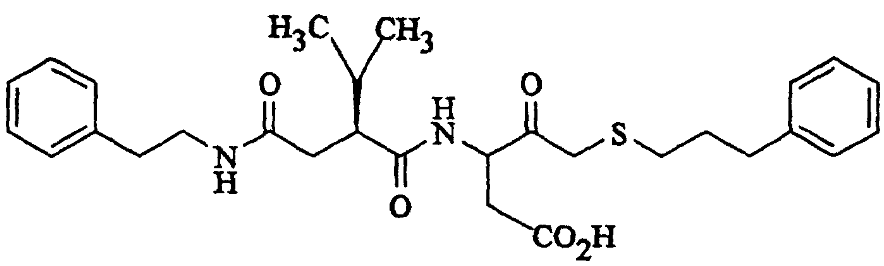

A white solid.
MS (APCI) m/z 527.2 (M+1, 100%).

| Analysis Calcd for $C_{29}H_{38}N_2O_5S$ (526.701): | | | |
|---|---|---|---|
| | C, 66.13; | H, 7.27; | N, 5.32. |
| Found | C, 65.99; | H, 7.24; | N, 5.20. |

EXAMPLE 11

**5-(7,7-Dimethyl-2-oxo-bicyclo[2.2.1]hept-1-ylmethanesulfonylamino)-3-{3-methyl-2-[(methyl-phenethyl-carbamoyl)-methyl]-butyrylamino}-4-oxopentanoic acid**

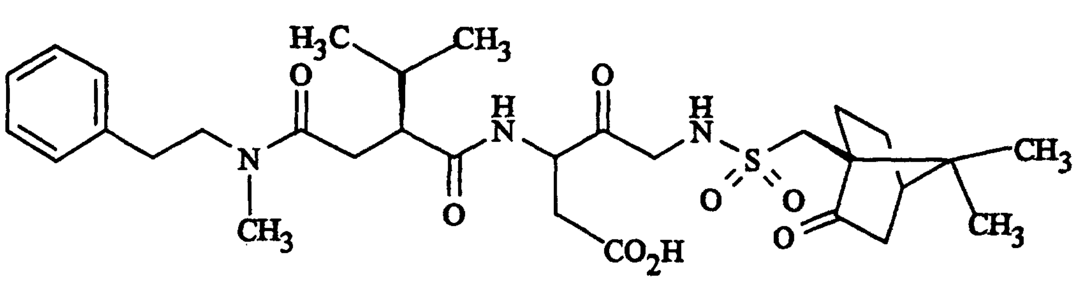

An off-white foamy solid.
MS (APCI) m/z 620.2 (M+1, 40.3%), 360.2 (100%).

| Analysis Calcd $C_{31}H_{45}N_3O_8S \times 0.70\ H_2O$ (632.394): | | | |
|---|---|---|---|
| | C, 58.88; | H, 7.40; | N, 6.64. |
| Found | C, 58.89; | H, 7.48; | N, 6.39. |

EXAMPLE 12

**5-(7,7-Dimethyl-2-oxo-bicyclo[2.2.1]hept-1-ylmethanesulfonylamino)-3-{3-methyl-2-[(3-phenyl-propylcarbamoyl)-methyl]-butyrylamino}-4-oxo-pentanoic acid**

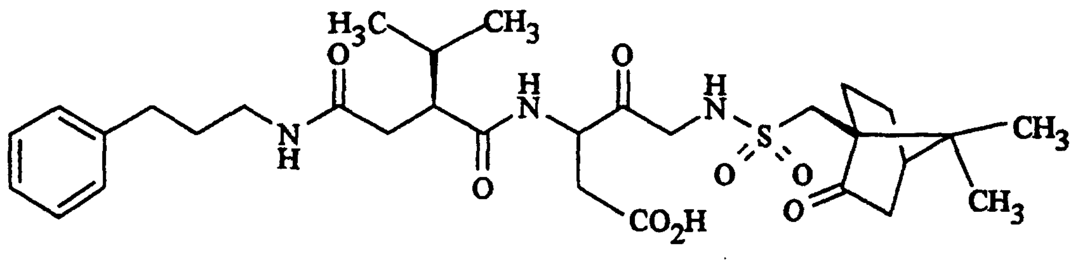

A foamy white solid.
MS (APCI) m/z 620.2 (M+1, 37.7%), 360.2 (100%).

| Calcd for $C_{31}H_{45}N_3O_8S \times 0.50\ H_2O$ (628.791): | | | |
|---|---|---|---|
| | C, 59.22; | H, 7.37; | N, 6.68. |
| Found | C, 59.22; | H, 7.37; | N, 6.50. |

EXAMPLE 13

**3-{3-Methyl-2-[(3-phenyl-propylcarbamoyl)-methyl]-butyrylamino}-4-oxo-5-(2-oxo-2H-chromen-6-yloxy)-pentanoic acid**

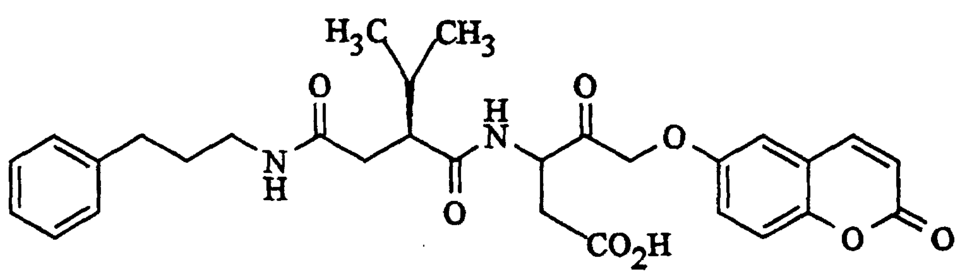

A tan solid.
MS (APCI) m/z 551.2 (M+1, 62.3%), 345.2 (100%).

| Analysis Calcd for $C_{30}H_{34}N_2O_8 \times 0.50\ H_2O$ (559.622): | | | |
|---|---|---|---|
| | C, 64.39; | H, 6.30; | N, 5.01. |
| Found | C, 64.39; | H, 6.18; | N, 5.00. |

EXAMPLE 14

**5-[3-(1H-Imidazol-2-yl)-naphthalen-2-yloxy]-3-{3-methyl-2-[(3-phenyl-propylcarbamoyl)-methyl]-butyrylamino}-4-oxo-pentanoic acid**

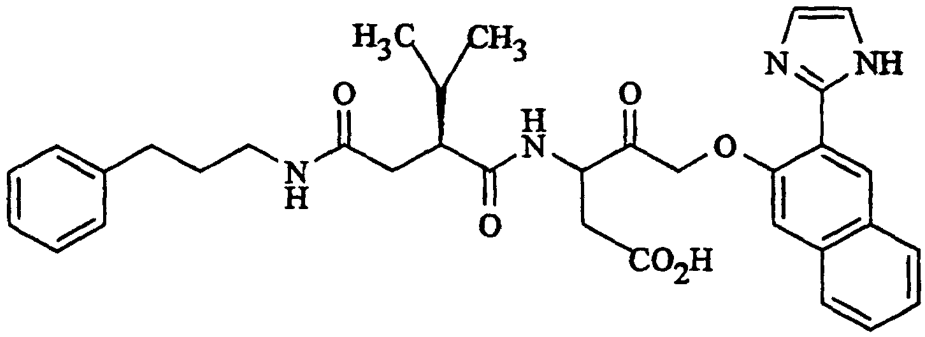

MS (APCI) m/z 599 (M+1, 13%).

| Analysis Calcd for $C_{34}H_{38}N_4O_6 \times CF_3CO_2H \times H_2O$ (730.745): | | | |
|---|---|---|---|
| | C, 59.17; | H, 5.66; | N, 7.67. |
| Found | C, 58.84; | H, 5.61; | N, 7.68. |

EXAMPLE 15

**5-(7,7-Dimethyl-2-oxo-bicyclo[2.2.1]hept-1-ylmethanesulfonylamino)-3-(2-hydroxycarbamoylmethyl-3-methyl-butyrylamino)-4-oxo-pentanoic acid**

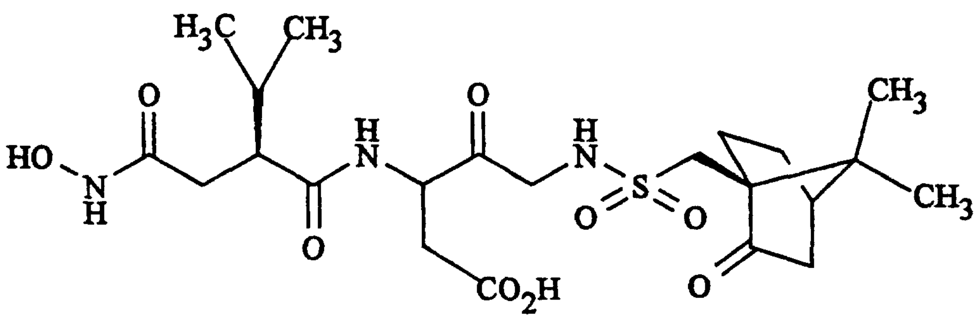

**Step A**

Using the procedure described in Example 2, Step F, [S-(R*,R*)]-2-(2-tert-butoxycarbonylmethyl-3-methyl-butyrylamino)-succinic acid 1-allyl ester 4-benzyl ester (10.68 g, 23.52 mmol, Example 2, Step D) is converted to 7.70 g (78%) of [S-(R*,R*)]-2-(2-tert-butoxycarbonylmethyl-3-methyl-butyrylamino)-succinic acid 4-benzyl ester as a white solid.
MS (APCI) m/z 422.3 (M+1, 12.1%) and 366.2 (M-55, 100%).

| Analysis Calcd for $C_{22}H_{31}NO_7$ (421.495): | | | |
|---|---|---|---|
| | C, 62.69; | H, 7.41; | N, 3.32. |
| Found | C, 62.42; | H, 7.33; | N, 3.17. |

**Step B**

Using the procedure described in Example 1, Step D, [S-(R*,R*)]-2-(2-tert-butoxycarbonylmethyl-3-methyl-butyrylamino)-succinic acid 4-benzyl ester (6.08 g, 14.43 mmol, Example 4, Step A) is converted to 4.62 g (64%) of [S-(R*,R*)]-5-bromo-3-(2-tert-butoxycarbonylmethyl-3-methyl-butyrylamino)-4-oxo-pentanoic acid benzyl ester as a white solid.
MS (APCI) m/z 498.2/500.2 (M+1, 76.5/74.7%) and 442.2/444.2 (M-55, 100/97.1%).

| Analysis Calcd for $C_{23}H_{32}BrNO_6$ (498.424): | | | |
|---|---|---|---|
| | C, 55.43; | H, 6.47; | N, 2.81. |
| Found | C, 55.28; | H, 6.33; | N, 2.77. |

**Step C**

To a solution of (S)-1,1-dimethylethyl[[(7,7-dimethyl-2-oxobicyclo[2.2.1]hept-1-yl)methyl]sulfonyl]carbamate (0.862 g, 2.60 mmol) in anhydrous DMF (4 mL) is added potassium tert-butoxide (0.307 g, 2.74 mmol). The reaction is stirred under a nitrogen atmosphere for 30 minutes. This solution is then added dropwise to a solution of [S-(R*,R*)]-5-bromo-3-(2-tert-butoxycarbonylmethyl-3-methyl-butyrylamino)-4-oxo-pentanoic acid benzyl ester (1.18 g, 2.37 mmol, Example 5, Step B) in anhydrous DMF (4 mL) and the reaction is stirred overnight. The sample is diluted with EtOAc and washed with brine (2×). The EtOAc layer is dried ($Na_2SO_4$) and concentrated. The residue is chromatographed (silica gel, 20% EtOAc in hexanes) to afford 0.850 g of phenylmethyl-5-[[(1,1-dimethylethoxy)carbonyl]-[[(7,7-dimethyl-2-oxobicyclo[2.2.1]hept-1-yl)methyl]sulfonyl]amino]-3-[[4-(1,1-dimethylethoxy)-2-(1-methylethyl)-1,4-dioxobutyl]amino]-4-oxopentanoate as a white foamy solid.

**Step D**

Phenylmethyl-5-[[(1,1-dimethylethoxy)carbonyl]-[[(7,7-dimethyl-2-oxobicyclo[2.2.1]hept-1-yl)methyl]sulfonyl]amino]-3-[[4-(1,1-dimethylethoxy)-2-(1-methylethyl)-1,4-dioxobutyl]amino]-4-oxopentanoate (0.850 g, 1.15 mmol, Example 5, Step C) is treated with trifluoroacetic acid/$CH_2Cl_2$ (1:1, 15 mL) for 1 hour. The sample is concentrated to give an oily residue which is carried on to the next step without further purification. The residue is dissolved into 20 mL of $CH_2Cl_2$ and treated with EDCI·HCl (0.441 g, 2.3 mmol), HOBT·$H_2O$ (0.311 g, 2.3 mmol) and O-benzylhydroxylamine hydrochloride (0.276 g, 1.73 mmol). 4-Methylmorpholine (0.506 g, 5.0 mmol) is added dropwise, and the reaction mixture is stirred at room temperature overnight. The sample is diluted with EtOAc and washed successively with 5% HCl and saturated $NaHCO_3$ solutions. The organic extract is dried ($Na_2SO_4$) and concentrated. The residue is chromatographed (silica gel, 10% EtOAc in hexanes) to afford 0.400 g of 3-[2-(benzyloxycarbamoyl-methyl)-3-methyl-butyrylamino]-5-(7,7-dimethyl-2-oxo-bicyclo[2.2.1]hept-1-ylmethanesulfonylamino)-4-oxo-pentanoic acid benzyl ester as a white solid.

**Step E**

To a solution of 3-[2-(benzyloxycarbamoyl-methyl)-3-methyl-butyrylamino]-5-(7,7-dimethyl-2-oxo-bicyclo[2.2.1]hept-1-ylmethanesulfonylamino)-4-oxo-pentanoic acid benzyl ester (0.237 g, Example 5, Step D) in 75 mL of THF is added 10% Pd/C (0.50 g), and the mixture is hydrogenated at 50 psi hydrogen, room temperature for 5 hours. The reaction mixture is filtered and concentrated. The residue is partitioned between EtOAc and 0.5N NaOH solution. The aqueous layer is acidified with HCl and extracted with EtOAc. The organic extract is dried ($Na_2SO_4$) and concentrated, and the residue is triturated in ether/hexanes. The solid is collected by filtration and dried to give 5-(7,7-dimethyl-2-oxo-bicyclo[2.2.1]hept-1-ylmethanesulfonylamino)-3-(2-hydroxycarbamoylmethyl-3-methyl-butyrylamino)-4-oxo-pentanoic acid (Compound 15).
MS (APCI) m/z 518.1 (M+1, 100%).

| Analysis Calcd for $C_{22}H_{35}N_3O_9S \times 0.45\ C_4H_8O_2 \times 0.59\ H_2O$ (567.880): | | | |
|---|---|---|---|
| | C, 50.34; | H, 7.06; | N, 7.40. |
| Found | C, 50.34; | H, 6.80; | N, 7.40. |

The following compounds can be prepared according to the procedure described in Example 15.

EXAMPLE 16

**5-(7,7-Dimethyl-2-oxo-bicyclo[2.2.1]hept-1-ylmethanesulfonylamino)-3-(2-{[2-(1H-indol-3-yl)-ethylcarbamoyl]-methyl}-3-methyl-butyrylamino)-4-oxo-pentanoic acid**

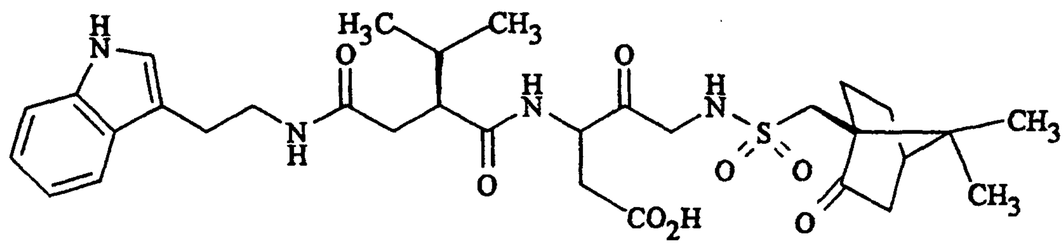

A white foamy solid.
MS (APCI) m/z 645.3 (M+1, 79.2%) and 285.1 (100%).

| Analysis Calcd for $C_{32}H_{44}N_4O_8S \times 0.85\ H_2O$ (660.107): | | | |
|---|---|---|---|
| | C, 58.23; | H, 6.98; | N, 8.49. |
| Found | C, 58.23; | H, 6.74; | N, 8.26. |

EXAMPLE 17

**5-(7,7-Dimethyl-2-oxo-bicyclo[2.2.1]hept-1-ylmethanesulfonylamino)-3-{3-methyl-2-[[3-(4-hydroxyphenyl)-propylcarbamoyl]-methyl]-butyrylamino}-4-oxo-pentanoic acid**

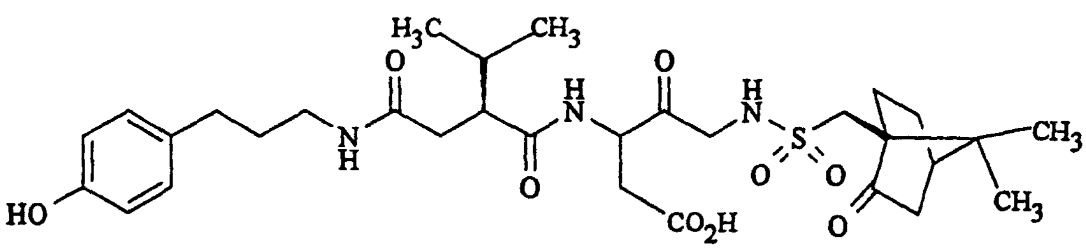

An off-white solid.
MS (APCI) m/z 636.2 (M+1, 36.1%) and 376.1 (100%).

| Analysis Calcd for $C_{31}H_{45}N_3O_9S \times 0.40\ H_2O$ (642.989): | | | |
|---|---|---|---|
| | C, 57.91; | H, 7.18; | N, 6.54. |
| Found | C, 58.10; | H, 7.46; | N, 6.14. |

In a process analogous to Example 2 using appropriate starting materials, the corresponding compounds (Examples 18 to 20) can be prepared.

EXAMPLE 18

**5-(7,7-Dimethyl-2-oxo-bicyclo[2.2.1]hept-1-ylmethanesulfonylamino)-3-{3-methyl-2-[(2-naphthalen-2-yl-ethylcarbamoyl)-methyl]-butyrylamino}-4-oxo-pentanoic acid**

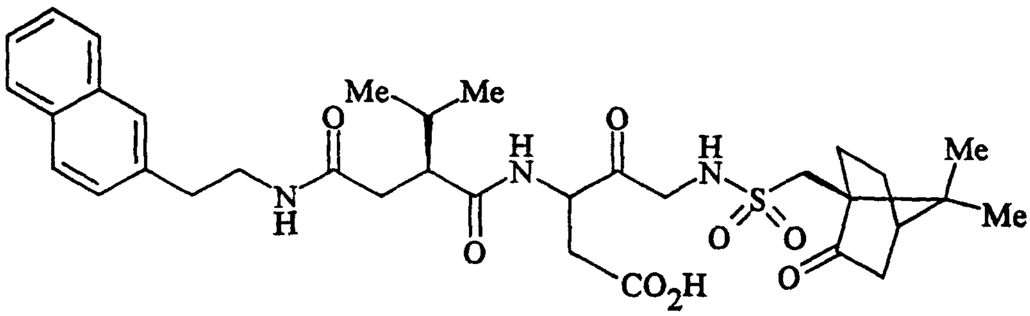

A white solid.
MS (APCI) *m/z* 656.2 (M+1, 31.2%), 217.1 (100%).

| Analysis Calcd for $C_{34}H_{45}NO_8S \times 0.90\ H_2O$ (672.031): | | | |
|---|---|---|---|
| | C, 60.77; | H, 7.02; | N, 6.25. |
| Found | C, 60.80; | H, 6.95; | N, 5.93. |

EXAMPLE 19

**5-(7,7-Dimethyl-2-oxo-bicyclo[2.2.1]hept-1-ylmethanesulfonylamino)-3-(3-methyl-2-{(2-(7-methyl-1H-indol-3-yl)-ethylcarbamoyl]-methyl}-butyrylamino)-4-oxo-pentanoic acid**

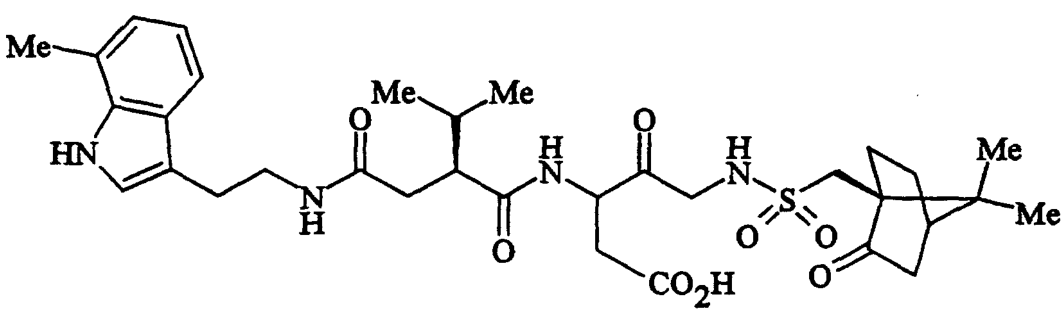

A pink solid.
MS (APCI) *m/z* 659.3 (M+1, 31.2%), 399.2 (100%).

| Analysis Galcd for $C_{33}H_{46}N_4O_8S \times 0.20\ H_2O$ (662.424): | | | |
|---|---|---|---|
| | C, 59.84; | H, 7.06; | N, 8.46. |
| Found | C, 59.98; | H, 7.35; | N, 8.08. |

EXAMPLE 20

**5-(7,7-Dimethyl-2-oxo-bicyclo[2.2.1]hept-1-ylmethanesulfonylamino)-3-(2-{[2-(6-fluoro-1H-indol-3-yl)-ethylcarbamoyl]-methyl}-3-methyl-butyrylamino)-4-oxo-pentanoic acid**

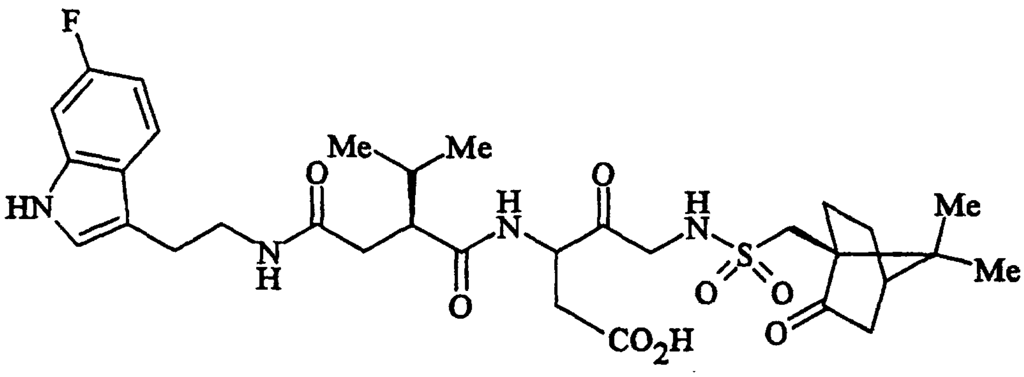

A foamy off-white solid.
MS (APCI) *m/z* 663.3 (M+1, 14.1%), 403.2 (100%).

| Analysis Calcd for $C_{32}H_{43}FN_4O_8S \times 0.90\ H_2O$ (678.998): | | | |
|---|---|---|---|
| | C, 56.61; | H, 6.65; | N, 8.25. |
| Found | C, 56.92; | H, 6.78; | N, 7.86. |

In a process analogous to Example 7 using appropriate starting materials, the corresponding compounds (Examples 21 to 23) can be prepared.

EXAMPLE 21

**3-[3-Methyl-2-({methyl-[2-(1-methyl-1H-indol-3-yl)-ethyl]-carbamoyl}-methyl)-butyrylamino]-4-oxo-butyric acid**

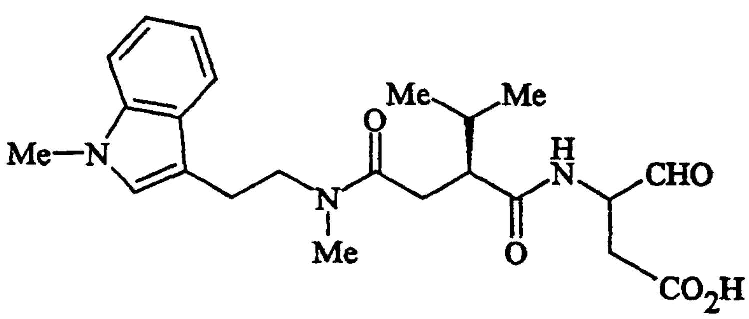

A foamy off-white solid.
MS (APCI) *m/z* 428.3 (M-1, 100%).

| Analysis Calcd for $C_{23}H_{31}N_3O_5 \times 0.35\ CF_3CO_2H$ (469.429): | | | |
|---|---|---|---|
| | C, 60.64; | H, 6.73; | N, 8.95. |
| Found | C, 60.46; | H, 6.93; | N, 8.78. |

EXAMPLE 22

**3-(3-Methyl-2-{[methyl-(2-phenoxy-ethyl)-carbamoyl]-methyl}-butyrylamino)-4-oxo-butyric acid**

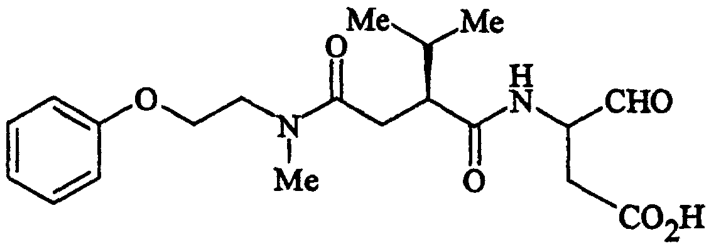

A foamy white solid.
MS (APCI) *m/z* 393.2 (M+1, 100%).

| Analysis Calcd for $C_{20}H_{28}N_2O_6 \times 0.85\ H_2O$ (407.769): | | | |
|---|---|---|---|
| | C, 58.91; | H, 7.34; | N, 6.87. |
| Found | C, 58.87; | H, 7.02; | N, 6.59. |

EXAMPLE 23

**3-(2-{[2-(5,6-Dimethyl-benzoimidazol-1-yl)-ethylcarbamoyl]-methyl}-3-methyl-butyrylamino)-4-oxo-butyric acid, trifluoroacetate salt**

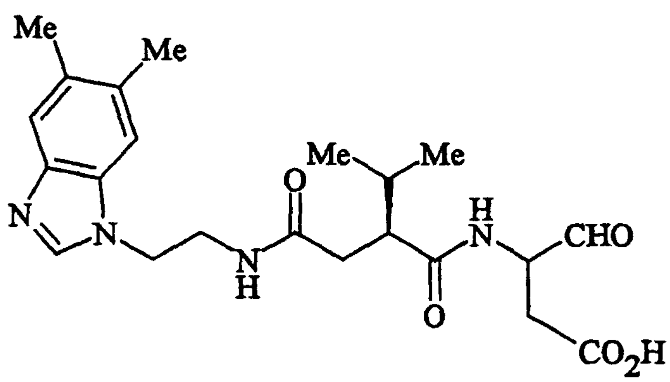

A white solid.
MS (APCI) *m/z* 431.1 (M+1, 35.7%), 190.2 (100%).

| Analysis Calcd for $C_{22}H_{30}N_4O_5 \times 1.30\ CF_3CO_2H$ (578.740): | | | |
|---|---|---|---|
| | C, 51.06; | H, 5.45; | N, 9.68. |
| Found | C, 51.03; | H, 5.50; | N, 9.38. |

In a process analogous to Example 9 using appropriate starting materials, the corresponding compounds (Examples 24 to 25) can be prepared.

EXAMPLE 24

**5-(7,7-Dimethyl-bicyclo[2.2.1]hept-1-ylmethanesulfonylamino)-3-{3-methyl-2-[(3-phenyl-propylcarbamoyl)-methyl]-butyrylamino}-4-oxo-pentanoic acid**

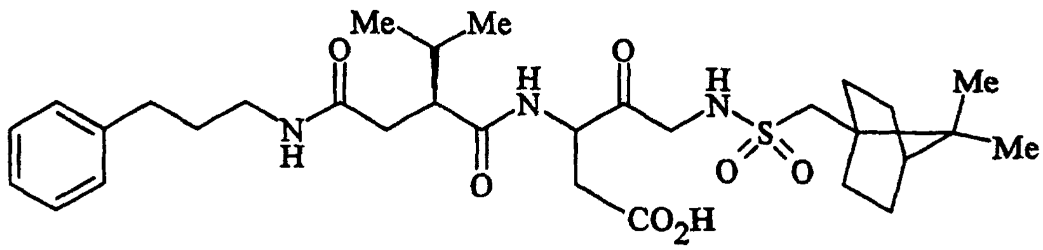

A off-white solid.
MS (APCI) *m/z* 606.1 (M+1), 360.1 (100%).

| Analysis Calcd for $C_{31}H_{47}N_3O_7S \times 0.25\ CF_3CO_2H$ (634.306): | | | |
|---|---|---|---|
| | C, 59.65; | H, 7.51; | N, 6.62. |
| Found | C, 59.73; | H, 7.67; | N, 6.27. |

EXAMPLE 25

**5-(7,7-Dimethyl-bicyclo[2.2.1]hept-1-ylmethanesulfonylamino)-3-(2-{[2-(1H-indol-3-yl)-ethylcarbamoyl]-methyl}-3-methyl-butyrylamino)-4-oxo-pentanoic acid**

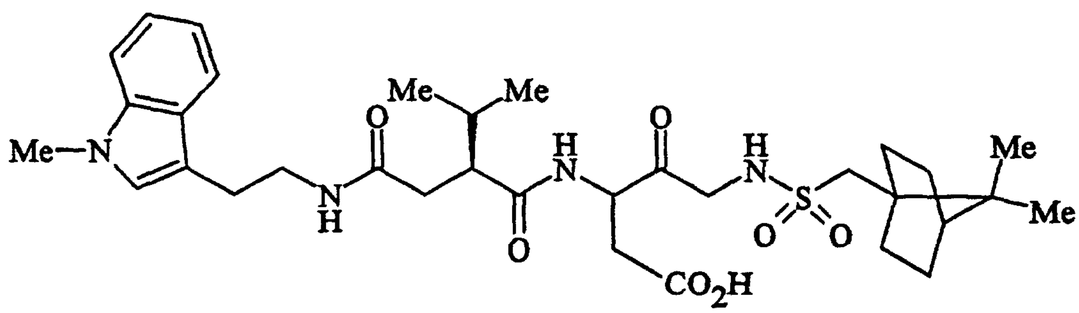

A tan solid.
MS (APCI) *m/z* 645.2 (M+1), 399.1 (100%).

| Analysis Calcd for $C_{33}H_{48}N_4O_7S \times 0.34\ CF_3CO_2H$ (683.605): | | | |
|---|---|---|---|
| | C, 59.18; | H, 7.13; | N, 8.20. |
| Found | C, 59.34; | H, 7.13; | N, 7.81. |

In a process analogous to Example 15 using appropriate starting materials, the corresponding compounds (Examples 26 to 34) can be prepared.

EXAMPLE 26

**5-(7,7-Dimethyl-2-oxo-bicyclo[2.2.1]hept-1-ylmethanesulfonylamino)-3-{3-methyl-2-[(3-pyridin-4-yl-propylcarbamoyl)-methyl]-butyrylamino}-4-oxo-pentanoic acid**

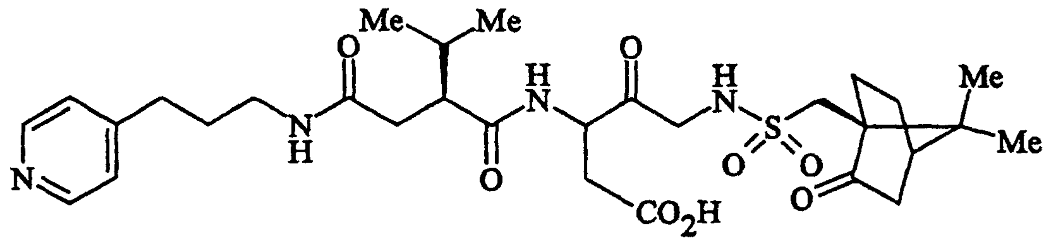

An off-white solid.
MS (APCI) *m/z* 621.1 (M+1, 5.1%), 261.1 (100%).

| Analysis Calcd for $C_{30}H_{44}N_4O_8S \times 1.26\ CF_3CO_2H$ (764.442): | | | |
|---|---|---|---|
| | C, 51.10; | H, 5.97; | N, 7.33. |
| Found | C, 51.06; | H, 6.06; | N, 7.08. |

EXAMPLE 27

**5-(7,7-Dimethyl-2-oxo-bicyclo[2.2.1]hept-1-ylmethanesulfonylamino)-3-{3-methyl-2-[(3-quinolin-2-yl-propylcarbamoyl)-methyl]-butyrylamino}-4-oxo-pentanoic acid**

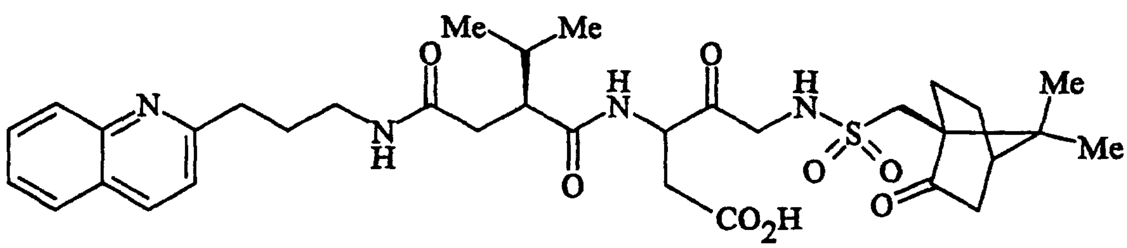

An off-white solid.
MS (APCI) *m/z* 671.3 (M+1, 70.5%), 158.1 (100%).

| Analysis Calcd for $C_{34}H_{46}N_4O_8S \times 0.95\ H_2O$ (687.946): | | | |
|---|---|---|---|
| | C, 59.36; | H, 7.02; | N, 8.14. |
| Found | C, 59.60; | H, 6.71; | N, 7.75. |

EXAMPLE 28

**5-(7,7-Dimethyl-2-oxo-bicyclo[2.2.1]hept-1-ylmethanesulfonylamino)-3-{3-methyl-2-[(3-naphthalen-1-yl-propylcarbamoyl)-methyl]-butyrylamino}-4-oxo-pentanoic acid**

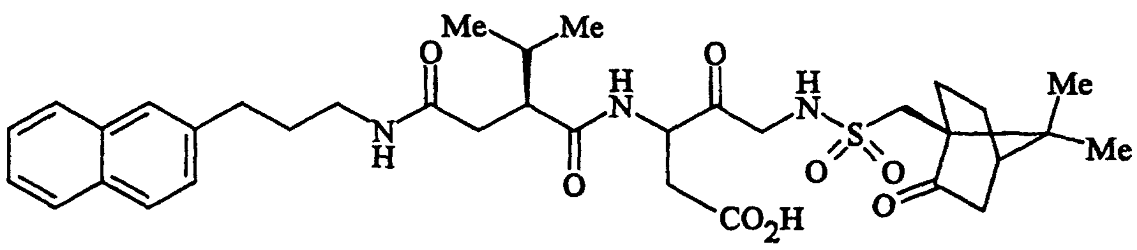

An off-white solid.
MS (APCI) *m/z* 670.3 (M+1, 79.2%), 410.2 (100%).

| Analysis Calcd for $C_{35}H_{47}N_3O_8S \times 0.90$ EtOAc (749.141): | | | |
|---|---|---|---|
| | C, 61.89; | H, 7.29; | N, 5.61. |
| Found | C, 61.90; | H, 7.28; | N, 5.57. |

EXAMPLE 29

**5-(7,7-Dimethyl-2-oxo-bicyclo[2.2.1]hept-1-ylmethanesulfonylamino)-3-{3-methyl-2-[(3-pyridin-3-yl-propylcarbamoyl)-methyl]-butyrylamino}-4-oxo-pentanoic acid**

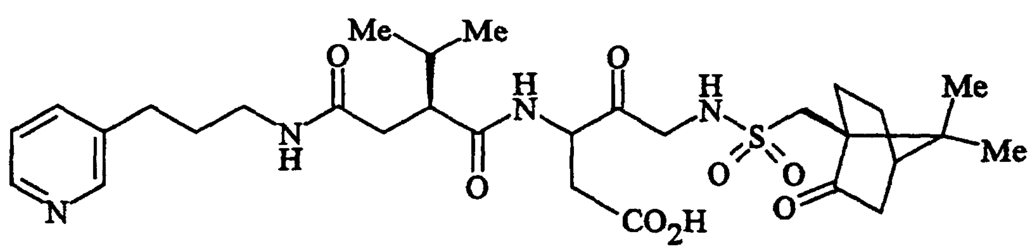

An off-white solid.
MS (APCI) *m/z* 621.1 (M+1, 6.5%), 261.1 (100%).

| Analysis Calcd for $C_{30}H_{44}N_4O_8S \times 1.20$ $CF_3CO_2H$ (757.600): | | | |
|---|---|---|---|
| | C, 51.37; | H, 6.23; | N, 7.34. |
| Found | C, 51.37; | H, 6.01; | N, 7.40. |

EXAMPLE 30

**3-{2-[(2-Benzoimidazol-1-yl-ethylcarbamoyl)-methyl]-3-methyl-butyrylamino}-5-(7,7-dimethyl-2-oxo-bicyclo [2.2.1]hept-1-ylmethanesulfonylamino)-4-oxo-pentanoic acid**

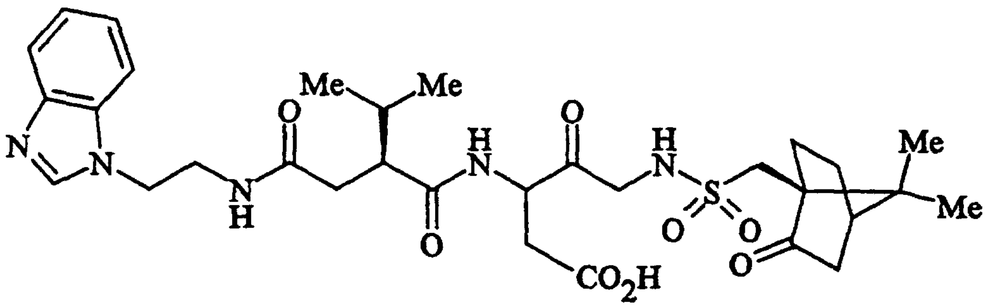

A foamy white solid.
MS (APCI) *m/z* 646.2 (M+1, 10.4%), 286.1 (100%).

| Analysis Calcd for $C_{31}H_{43}N_5O_8S \times 1.46\ CF_3CO_2H$ (812.256): | | | |
|---|---|---|---|
| | C, 50.16; | H, 5.52; | N, 8.62. |
| Found | C, 50.18; | H, 5.73; | N, 8.46. |

EXAMPLE 31

**5-(7,7-Dimethyl-2-oxo-bicyclo[2.2.1]hept-1-ylmethanesulfonylamino)-3-(3-methyl-2-{[2-(1-methyl-1H-indol-3-yl)-ethylcarbamoyl]-methyl}-butyrylamino)-4-oxo-pentanoic acid**

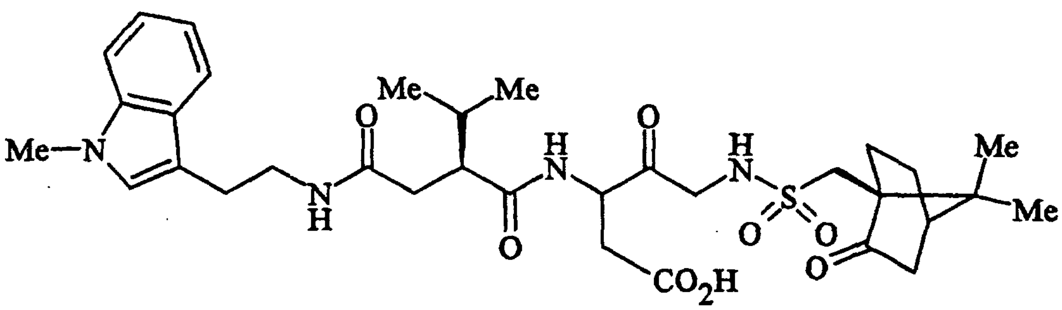

A white solid.
MS (APCI) *m/z* 659.2 (M+1, 41.6%), 399.1 (100%).

| Analysis Calcd for $C_{33}H_{46}N_4O_8S \times 0.17\ CF_3CO_2H$ (678.205): | | | |
|---|---|---|---|
| | C, 59.05; | H, 6.86; | N, 8.26: |
| Found | C, 59.07; | H, 6.95; | N, 7.98. |

EXAMPLE 32

**5-(7,7-Dimethyl-2-oxo-bicyclo[2.2.1]hept-1-ylmethanesulfonylamino)-3-{3-methyl-2-[(2-pyridin-4-yl-ethylcarbamoyl)-methyl]-butyrylamino}-4-oxo-pentanoic acid**

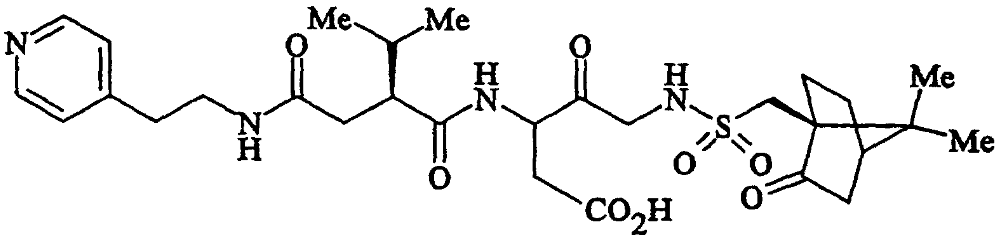

A white solid.
MS (APCI) *m/z* 608.2 (M+2, 64.9%), 607.2 (M+1, 54.5%), 347.2 (100%).

| Analysis Calcd for $C_{29}H_{42}N_4O_8S \times 1.66\ CF_3CO_2H$ (796.024): | | | |
|---|---|---|---|
| | C, 48.77; | H, 5.53; | N, 7.04. |
| Found | C, 49.12; | H, 5.43; | N, 6.60. |

EXAMPLE 33

**3-(2-{[2-(5-Acetyl-1H-indol-3-yl)-ethylcarbamoyl]-methyl}-3-methyl-butyrylamino)-5-(7,7-dimethyl-2-oxo-bicyclo[2.2.1]hept-1-ylmethanesulfonylamino)-4-oxo-pentanoic acid**

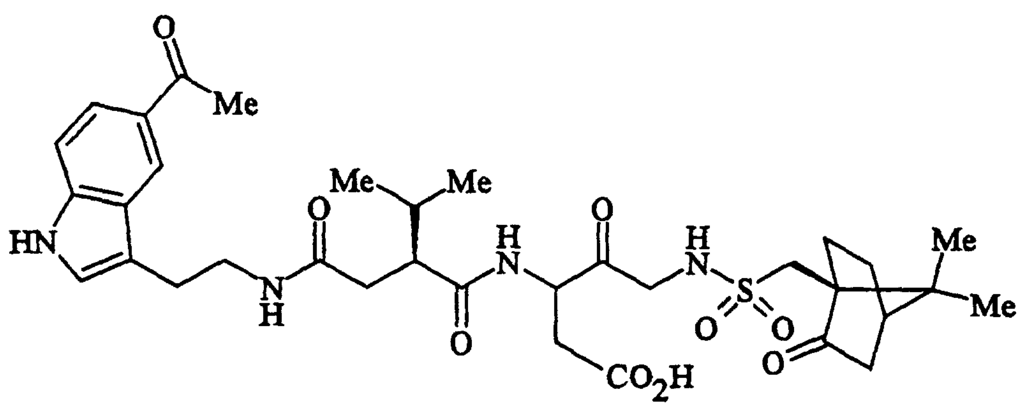

A white solid.
MS (APCI) *m/z* 687.3 (M+1, 18.7%), 327.2 (100%).

| Analysis Calcd for $C_{34}H_{46}N_4O_9S \times 0.75\ H_2O$ (700.343): | | | |
|---|---|---|---|
| | C, 58.31; | H, 6.84; | N, 8.00. |
| Found | C, 58.51; | H, 6.88; | N, 7.61. |

EXAMPLE 34

**5-(7,7-Dimethyl-2-oxo-bicyclo[2.2.1]hept-1-ylmethanesulfonylamino)-3-(3-methyl-2-{[2-(1H-tetrazol-5-yl)-ethylcarbamoyl]-methyl}-butyrylamino)-4-oxo-pentanoic acid**

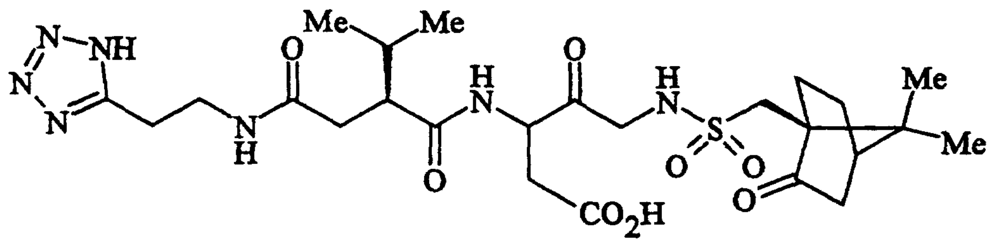

A gray solid.
MS (APCI) *m/z* 596.3 (M-1, 24.7%), 113.2 (100%).

| Analysis Calcd for $C_{25}H_{39}N_7O_8S \times CF_3CO_2H \times 0.27\ Et_2O$ (731.733): | | | |
|---|---|---|---|
| | C, 46.09; | H, 5.88; | N, 13.40. |
| Found | C, 46.08; | H, 5.78; | N, 13.37. |

EXAMPLE 35

**$N^4$-(2-Benzoimidazol-1-yl-ethyl)-$N^1$-(2-ethoxy-5-oxo-tetrahydro-furan-3-yl)-2-isopropyl-succinamide**

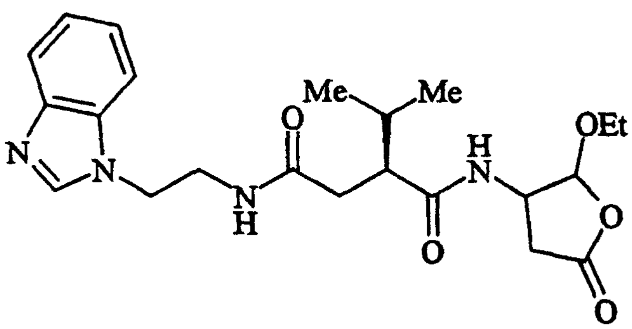

**Step A**

A solution of N-benzyloxycarbonyl-3-amino-4-oxo-butanoic acid β-tert-butyl ester diethyl acetal (24.1 g, 63.2 mmol, prepared from Cbz-Asp(OtBu)-OH following the procedure of Chapman K.T. (*Bioorganic &Medicinal Chemistry Letters* 1992; 2:613-618) in 150 mL of ethanol was treated with 1.0 g of 20% Pd/C. The sample was hydrogenated at room temperature and 51 psig $H_2$ for 3.5 hours. The sample was filtered and concentrated to give 15.8 g (~100%) of 3-amino-4-oxo-butanoic acid β-tert-butyl ester diethyl acetal as a yellow liquid.
MS (APCI) *m/z* 248.1 (M+1, 100%).

**Step B**

A mixture of 3-amino-4-oxo-butanoic acid β-tert-butyl ester diethyl acetal (15.8 g, 63.9 mmol, Example 35, Step A), (S)-2-isopropyl-succinic acid 4-tert-butyl ester (15.2 g, 70.3 mmol, Example 2, Step C), EDCI × HCl (13.5 g, 70.4 mmol), HOBT × $H_2O$ (10.8 g, 70.5 mmol) and 4-methylmorpholine (8.8 mL, 80.0 mmol) in 250 mL of $CH_2Cl_2$ was

stirred at room temperature for 12 hours. The solution was concentrated, then partitioned between EtOAc and saturated $NaHCO_3$ solution. The organic extract was washed with sat. $KH_2PO_4$ and brine solutions, dried ($MgSO_4$), filtered, and concentrated. The resultant dark brown oil was chromatographed (MPLC, silica gel, 100% $CH_2Cl_2$ to 2% MeOH in $CH_2Cl_2$) to give 13.9 g (49%) of 3-(2-tert-butoxycarbonylmethyl-3-methyl-butyrylamino)-4-oxo-butanoic acid β-tert-butyl ester diethyl acetal as a light yellow oil.
MS (APCI) *m/z* 224.1 (100%).

**Step C**

A solution of 3-(2-tert-butoxycarbonylmethyl-3-methyl-butyrylamino)-4-oxo-butanoic acid β-tert-butyl ester diethyl acetal (8.3 g, 18.6 mmol, Example 35, Step B) in 100 mL of $CH_2Cl_2$ was treated with 20 mL of trifluoroacetic acid. The solution was stirred at room temperature for 2 hours, concentrated, then partitioned between EtOAc and saturated $KH_2PO_4$ solution. The organic extract was washed with brine solution, dried ($MgSO_4$), filtered, and concentrated to give 3.8 g (71%) of N-(2-ethoxy-5-oxo-tetrahyrdo-furan-3-yl)-2-isopropyl-succinamic acid as a yellow oil.
MS (APCI) *m/z* 288.1 (M+1, 100%).

**Step D**

A mixture of N-(2-ethoxy-5-oxo-tetrahyrdo-furan-3-yl)-2-isopropyl-succinamic acid (3.8 g, 13.1 mmol, Example 35, Step C), 1-(2-aminoethyl)benzimidazole (3.2 g, 19.8 mmol, prepared from benzimidazole using the procedure of Cuadro A.M. et al., *Synthetic Communications*, 1991;21:535-544), HOBT × $H_2O$ (2.4 g, 15.8 mmol), EDCI × HCl (3.0 g, 15.8 mmol) and N-methylmorpholine (2.2 mL, 20.0 mmol) in 100 mL of $CH_2Cl_2$ was stirred at room temperature for 12 hours. The solution was concentrated, then partitioned between EtOAc and sat. $NaHCO_3$ solution. The organic extract was washed with saturated $KH_2PO_4$ and brine solutions, dried ($MgSO_4$), filtered, and concentrated. The resultant tan oily solid was chromatographed (MPLC, silica gel, 5% MeOH in $CH_2Cl_2$) to give 2.8 g (49%) of $N^4$-(2-benzoimidazol-1-yl-ethyl)-$N^1$-(2-ethoxy-5-oxo-tetrahydro-furan-3-yl)-2-isopropyl-succinamide as a white foamy solid.
MS (APCI) *m/z* 432.2 (M+2, 100%).

| Analysis Calcd for $C_{22}H_{30}N_4O_5$ (430.508): | | | |
|---|---|---|---|
| | C, 61.38; | H, 7.02; | N, 13.01. |
| Found | C, 61.18; | H, 6.95; | N, 13.05. |

In a process analogous to Example 35 using appropriate starting materials, the corresponding compounds (Examples 36 to 52) can be prepared. The starting 2-aryl-ethylamines that were not commercially available were prepared following the procedure of Cuadro A.M. et al., *Synthetic Communications* 1991;21:535-544.

EXAMPLE 36

**$N^1$-(2-Ethoxy-5-oxo-tetrahydro-furan-3-yl)-$N^4$-[2-(1H-indol-3-yl)-ethyl]-2-isopropyl-succinamide**

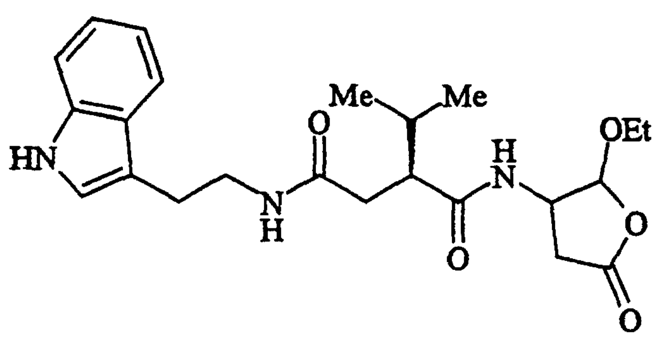

An off-white solid.
MS (APCI) *m/z* 430.3 (M+1, 100%).

| Analysis Calcd for $C_{23}H_{31}N_3O_5 \times 0.25\ H_2O$ (434.024): | | | |
|---|---|---|---|
| | C, 63.65; | H, 7.32; | N, 9.68. |
| Found | C, 63.68; | H, 7.34; | N, 9.36. |

EXAMPLE 37

**N¹-(2-Ethoxy-5-oxo-tetrahydro-furan-3-yl)-2-isopropyl-N⁴-[2-(1-methyl-1H-indol-3-yl)-ethyl]-succinamide**

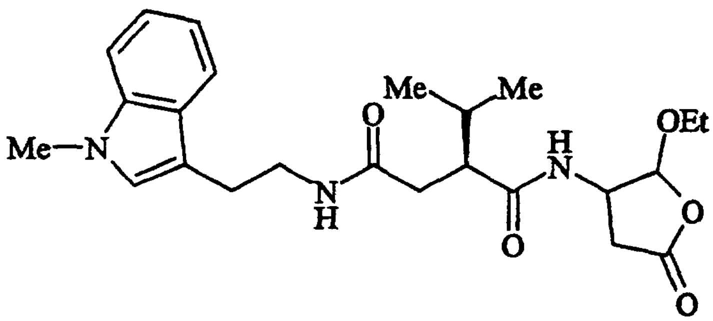

An off-white solid.
MS (APCI) *m/z* 444.3 (M+1, 53.5%), 299.2 (100%).

| Analysis Calcd for $C_{24}H_{33}N_3O_5$ (443.548): | | | |
|---|---|---|---|
| | C 64.99; | H, 7.50; | N, 9.47. |
| Found | C, 65.16; | H, 7.47; | N, 9.40. |

EXAMPLE 38

**N⁴-[2-(5,6-Dichloro-benzoimidazol-1-yl)-ethyl]-N¹-(2-ethoxy-5-oxo-tetrahydro-furan-3-yl)-2-isopropyl-succinamide**

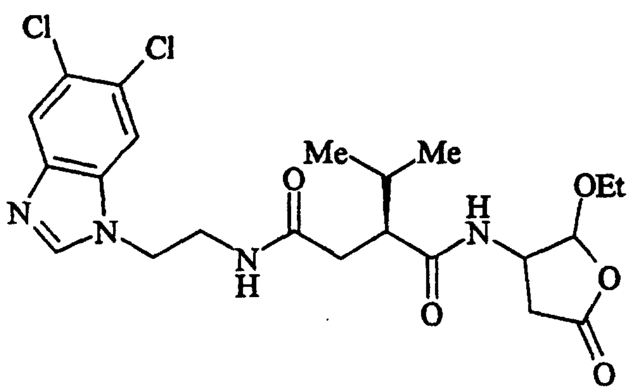

A white solid.
MS (APCI) *m/z* 354.1 (100%).

| Analysis Calcd for $C_{22}H_{28}Cl_2N_4O_5$ (499.398): | | | |
|---|---|---|---|
| | C, 52.91; | H, 5.65; | N, 11.22. |
| Found | C, 52.91; | H, 5.70; | N, 11.07. |

EXAMPLE 39

**$N^1$-(2-Ethoxy-5-oxo-tetrahydro-furan-3-yl)-2-isopropyl-$N^4$-(2-phenoxyethyl)-succinamide**

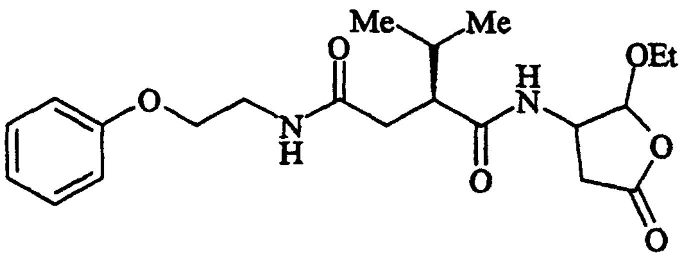

A white solid.
MS (APCI) *m/z* 407.2 (M+1, 66.2%), 262.1 (100%).

| Analysis Calcd for $C_{21}H_{30}N_2O_6$ (406.483): | | | |
|---|---|---|---|
| | C, 62.05; | H, 7.44; | N, 6.89. |
| Found | C, 62.29; | H, 7.49; | N, 6.74. |

EXAMPLE 40

**$N^1$-(2-Ethoxy-5-oxo-tetrahydro-furan-3-yl)-2-isopropyl-$N^4$-[2-(6-methoxy-1H-indol-3-yl)-ethyl]-succinamide**

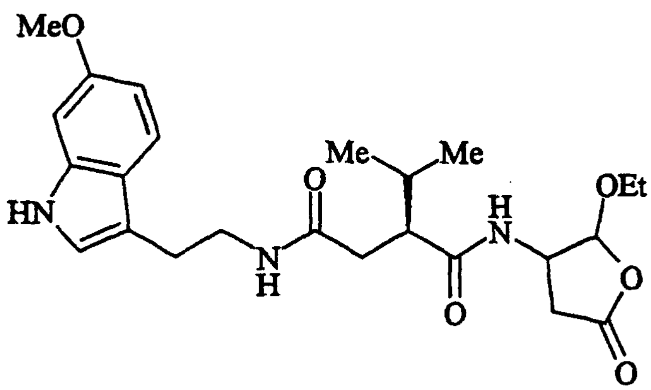

An off-white solid.
MS (APCI) *m/z* 460.1 (M+1, 100%).

| Analysis Calcd for $C_{24}H_{33}N_3O_6$ (459.547): | | | |
|---|---|---|---|
| | C, 62.73; | H, 7.24; | N, 9.14. |
| Found | C, 63.11; | H, 7.02; | N, 9.11. |

EXAMPLE 41

**$N^4$-(2-Benzotriazol-1-yl-ethyl)-$N^1$-(2-ethoxy-5-oxo-tetrahydro-furan-3-yl)-2-isopropyl-succinamide**

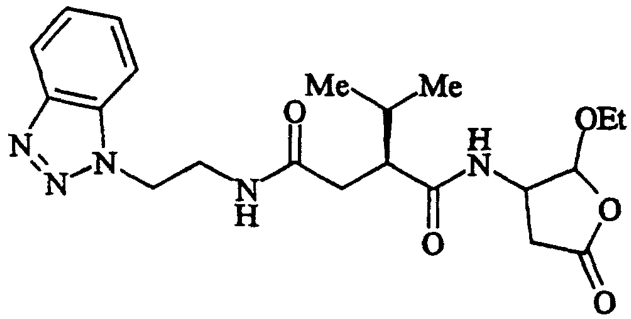

A yellow solid.
MS (APCI) *m/z* 432.1 (M+1, 100%).

| Analysis Calcd for $C_{21}H_{29}N_5O_5$ (431.496): | | | |
|---|---|---|---|
| | C, 58.46; | H, 6.77; | N, 16.23. |
| Found | C, 56.96; | H, 6.45; | N, 15.64. |

EXAMPLE 42

**$N^1$-(2-Ethoxy-5-oxo-tetrahydro-furan-3-yl)-$N^4$-(2-indazol-1-yl-ethyl)-2-isopropyl-succinamide**

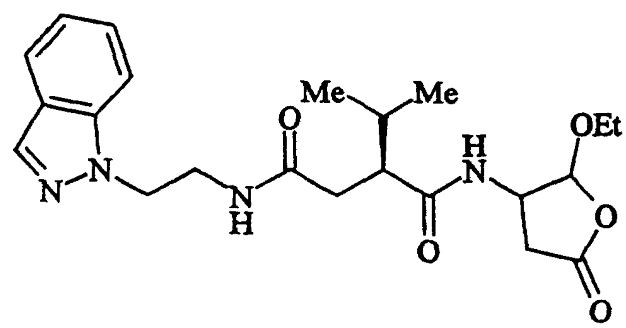

A yellow solid.
MS (APCI) *m/z* 431.2 (M+1, 100%).

| Analysis Calcd for $C_{22}H_{30}N_4O_5$ (430.508): | | | |
|---|---|---|---|
| | C, 61.38; | H, 7.02; | N, 13.01. |
| Found | C, 60.91; | H, 7.16; | N, 12.81. |

EXAMPLE 43

**N[1]-(2-Ethoxy-5-oxo-tetrahydro-furan-3-yl)-2-isopropyl-N[4]-(2-phenylamino-ethyl)-succinamide**

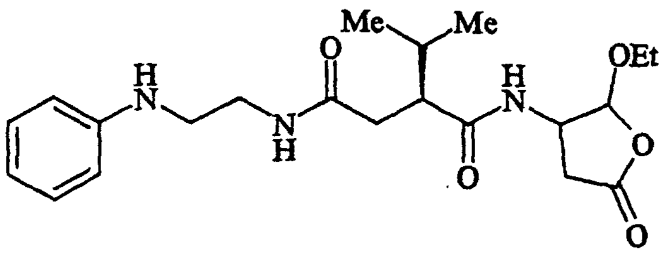

An off-white solid.
MS (APCI) *m/z* 406.1 (M+1, 100%).

| Analysis Calcd for $C_{21}H_{31}N_3O_5 \times 0.15\ H_2O$ (408.201): | | | |
|---|---|---|---|
| | C, 61.79; | H, 7.73; | N, 10.29. |
| Found | C, 61.77; | H, 7.56; | N, 10.20. |

EXAMPLE 44

**N[1]-(2-Ethoxy-5-oxo-tetrahydro-furan-3-yl)-N[4]-[2-(6-fluoro-1H-indol-3-yl)-ethyl]-2-isopropyl-succinamide**

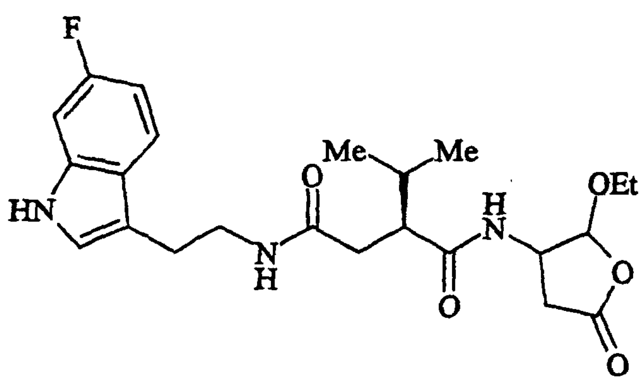

A white solid.
MS (APCI) *m/z* 446.1 (M-1, 100%).

| Analysis Calcd for $C_{23}H_{30}FN_3O_5$ (447.511): | | | |
|---|---|---|---|
| | C, 61.73; | H, 6.76; | N, 9.39. |
| Found | C, 62.22; | H, 6.59; | N, 10.45. |

EXAMPLE 45

**N[1]-(2-Ethoxy-5-oxo-tetrahydro-furan-3-yl)-N[4]-[2-(7-methyl-1H-indol-3-yl)-ethyl]-2-isopropyl-succinamide**

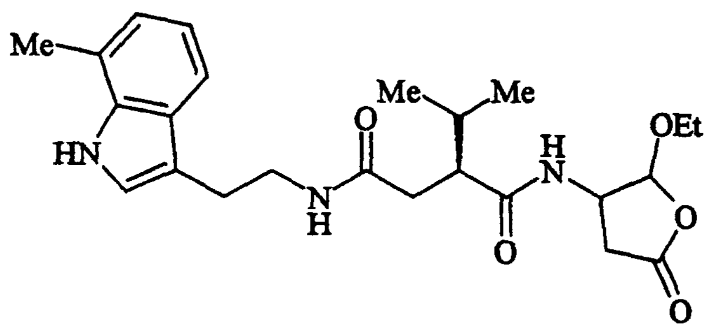

A white solid.
MS (APCI) *m/z* 442.1 (M-1, 100%).

| Analysis Calcd for $C_{24}H_{33}N_3O_5$ (443.548): | | | |
|---|---|---|---|
| | C, 64.99; | H, 7.50; | N, 9.47. |
| Found | C, 65.28; | H, 7.65; | N, 9.46. |

EXAMPLE 46

**N[1]-(2-ethoxy-5-oxotetrahydro-furan-3-yl)-2-isopropyl-N[4]-[2-(2-methyl-benzoimidazol-1-yl)-ethyl]-succinamide**

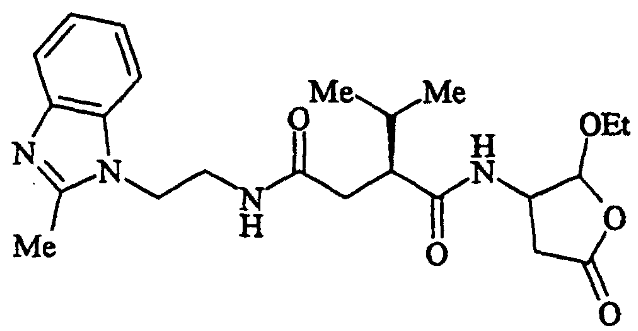

A foamy off-white solid.
MS (APCI) *m/z* 446.2 (M+2, 55.9%), 445.2 (M+1, 51.5%), 300.1 (100%).

| Analysis Calcd for $C_{23}H_{32}N_4O_5 \times 0.50\ H_2O$ (453.543): | | | |
|---|---|---|---|
| | C, 60.91; | H, 7.33; | N, 12.35. |
| Found | C, 60.94; | H, 7.04; | N, 12.05. |

EXAMPLE 47

**N[1]-(2-Ethoxy-5-oxo-tetrahydro-furan-3-yl)-2-isopropyl-N[4]-[3-(3,4,5-trimethoxy-phenyl)-propyl]-succinamide**

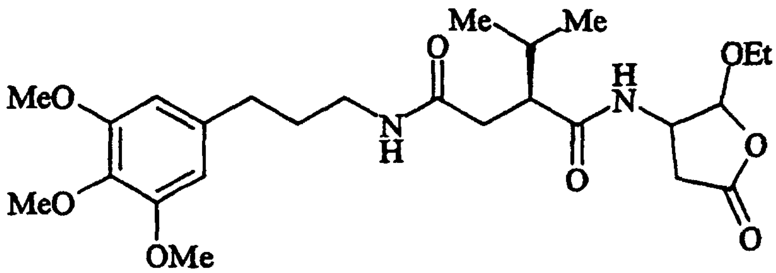

A white solid.
MS (APCI) *m/z* 495.1 (M+1, 100%).

| Analysis Calcd for $C_{25}H_{38}N_2O_8$ (494.590): | | | |
|---|---|---|---|
| | C, 60.71; | H, 7.74; | N, 5.66. |
| Found | C, 60.47; | H, 7.85; | N, 5.77. |

EXAMPLE 48

**N[1]-(2-Ethoxy-5-oxo-tetrahydro-furan-3-yl)-2-isopropyl-N[4]-[2-(phenyl)-ethyl]-succinamide**

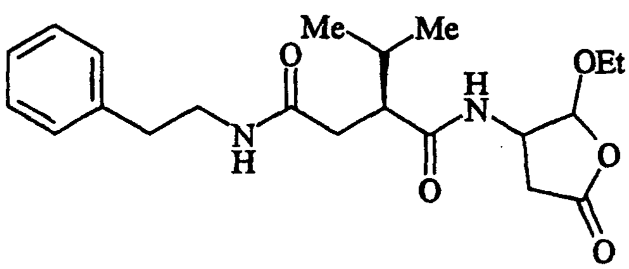

A white solid.
MS (APCI) *m/z* 389.1 (M-1, 100%).

| Analysis Calcd for $C_{21}H_{30}N_2O_5$ (390.484): | | | |
|---|---|---|---|
| | C, 64.60; | H, 7.74; | N, 7.17. |
| Found | C, 64.50; | H, 7.90; | N, 6.83. |

EXAMPLE 49

**N[1]-(2-Ethoxy-5-oxo-tetrahydro-furan-3-yl)-2-isopropyl-N[4]-[4-(phenyl)-butyl]-succinamide**

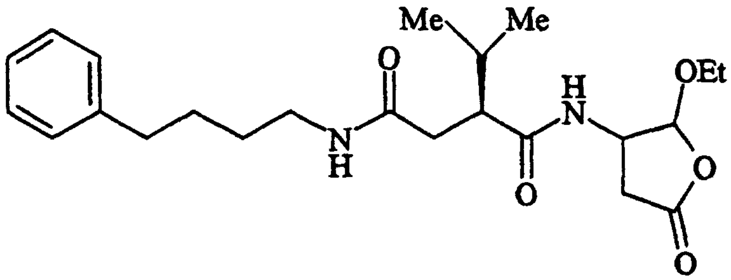

A fluffy white solid.
MS (APCI) *m/z* 419.1 (M+1, 100%).

| Analysis Calcd for $C_{23}H_{34}N_2O_5$ (418.538): | | | |
|---|---|---|---|
| | C, 66.01; | H, 8.19; | N, 6.69. |
| Found | C, 65.91; | H, 8.21; | N, 6.50. |

EXAMPLE 50

**N[1]-(2-Ethoxy-5-oxo-tetrahydro-fu ran-3-yl)-N[4]-(2-indol-1-yl-ethyl)-2-isopropyl-succinamide**

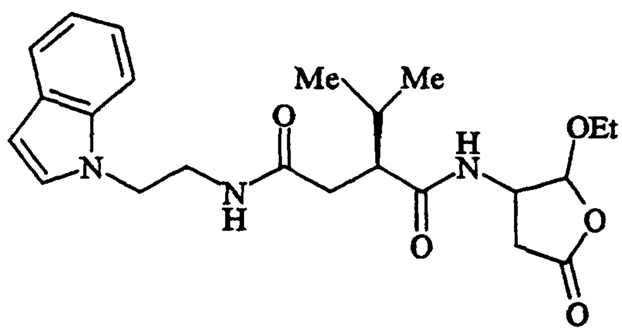

A dark red-brown solid.
MS (APCI) *m/z* 430.1 (M+1, 100%).

| Analysis Calcd for $C_{23}H_{31}N_3O_5$ (429.521): | | | |
|---|---|---|---|
| | C, 64.32; | H, 7.28; | N, 9.78. |
| Found | C, 64.68; | H, 7.02; | N, 10.07. |

EXAMPLE 51

**N1-(2-Ethoxy-5-oxo-tetrahydro-furan-3-yl)-2-isopropyl-N4-[4-(phenyl)-propyl]-succinamide**

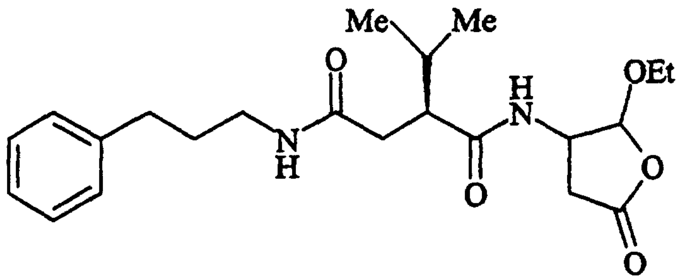

A tan solid.
MS (APCI) *m/z* 405.1 (M+1, 100%).

| Analysis Calcd for $C_{22}H_{32}N_2O_5$ (404.511): | | | |
|---|---|---|---|
| | C, 65.32; | H, 7.97; | N, 6.93. |
| Found | C, 66.27; | H, 8.07; | N, 7.21. |

EXAMPLE 52

**N1-(2-Ethoxy-5-oxo-tetrahydro-furan-3-yl)-N4-[2-(5-fluoro-1H-indol-3-yl)-ethyl]-2-isopropyl-succinamide**

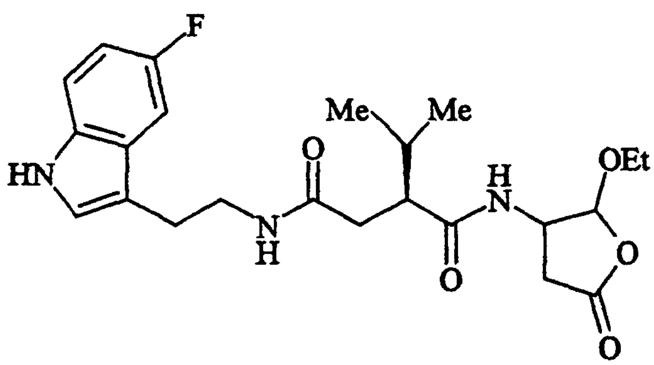

An off-white solid.
MS (APCI) *m/z* 446.1 (M-1, 100%).

| Analysis Calcd for $C_{23}H_{30}FN_3O_5$ (447.511): | | | |
|---|---|---|---|
| | C, 61.73; | H, 6.76; | N, 9.39. |
| Found | C, 61.35; | H, 6.78; | N, 9.36. |

EXAMPLE 53

**3-{2-[(2-Benzoimidazol-1-yl-ethylcarbamoyl)-methyl]-3-methyl-butyrylamino}-4-oxo-butyric acid**

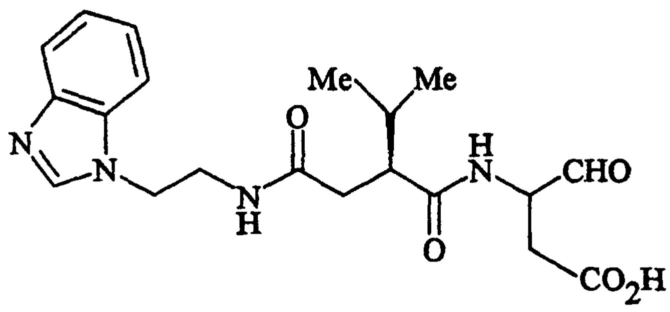

A solution of $N^4$-(2-benzoimidazol-1-yl-ethyl)-$N^1$-(2-ethoxy-5-oxo-tetrahydro-furan-3-yl)-2-isopropyl-succinamide (1.25 g, 2.54 mmol, Example 35, Step D) in 25 mL of acetonitrile was treated with 25 mL of 5% HCl solution. The sample was stirred for 1 hour, then concentrated to an off-white solid. The sample was washed with acetone, filtered, and vacuum dried to give 1.10 g of 3-{2-[(2-benzoimidazol-1-yl-ethylcarbamoyl)-methyl]-3-methyl-butyrylamino}-4-oxo-butyric acid, hydrochloride as a white solid, mp 134-141°C, dec.
MS (APCI) *m/z* 403.3 (M+1, 100%).

| Analysis Calcd for $C_{20}H_{26}N_4O_5 \times HCl \times 1.18\ H_2O$ (460.173): | | | |
|---|---|---|---|
| | C, 52.20; | H, 6.43; | N, 12.18. |
| Found | C, 52.21; | H, 6.70; | N, 11.52. |

In a process analogous to Example 53 using appropriate starting materials, the corresponding compounds (Examples 54 to 62) can be prepared.

EXAMPLE 54

**3-(3-Methyl-2-{[2-(1-methyl-1H-indol-3-yl)-ethylcarbamoyl]-methyl}-butyrylamino)-4-oxo-butyric acid**

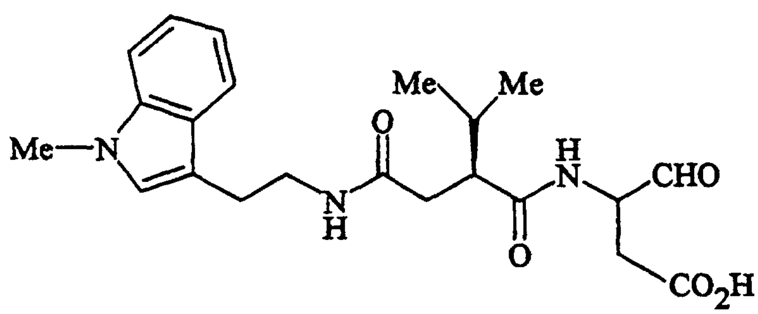

The reaction mixture was basified, extracted, then chromatographed (MPLC, silica gel, 1% $CF_3CO_2H$-20% acetone in $CH_2Cl_2$) to give the title compound as a gray solid.
MS (APCI) *m/z* 416.2 (M+1, 32.5%), 398.1 (M-17, 100%).

| Analysis Calcd for $C_{22}H_{29}N_3O_5 \times 0.05\ CF_3CO_2H$ (421.195): | | | |
|---|---|---|---|
| | C, 63.02; | H, 6.95; | N, 9.98. |

(continued)

| Analysis Calcd for $C_{22}H_{29}N_3O_5 \times 0.05\ CF_3CO_2H$ (421.195): | | | |
|---|---|---|---|
| Found | C, 63.16; | H, 6.84; | N, 9.62. |

EXAMPLE 55

**3-(2-{[2-(5-Fluoro-1-methyl-1H-indol-3-yl)-ethylcarbamoyl]-methyl}-3-methyl-butyrylamino)-4-oxo-butyric acid**

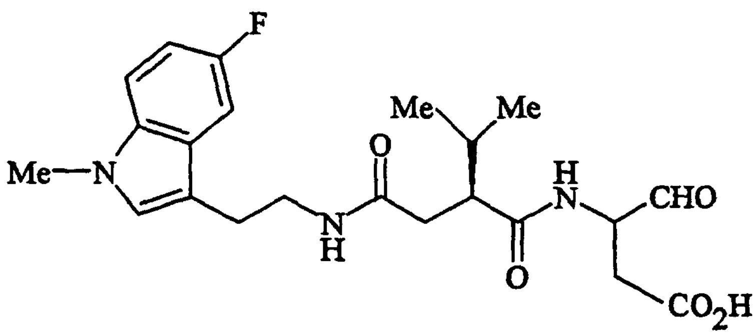

The reaction mixture was basified, extracted, then chromatographed (MPLC, silica gel, 1% $HCO_2H$-20% acetone in $CH_2Cl_2$) to give the title compound as a white solid.
MS (APCI) *m/z* 434.2 (M+1, 100%).

| Analysis Calcd for $C_{22}H_{28}FN_3O_5 \times 0.96\ HCO_2H$ (477.669): | | | |
|---|---|---|---|
| | C, 57.73; | H, 6.31; | N, 8.80. |
| Found | C, 57.73; | H, 6.39; | N, 8.65. |

EXAMPLE 56

**3-(2-{[2-(5,6-Dichloro-benzoimidazol-1-yl)-ethylcarbamoyl]-methyl}-3-methyl-butyrylamino)-4-oxo-butyric acid**

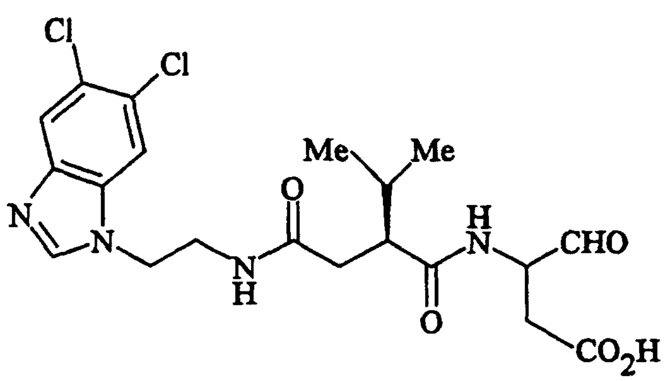

A white solid.
MS (APCI) *m/z* 354.1/356.1/358.1 (M+1, 100/48.6/14.3%).

| Analysis Calcd for $C_{20}H_{24}Cl_2N_4O_5 \times HCl \times 0.50\ H_2O$ (516.813): | | | |
|---|---|---|---|
| | C, 46.48; | H, 5.07; | N, 10.84. |
| Found | C, 46.56; | H, 5.31; | N, 10.17. |

EXAMPLE 57

**3-(2-{[(2-Benzoimidazol-1-yl-ethyl)-methyl-carbamoyl]-methyl}-3-methyl-butyrylamino)-4-oxo-butyric acid**

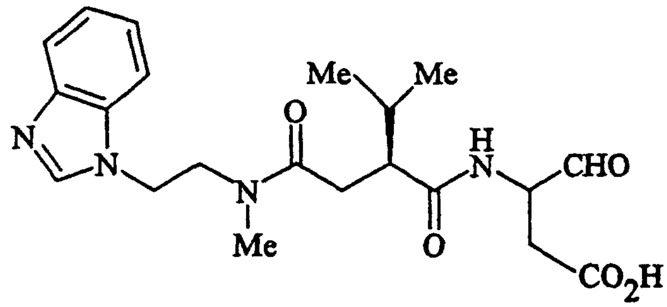

A beige solid.
MS (APCI) *m/z* 417.2 (M+1, 100%).

| Analysis Calcd for $C_{21}H_{28}N_4O_5 \times 2.57\ HCl$ (510.186): | | | |
|---|---|---|---|
| | C, 49.44; | H, 6.04; | N, 10.98. |
| Found | C, 49.46; | H, 6.41; | N, 10.63. |

EXAMPLE 58

**3-{2-[(2-Benzotriazol-1-yl-ethylcarbamoyl)-methyl]-3-methyl-butyrylamino}-4-oxo-butyric acid**

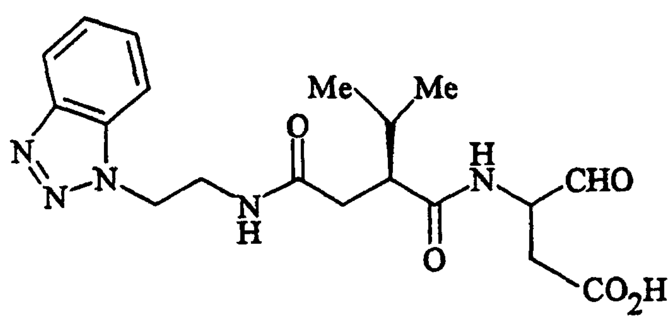

An off-white solid.
MS (APCI) *m/z* 404.0 (M+1, 100%).

| Analysis Calcd for $C_{19}H_{25}N_5O_5 \times 1.54\ HCl \times 0.59\ H_2O$ (470.221): | | | |
|---|---|---|---|
| | C, 48.53; | H, 5.94; | N, 14.89. |
| Found | C, 48.54; | H, 5.94; | N, 14.51. |

EXAMPLE 59

**3-{2-[(2-Indazol-1-yl-ethylcarbamoyl)-methyl]-3-methyl-butyrylamino}-4-oxo-butyric acid**

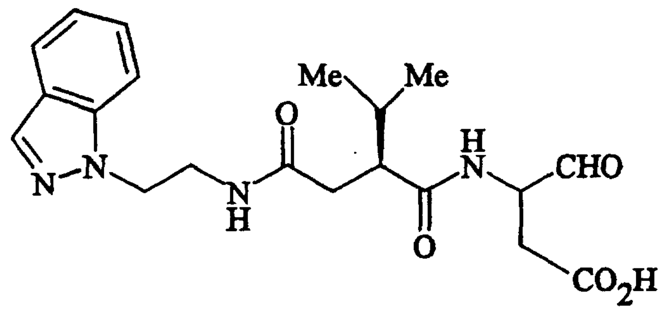

An off-white solid.
MS (APCI) *m/z* 403.1 (M+1, 100%).

| Analysis Calcd for $C_{20}H_{26}N_4O_5 \times 1.34\ HCl \times 1.22\ H_2O$ (473.290): | | | |
|---|---|---|---|
| | C, 50.76; | H, 6.34; | N, 11.84. |
| Found | C, 50.75; | H, 6.34; | N, 11.71. |

EXAMPLE 60

**3-[2-({[2-(5,6-Dimethyl-benzoimidazol-1-yl)-ethyl]-methylcarbamoyl}-methyl)-3-methyl-butyrylamino]4-oxo-butyric acid**

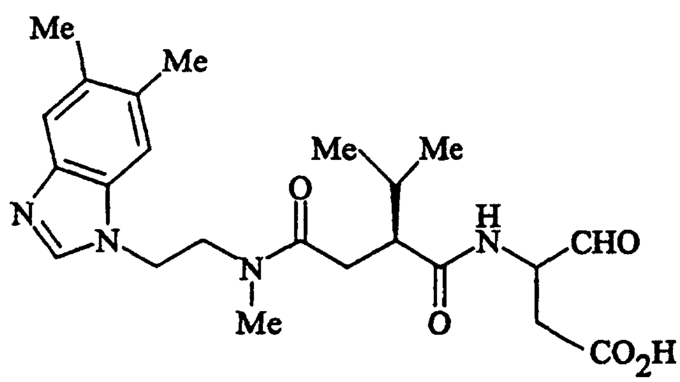

A white solid.
MS (APCI) *m/z* 443.1 (M-1, 100%).

| Analysis Calcd for $C_{23}H_{32}N_4O_5 \times 2.0\ HCl \times 0.35\ H_2O$ (523.763): | | | |
|---|---|---|---|
| | C, 52.74; | H, 6.68; | N, 10.70. |
| Found | C, 52.75; | H, 6.88; | N, 10.39. |

EXAMPLE 61

**3-[2-({[2-(2-Methyl-benzoimidazol-1-yl)-ethyl]-methylcarbamoyl}-methyl)-3-methyl-butyrylamino]4-oxo-butyric acid**

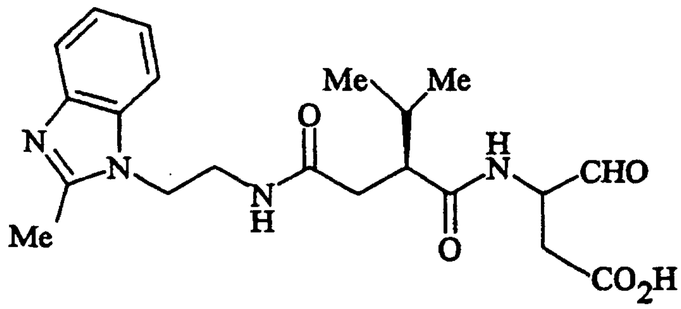

An off-white solid.
MS (APCI) *m/z* 415.1 (M-1, 100%).

| Analysis Calcd for $C_{21}H_{28}N_4O_5 \times 1.1$ HCl (456.588): | | | |
|---|---|---|---|
| | C, 55.24; | H, 6.42; | N, 12.27. |
| Found | C, 55.14; | H, 6.64; | N, 11.01. |

EXAMPLE 62

**3-(3-methyl-2-{[3-(3,4,5-trimethoxy-pheny)-propylcarbamoyl]-methyl}-butyrylamino)-4-oxo-butyric acid**

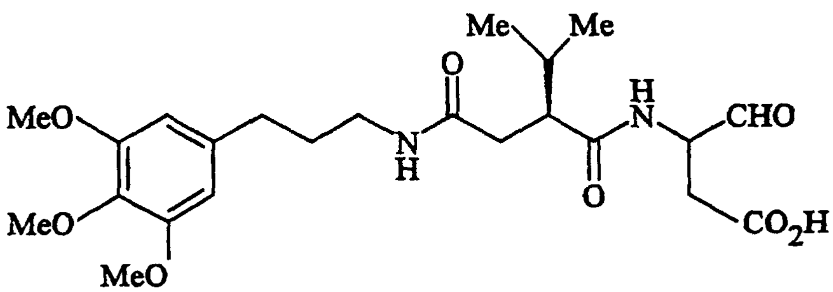

A white solid.
MS (APCI) *m/z* 467.1 (M+1, 100%).

| Analysis Calcd for $C_{23}H_{34}N_2O_8 \times 0.70$ $H_2O$ (479.147): | | | |
|---|---|---|---|
| | C, 57.66; | H, 7.45; | N, 5.85. |
| Found | C, 57.44; | H, 7.13; | N, 5.72. |

EXAMPLE 63

**3-{3-Methyl-2-[(2-phenoxy-ethylcarbamoyl)-methyl]-butyrylamino}-4-oxo-butyric acid ethyl ester**

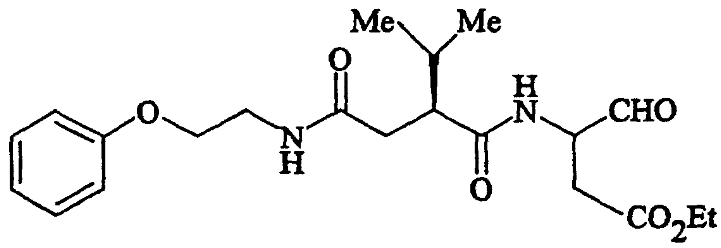

**Step A**

A mixture of (S)-2-isopropyl-succinic acid 1-benzyl ester (10.1 g, 40.2 mmol, Example 3, Step A), 2-phenoxy-l-ethylamine (6.1 g, 44.2 mmol), HOBT × $H_2O$ (7.7 g, 50.2 mmol), EDCI × HCl (9.6 g, 50.2 mmol) and N-methylmorpholine (6.6 mL, 60.0 mmol) in 100 mL of $CH_2Cl_2$ was stirred at room temperature for 12 hours. The sample was concentrated, then partitioned between EtOAc and saturated $NaHCO_3$ solution. The organic extract was washed with saturated $KH_2PO_4$ and brine solutions, dried ($MgSO_4$), filtered, and concentrated. The resultant brown liquid was chromatographed (MPLC, silica gel, 80% hexanes-20% EtOAc to 50% hexanes-50% EtOAc) to give 12.3 g (83%) of (S)-2-[(2-phenoxy-ethylcarbamoyl)-methyl]-3-methyl-butyric acid benzyl ester as a light yellow liquid. MS (APCI) *m/z* 370.0 (M+1, 100%).

Hydrogenation of (S)-2-[(2-phenoxy-ethylcarbamoyl)-methyl]-3-methyl-butyric acid benzyl ester (12.3 g, 33.2 mmol) with 20% Pd/C in 100 mL of ethanol at balloon pressure gave 9.6 g (~100%) of (S)-2-[(2-phenoxy-ethylcaxbamoyl)-methyl]-3-methyl-butyric acid as a light yellow oil. MS (APCI) *m/z* 280.0 (M+1, 100%).

**Step B**

A mixture of (S)-2-[(2-phenoxy-ethylcarbamoyl)-methyl]-3-methyl-butyric acid (4.8 g, 17.2 mmol, Example 63, Step A), 3-amino-4-pentenoic acid ethyl ester hydrochloride (3.8 g, 21.2 mmol, prepared using the procedure of Hauser F.M. et al., *J. Org. Chem*. 1987;52:5041-5044), HOBT × $H_2O$ (3.3 g, 21.5 mmol), EDCI × HCl (4.1 g, 21.5 mmol) and N-methylmorpholine (4.7 mL, 42.7 mmol) in 150 mL of $CH_2Cl_2$ was stirred at room temperature for 12 hours. The sample was concentrated, then partitioned between EtOAc and saturated $NaHCO_3$ solution. The organic extract was washed with sat. $KH_2PO_4$ and brine solutions, dried ($MgSO_4$), filtered, and concentrated. The resultant yellow oily-solid was chromatographed (MPLC, silica gel, 25% hexanes-75% EtOAc) to give 4.2 g (60%) of 3-{3-methyl-2-[(2-phenoxy-ethylcarbamoyl)-methyl]-butyrylamino}-4-pentenoic acid ethyl ester as a waxy light yellow solid.
MS (APCI) *m/z* 405.1 (M+1, 100%).

**Step C**

Ozone gas was bubbled through a solution at -78°C of 3-{3-methyl-2-[(2-phenoxy-ethylcarbamoyl)-methyl]-butyrylamino}-4-pentenoic acid ethyl ester (4.2 g, 10.3 mmol, Example 63, Step B) in 100 mL of $CH_2Cl_2$ until a blue color formed. The reaction was quenched at -78°C by dropwise addition of dimethyl sulfide (2.3 mL, 31.3 mmol). The sample was concentrated, then partitioned between EtOAc and saturated $NaHCO_3$ solution. The organic extract was washed with saturated $KH_2PO_4$ and brine solutions, dried ($MgSO_4$), filtered, concentrated and chromatographed (MPLC, silica gel, 25% hexanes-75% EtOAc) to give 3-{3-methyl-2-[(2-phenoxy-ethylcarbamoyl)-methyl]-butyrylamino}-4-oxo-butyric acid ethyl ester as a white solid.
MS (APCI) *m/z* 407.1 (M+1, 100%).

| Analysis Calcd for $C_{21}H_{30}N_2O_6$ × 0.17 $H_2O$ (409.546): | | | |
|---|---|---|---|
| | C, 61.59; | H, 7.47; | N, 6.84. |
| Found | C, 61.57; | H, 7.46; | N, 6.69 |

EXAMPLE 64

**3-Cyano-3-{3-methyl-2-[(2-phenoxy-ethylcarbamoyl)-methyl]-butyrylamino}-propionic acid ethyl ester**

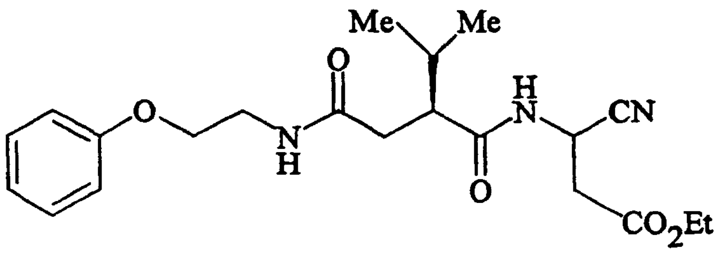

**Step A**

A solution of 4-amino-3-[(benzyloxycarbonyl)-amino]-4-oxo-butanoic acid (8.9 g, 33.6 mmol) and 10 drops of concentrated sulfuric acid in 250 mL of ethanol was refluxed for 12 hours. The solution was cooled, concentrated, then partitioned between EtOAc and saturated $NaHCO_3$ solution. The organic extract was washed with saturated $KH_2PO_4$ and brine solutions, dried ($MgSO_4$), filtered, and concentrated to give 4.7 g (48%) of 4-amino -3-[(benzyloxycarbonyl)-amino]-4-oxo-butanoic acid ethyl ester as a white solid.
MS (APCI) *m/z* 295.0 (M+1, 100%).

| Analysis Calcd for $C_{14}H_{18}N_2O_5$ (294.310): | | | |
|---|---|---|---|
| | C, 57.14; | H, 6.16; | N, 9.52. |
| Found | C, 57.28; | H, 6.11; | N, 9.45. |

Hydrogenation of 4-amino-3-[(benryloxycarbonyl)-amino]-4-oxo-butanoic acid ethyl ester (2.1 g, 7.4 mmol) with 20% Pd/C in 100 mL of ethanol at balloon pressure gave 1.2 g (~100%) of 3-4-diamino-4-oxo-butanoic acid ethyl ester as a colorless oily solid.
MS (APCI) *m/z* 161.1 (M+1, 100%).

**Step B**

A mixture of (S)-2-[(2-phenoxy-ethylcarbamoyl)-methyl]-3-methyl-butyric acid (2.5 g, 9.0 mmol, Example 63, Step A), 3,4-diamino-4-oxo-butanoic acid ethyl ester (1.2 g, 7.4 mmol, Example 64, Step A), HOBT × $H_2O$ (1.4 g, 9.3 mmol), EDCI × HCl (3.6 g, 18.6 mmol) and N-methylmorpholine (2.1 mL, 19.1 mmol) in 100 mL of $CH_2Cl_2$ was stirred at room temperature for 12 hours. The sample was concentrated, then partitioned between EtOAc and saturated $NaHCO_3$ solution. The organic extract was washed with saturated $KH_2PO_4$ and brine solutions, dried ($MgSO_4$), filtered, and concentrated. The resultant off-white oily-solid was chromatographed (MPLC, silica gel, 5% methanol in chloroform) to give 0.35 g (11%) of 4-amino-3-{3-methyl-2-[(2-phenoxy-ethylcarbamoyl)-methyl]-butyrylamino}-4-oxo-butyric acid ethyl ester as a white solid.
MS (APCI) *m/z* 420.1 (M-1, 30.9%), 374.1 (100%).

| Analysis Calcd for $C_{21}H_{31}N_3O_6$ (421.498): | | | |
|---|---|---|---|
| | C, 59.84; | H, 7.41; | N, 9.97. |
| Found | C, 60.23; | H, 7.38; | N, 9.90 |

**Step C**

To a stirring solution at 0°C under $N_2$ of 4-amino-3-{3-methyl-2-[(2-phenoxy-ethylcarbamoyl)-methyl]-butyrylamino}-4-oxo-butyric acid ethyl ester (0.35 g, 0.83 mmol, Example 64, Step B) and triethylamine (0.29 mL, 2.08 mmol) in 50 mL of THF was added dropwise trifluoroacetic anhydride (0.14 mL, 0.99 mmol). The sample was stirred at 0°C for 1 hour, then quenched at 0°C by dropwise addition of saturated $NaHCO_3$ solution (~10 mL). The sample was

concentrated, then extracted with EtOAc. The organic extract was washed with saturated $KH_2PO_4$ and brine solutions, dried ($MgSO_4$), filtered, and concentrated. Chromatography (MPLC, silica gel, 25% hexanes-75% EtOAc) afforded 0.21 g (63%) of 3-cyano-3-{3-methyl-2-[(2-phenoxy-ethylcarbamoyl)-methyl]-butyrylamino}-propionic acid ethyl ester as a white solid.
MS (APCI) *m/z* 404.1 (M+1, 100%).

| Analysis Calcd for $C_{21}H_{29}N_3O_5 \times 0.35\ H_2O$ (409.788): | | | |
|---|---|---|---|
| | C, 61.55; | H, 7.30; | N, 10.25. |
| Found | C, 61.73; | H, 7.23; | N, 9.87. |

EXAMPLE 65

**3-Cyano-3-{3-methyl-2-[(2-phenoxy-ethylcarbamoyl)-methyl]-butyrylamino}-propionic acid**

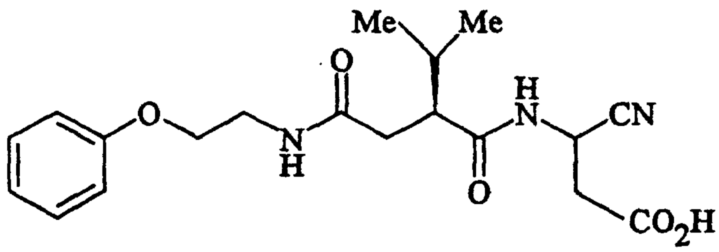

**Step A**

A mixture of (S)-2-[(2-phenoxy-ethylcarbamoyl)-methyl]-3-methyl-butyric acid (1.67 g, 5.99 mmol, Example 63, Step A), H-Asp(OtBu)-Ome-HCl (1.58 g, 6.59 mmol), HOBT·$H_2O$ (1.00 g, 6.53 mmol), EDCI·HCl (1.26 g, 6.57 mmol) and N-methylmorpholine (1.50 mL, 13.64 mmol) in 50 mL of $CH_2Cl_2$ was stirred at room temperature for 12 hours. The sample was concentrated, then partitioned between EtOAc and sat. $NaHCO_3$ solution. The organic extract was washed with saturated $KH_2PO_4$ and brine solutions, dried ($MgSO_4$), filtered, and concentrated. The resultant yellow oil was chromatographed (MPLC, silica gel, 25% hexanes/75% EtOAc) to give 1.69 g (61%) of 2-{3-methyl-2-[(2-phenoxy-ethylcarbamoyl)-methyl]-butyrylamino}-succinic acid 4-tert-butyl ester 1-methyl ester as a waxy white solid.
MS (APCI) *m/z* 465.2 (M+1, 100%).

**Step B**

A solution of 2-{3-methyl-2-[(2-phenoxy-ethylcarbamoyl)-methyl]-butyrylamino}-succinic acid 4-tert-butyl ester 1-methyl ester (1.69 g, 3.65 mmol, Example 65, Step A) in 20 mL of THF at room temperature was treated with 1.0 M lithium hydroxide solution (4.4 mL, 4.40 mmol). The cloudy sample was stirred at room temperature for 1 hour, acidified with saturated $KH_2PO_4$ solution (~50 mL), concentrated (to remove most of the THF), then extracted with EtOAc. The organic extract was washed with brine solution, dried ($MgSO_4$), and filtered to give 1.38 g (84%) of 2-{3-methyl-2-[(2-phenoxy-ethylcarbamoyl)-methyl]-butyrylamino}-succinic acid 4-tert-butyl ester as a white solid.
MS (APCI) *m/z* 451.2 (M+1, 76.5%), 179.1 (100%).

| Analysis Calcd for $C_{23}H_{34}N_2O_7 \times 0.20\ H_2O$ (454.140): | | | |
|---|---|---|---|
| | C, 60.83; | H, 7.64; | N, 6.17. |
| Found | C, 60.84; | H, 7.63; | N, 5.98. |

**Step C**

A stirring solution of 2-{3-methyl-2-[(2-phenoxy-ethylcarbamoyl)-methyl]-butyrylamino}-succinic acid 4-tert-butyl ester (1.00 g, 2.23 mmol, Example 65, Step B) and N-methylmorpholine (0.31 mL, 2.82 mmol) in 25 mL of $CH_2Cl_2$ at ca. -45°C (Dry Ice-$CH_3CN$ slurry) was treated dropwise with iso-butyl chloroformate (0.32 mL, 2.47 mmol). The

sample was stirred for 15 minutes, then quenched by dropwise addition of concentrated ammonium hydroxide solution (5 mL). The sample was allowed to warm to room temperature, concentrated, then extracted with EtOAc. The organic extract was washed with brine solution, dried ($MgSO_4$), and filtered to give 1.12 g (>100%) of 4-amino-3-{3-methyl-2-[(2-phenoxy-ethylcarbamoyl)-methyl]-butyrylamino}-4-oxo-butanoic acid tert-butyl ester as a white solid: MS (APCI) *m/z* 448.1 (M-1, 92.6%), 374.0 (100%).

The above primary amide was dehydrated using the same conditions as described in Example 64, Step C, then chromatographed (MPLC, silica gel, 25% hexanes/75% EtOAc) to give 0.64 g (62%) of 3-cyano-3-{3-methyl-2-[(2-phenoxy-ethylcarbamoyl)-methyl]-butyrylamino}-propionic acid tert-butyl ester as a white solid.
MS (APCI) *m/z* 305.1 (100%).

**Step D**

A solution of 3-cyano-3-{3-methyl-2-[(2-phenoxy-ethylcarbamoyl)-methyl]-butyrylamino}-propionic acid tert-butyl ester (0.64 g, 1.48 mmol, Example 65, Step C) and 5.0 mL of trifluoroacetic acid in 25 mL of $CH_2Cl_2$ was stirred at room temperature for 1 hour. The solution was concentrated, then partitioned between EtOAc and saturated $KH_2PO_4$ solution. The organic extract was washed with brine solution, dried ($MgSO_4$), filtered, and concentrated to a light yellow oil. Diethyl ether (~50 mL) was added and the oil slowly solidified. The sample was filtered and vacuum dried to give 0.28 g (49%) of 3-cyano-3-{3-methyl-2-[(2-phenoxy-ethylcarbamoyl)-methyl]-butyrylamino}-propionic acid as a white solid.
MS (APCI) *m/z* 377.0 (M+2, 100%).

| Analysis Calcd for $C_{19}H_{25}N_3O_5 \times 1.20\ H_2O$ (397.047): | | | |
|---|---|---|---|
| | C, 57.48; | H, 6.96; | N, 10.58. |
| Found | C, 57.53; | H, 6.68; | N, 10.28. |

BIOLOGICAL ASSAYS

INHIBITION STUDIES

Compounds of Formula I are inhibitors of ICE as demonstrated by measurement of $K_i$ (μM) and $IC_{50}$ (μM) using the protocol described herein. Serial dilutions of each compound of the invention are prepared using an initial 8-fold dilution of a DMSO stock into HGE (100 mM HEPES, 20% glycerol v/v, 0.5 mM EDTA), followed by seven serial 2-fold dilutions into HGE + 12.5% DMSO. Ten microliters of diluted stocks or of vehicle (HGE + 12.5% DMSO) are placed in triplicate onto a 96-well microtiter plate. Enzyme is diluted into assay buffer (HGE, 5 mM DTT, 15 μM Ac-Tyr-Val-Ala-Asp-AMC; 0.5 nM final enzyme concentration, pre-warmed to 30°C), and this reaction mixture is added to the plate at 90 μL/well. Substrate hydrolysis is monitored for 300 seconds at 30°C using 385 and 460 nm excitation and emission filters, respectively. Triplicate curves are averaged, and slopes are evaluated by linear regression. To evaluate Ki, plots of percent inhibition vs inhibitor concentration are fit by non-linear regression to a reversible, competitive model:

$$\%\text{Inhibition} = \frac{100 \bullet [I]}{[I] + K_i \bullet \left(1 + \frac{[S]}{K_M}\right)}$$

where the competition factor is (1 + $[S]/K_M$) = 2.

**ICE Colorimetric Dose-Response ($IC_{50}$) Assay**

Diluted inhibitor stocks are prepared by two-fold serial dilution from a primary stock whose concentration is selected (based on screening results or on prior attempts at $IC_{50}$ evaluation) to achieve approximately 95% inhibition in the most concentrated well. Aliquots of each dilution are transferred to a microtitre plate in triplicate.

ICE enzyme is diluted to approximately 24 nM in HGE buffer (100 mM Hepes pH 7.5, 0.5 mM EDTA, 20% glycerol, 0.1% Bovine Serum Albumin (BSA), and activated by adding dithiothreitol (DTT) to a final concentration of 5 mM. The activated enzyme is then aliquoted into wells containing inhibitor or vehicle, and the plate is preincubated for 60 minutes at ambient temperature. Substrate (Ac-Tyr-Val-Ala-Asp-pNA) is added to each well to a final concentration

of 50 μM, and plates are placed in the microtitre plate-reader thermostated to 25°C. Beginning 5 minutes after addition of substrate, absorbance (405 nm) of wells is monitored for 1 hour, and activity is calculated as the mean rate of change in absorbance during this interval.

### PBMC Cellular Assay - $IC_{50}$ Determinations

Further evidence that compounds of Formula I are inhibitors of ICE is provided by their ability to inhibit IL-1β production in human peripheral blood mononuclear cells (PBMCs) as described herein. PBMCs are isolated from heparinized blood by centrifugation over a ficoll cushion, then washed three times with phosphate-buffered saline. PBMCs are suspended in a medium containing RPMI 1640 with glutamine, penicillin, streptomycin, and 2% human AB serum, then plated at $10^6$ cells per well in 96-well flat bottom plates. PBMCs are stimulated overnight with 10 ng/mL of lipopolysaccharide (LPS, *E. coli* strain 0111:B4; Calbiochem) in the presence or absence of a compound of Formula I. Medium is harvested, and the level of mature IL-1β was determined using an ELISA kit from R&D Systems. Cells were cultured for an additional 4 hours in the presence of 3-(4,5-dimethylthiazol-2-yl)-2,5-diphenyltetrazolium bromide (MTT) to determine viability.

### Ich-2 Colorimetric Dose-Response ($IC_{50}$) Assay

Inhibition of Ich-2 enzyme is assayed as described above for ICE, except that enzyme is used at 64 nM, and 60 μM of the Ich-2-specific substrate Ac-Leu-Glu-Val-Asp-pNA is used instead of the ICE substrate Ac-Tyr-Val-Ala-Asp-pNA.

The results of these tests are shown below in Table 2.

TABLE 2

| Example No. | ICE $K_i$ (μM) | ICE $IC_{50}$ (μM) | PBMC $IC_{50}$ (μM) | ICH2 $IC_{50}$ (Caspase 4) (μM) |
|---|---|---|---|---|
| 1 | 1.60 | 14.50 | 82.50 | 88.96 |
| 2 | 0.106 | 0.790 | 6.25 | 3.31 |
| 3 | 0.045 | 0.40 | 3.00 | 4.65 |
| 4 | 2.18 | 16.80 | 65.00 | 9.72 |
| 5 | 0.336 | 3.55 | 35.00 | 9.02 |
| 6 | 0.142 | 1.060 | 3.25 | 9.59 |
| 7 | 0.115 | 0.444 | 6.00 | 20.72 |
| 8 | 0.0150 | 0.54 | 3.75 | -- |
| 9 | 0.0234 | 0.053 | 2.10 | 4.22 |
| 10 | 0.539 | 3.270 | 21.50 | 25.74 |
| 11 | 0.058 | 0.440 | 5.50 | 15.69 |
| 12 | 0.0128 | 0.061 | 1.20 | 1.71 |
| 13 | 0.0067 | 0.052 | 2.35 | 0.25 |
| 14 | 0.1066 | 0.0087 | 19.00 | -- |
| 15 | 0.441 | 2.050 | 17.50 | 13.08 |
| 16 | 0.0025 | 0.0051 | 0.35 | 1.47 |
| 17 | 0.0329 | 0.0790 | 2.25 | 4.68 |
| 18 | 0.0236 | 0.0894 | 2.05 | 3.01 |
| 19 | 0.0009 | 0.0085 | 0.40 | 2.38 |
| 20 | 0.0015 | 0.0110 | 2.45 | 61.97 |
| 21 | 0.0280 | 0.130 | 7.50 | 209.07 |
| 22 | 0.1330 | 1.6700 | 19.00 | -- |
| 23 | 0.0029 | 0.0145 | 3.60 | 4.43 |
| 24 | 0.0207 | 0.3600 | 1.60 | 8.66 |
| 25 | 0.0058 | 0.0483 | 1.10 | 3.54 |
| 26 | 0.0266 | 0.1360 | 2.00 | 9.91 |
| 27 | 0.0175 | 0.1470 | 1.00 | 9.93 |
| 28 | 0.0061 | 0.2540 | 0.55 | 5.58 |
| 29 | 0.0245 | 0.1130 | 1.90 | 4.79 |

TABLE 2 (continued)

| Example No. | ICE $K_i$ (μM) | ICE $IC_{50}$ (μM) | PBMC $IC_{50}$ (μM) | ICH2 $IC_{50}$ (Caspase 4) (μM) |
|---|---|---|---|---|
| 30 | 0.0011 | 0.0052 | 0.41 | 3.14 |
| 31 | 0.0004 | 0.0059 | 0.24 | 1.24 |
| 32 | 0.0260 | 0.4580 | 3.30 | 14.12 |
| 33 | 0.0065 | 0.0230 | 1.60 | 3.09 |
| 34 | 0.0166 | 0.1150 | 2.60 | 1.10 |
| 35 | 0.0511 | 1.0600 | 5.50 | -- |
| 36 | 0.6300 | 0.5990 | 1.30 | 101.17 |
| 37 | 0.0297 | 0.5810 | 2.00 | 127.60 |
| 38 | 0.0164 | 0.3770 | 3.40 | 18.73 |
| 39 | 0.4465 | 13.2000 | 5.00 | -- |
| 40 | 0.0354 | 1.1500 | 2.90 | -- |
| 41 | 0.4090 | 13.0000 | 17.50 | -- |
| 42 | 0.1910 | 1.7300 | 7.50 | -- |
| 43 | 0.0981 | 2.2800 | 7.50 | -- |
| 44 | 0.0475 | 1.2200 | 2.50 | -- |
| 45 | 0.0370 | 0.7500 | 2.60 | 173.83 |
| 46 | 0.0284 | 0.4380 | 6.50 | 366.57 |
| 47 | 1.9300 | 112.0000 | 11.00 | -- |
| 48 | 1.0060 | 59.9000 | 13.50 | -- |
| 49 | 2.1500 | 123.0000 | -- | -- |
| 50 | 0.0538 | 0.9240 | 2.00 | -- |
| 51 | 0.259 | 15.00 | 16.25 | -- |
| 52 | 0.0185 | 0.4855 | 0.75 | -- |
| 53 | 0.0200 | 0.0737 | 8.30 | 32.51 |
| 54 | 0.0040 | 0.0370 | 2.85 | 11.13 |
| 55 | 0.0079 | 0.0370 | 5.30 | 11.48 |
| 56 | 0.0044 | 0.0286 | 3.25 | 4.02 |
| 57 | 0.0229 | 0.2270 | 3.75 | 38.03 |
| 58 | 0.0318 | 0.3360 | 7.75 | 10.63 |
| 59 | 0.0332 | 0.0566 | 3.75 | 10.40 |
| 60 | 0.0092 | 0.4680 | 5.00 | 12.71 |
| 61 | 0.0047 | 0.0233 | 2.90 | -- |
| 62 | 0.274 | 2.560 | 23.00 | |
| 63 | 6.92 | 197.00 | 14.50 | |
| 64 | >100 | -- | >100 | -- |
| 65 | 107.00 | -- | >100 | -- |

The following abbreviations are used throughout this patent application:

| | |
|---|---|
| HEPES | 4-(2-hydroxymethyl)-1-piperazine ethane sulfonic acid |
| DTT | Dithiothreitol |
| EDTA | Ethylene diamine tetra acetic acid |
| Ac | Acetyl |
| Glu | Glutamic acid |
| LEU | Leucine |
| Tyr | Tyrosine |
| Val | Valine |
| Ala | Alanine |
| Asp | Aspartic Acid |
| AMC | 7-amino-4-methyl coumarin |
| pNA | Para nitroaniline |

| | |
|---|---|
| mp | Melting point |
| EtOAc | Ethyl acetate |
| MS | Mass Spectrum |
| THF | Tetrahydrofuran |
| t-Bu | tert-Butyl |
| Me | Methyl |
| DMF | Dimethylformamide |
| MPLC | Medium pressure liquid chromatography |
| psig | Pounds per square inch (gauge) |
| HOBt | 1-Hydroxybenzotriazole |
| EDCI | N-ethyl-N'-dimethylaminopropylcarbodiimide |

## Claims

**1.** A compound of the Formula I

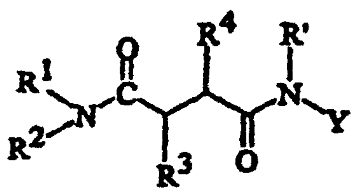

I

wherein
Y is

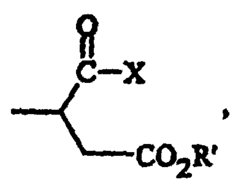

,

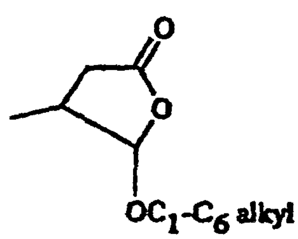

,

or

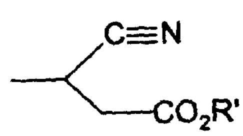

;

each R' is independently hydrogen or $C_1$-$C_6$ alkyl;
$R^1$ and $R^2$ are independently hydrogen, $C_1$-$C_6$ alkyl,

-OH, $-(CH_2)_n$ aryl,
$-(CH_2)_n$-substituted aryl,

$-(CH_2)_n$-O-aryl,
$-(CH_2)_n$-O-substituted aryl,
$-(CH_2)_n$-S-aryl,
$-(CH_2)_n$-S-substituted aryl,
$-(CH_2)_n$-S-heteroaryl,
$-(CH_2)_n$-S-substituted heteroaryl,
$-(CH_2)_n$-NR'-aryl,
$-(CH_2)_n$-NR'-substituted aryl,
$-(CH_2)_n$-NR'-heteroaryl,
$-(CH_2)_n$-NR'-substituted heteroaryl,
$-(CH_2)_n$-heteoaryl, or
$-(CH_2)_n$-substituted heteroaryl;

each n is independently 0 to 6;
$R^3$ is hydrogen or $C_1$-$C_6$ alkyl;
$R^4$ is $C_1$-$C_6$ alkyl or hydrogen; and
X is hydrogen,

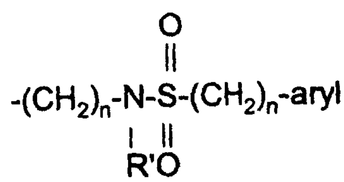

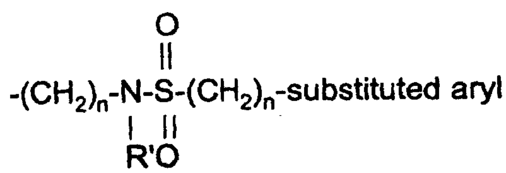

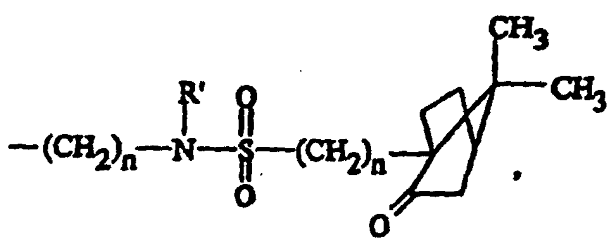

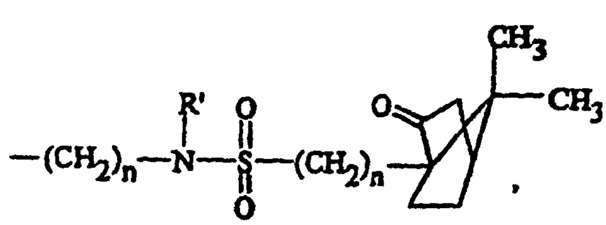

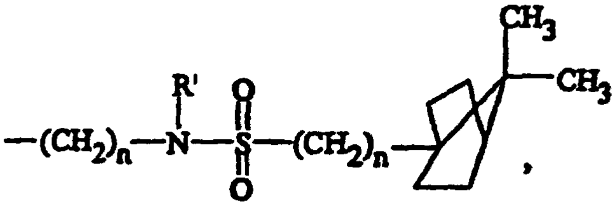,

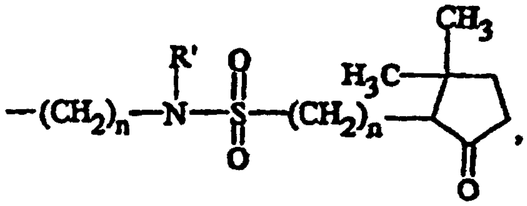,

$-(CH_2)_n-S-(CH_2)_n-$aryl,

$-(CH_2)_n-S-(CH_2)_n-$substituted aryl,

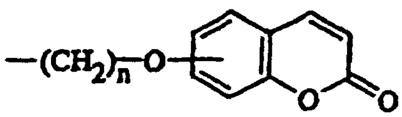,

or

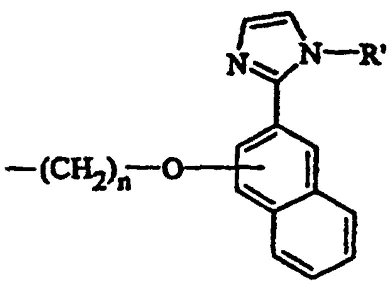;

and the pharmaceutically acceptable salts thereof.

**2.** A compound in accordance with Claim 1 wherein R' is hydrogen or methyl.

**3.** A compound in accordance with Claim 1 wherein each R' is hydrogen.

**4.** A compound in accordance with Claim 1 wherein
$R^3$ is hydrogen, and
$R^4$ is methyl, ethyl, or isopropyl.

**5.** A compound in accordance with Claim 1 wherein
$R^1$ is hydrogen or methyl; and
$R^2$ is

-$(CH_2)_n$-phenyl,
hydrogen,
-$(CH_2)_n$-O-phenyl,
-OH,
-$(CH_2)_n$-benzimidazoyl,
-$(CH_2)_n$-indolyl, or
-$(CH_2)_n$-phenol.

**6.** A compound in accordance with Claim 1 wherein

Y is 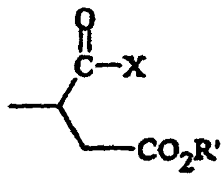 .

**7.** A compound in accordance with Claim 1 wherein
Y is

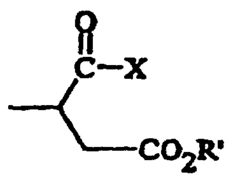 ,

X is hydrogen,

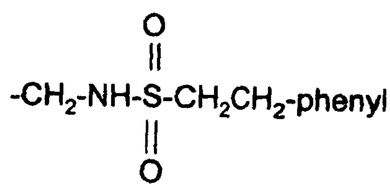

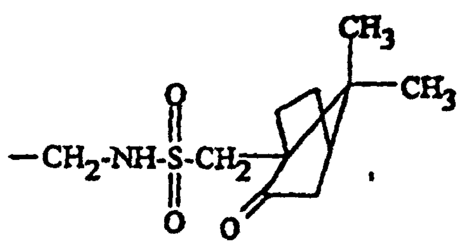 ,

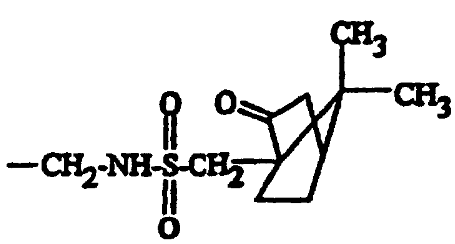 ,

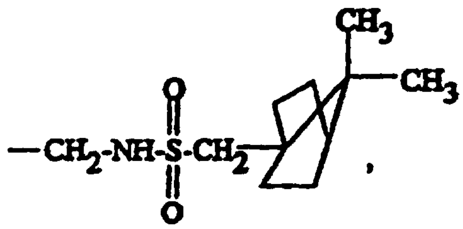

$-CH_2-S-CH_2CH_2CH_2$-phenyl,

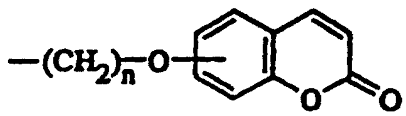

or

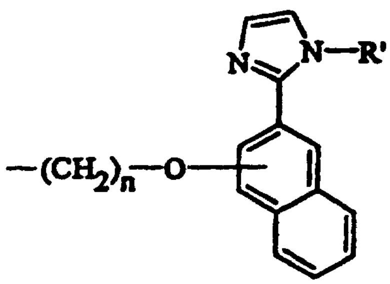

**8.** A compound of the Formula I:

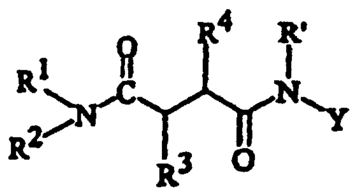

I

wherein
Y is

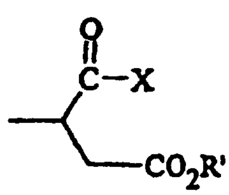

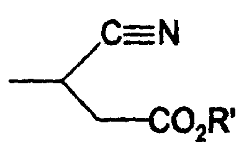

or

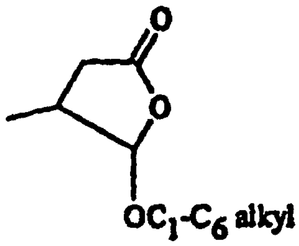

;

each R' is independently hydrogen or methyl;
each n is independently 1, 2 or 3;
$R^1$ and $R^2$ are independently hydrogen,

$-(CH_2)_n$-phenyl,

$-(CH_2)_n$-O-phenyl,

-OH,

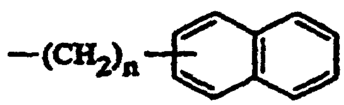

,

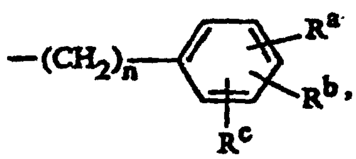

,

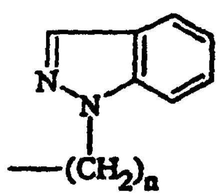

,

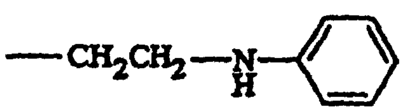

,

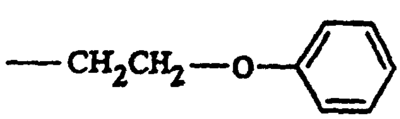

,

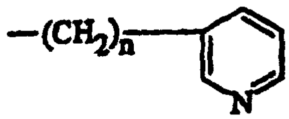,

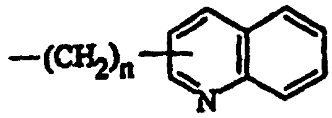,

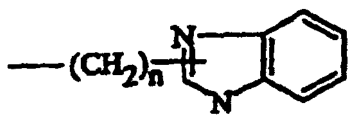,

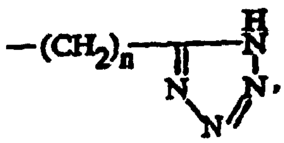,

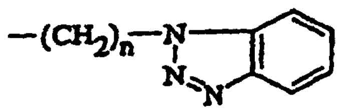,

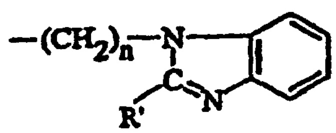,

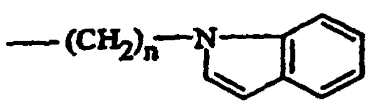,

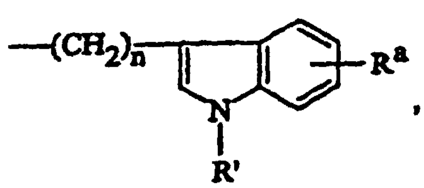,

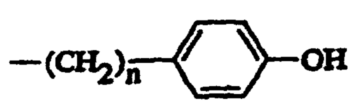;

$R^a$, $R^b$, and $R^c$ are independently halogen, $-OC_1-C_6$ alkyl or hydrogen;
$R^3$ is hydrogen;
$R^4$ is methyl, ethyl, or isopropyl; and

X is hydrogen,

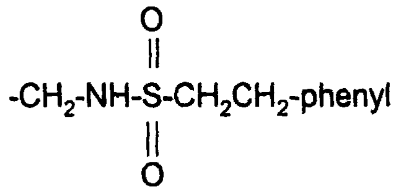

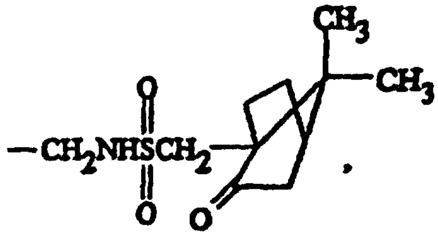
,

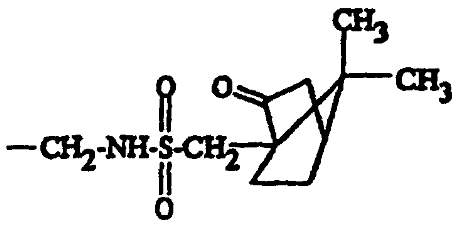
,

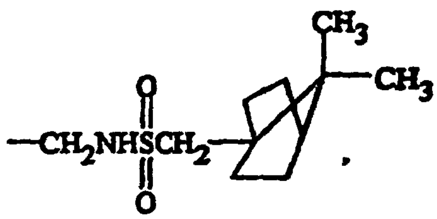
,

$-CH_2-S-CH_2CH_2CH_2$-phenyl,

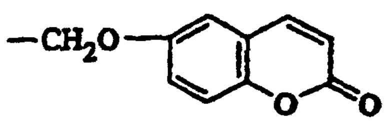
,

**or**

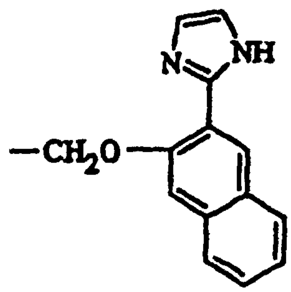

;

and the pharmaceutically acceptable salts thereof.

**9.** A compound in accordance with Claim 1 wherein $R^1$ is hydrogen and $R^2$ is

$-(CH_2)_n$-indolyl,
$-(CH_2)_n$-NH-phenyl,
$-(CH_2)_n$-O-phenyl,
$-(CH_2)_n$-substituted indolyl,
$-(CH_2)_n$-tetrazolyl,
$-(CH_2)_n$-phenyl,
$-(CH_2)_n$-substituted phenyl,
$-(CH_2)_n$-substituted benzimidazolyl,
$-(CH_2)_n$-benztriazolyl,
$-(CH_2)_n$-indazolyl,
$-(CH_2)_n$-benzimidazolyl,
$-(CH_2)_n$-pyridyl,
$-(CH_2)_n$-naphthyl, or
$-(CH_2)_n$-quinolinyl.

**10.** The compound:

3-(2-Methyl-3-phenethylcarbamoyl-propionylamino)-4-oxo-5-(2-phenyl-ethanesulfonylamino)-pentanoic acid;
3-(2-Carbamoylmethyl-3-methyl-butyrylamino)-5-(7,7-dimethyl-2-oxo-bicyclo[2.2.1]hept-1-ylmethanesulfonylamino)-4-oxo-pentanoic acid;
5-(7,7-Dimethyl-2-oxo-bicyclo[2.2.1]hept-1-ylmethanesulfonylamino)-3-[3-methyl-2-(phenethylcarbamoyl-methyl)-butyrylamino]-4-oxo-pentanoic acid;
3-(2-Carbamoylmethyl-3-methyl-butytylamino)-4-oxo-5-(2-phenyl-ethanesulfonyl-amino)-pentanoic acid;
3-[3-Methyl-2-(phenethylcarbamoyl-methyl)-butyrylamino]-4-oxo-5-(2-phenyl-ethanesulfonylamino)-pentanoic acid;
5-(7,7-Dimethyl-bicyclo[2.2.1]hept-1-ylmethanesulfonylamino)-3-[3-methyl-2-(phenethylcarbamoyl-methyl)-butyrylamino]-4-oxo-pentanoic acid;
(S,S)-3-{3-Methyl-2-[(3-phenyl-propylcarbamoyl)-methyl]-butyrylamino}-4-oxo-butyric acid;
3-{3-Methyl-2-[(3-phenoxy-ethylcarbamoyl)-methyl]-butyrylamino}-4-oxo-butyric acid;
5-(7,7-Dimethyl-2-oxo-bicyclo[2.2.1]hept-1-ylmethanesulfonylamino)-3-{3-methyl-2-[(2-phenoxy-ethylcarbamoyl)-methyl]-butyrylamino}-4-oxo-pentanoic acid;
3-[3-Methyl-2-(phenethylcarbamoyl-methyl)-butyrylamino]-4-oxo-5-(3-phenyl-propylsulfanyl)-pentanoic acid;
5-(7,7-Dimethyl-2-oxo-bicyclo[2.2.1]hept-1-ylmethanesulfonylamino)-3-{3-methyl-2-[(methyl-phenethyl-carbamoyl)-methyl]-butyrylamino}-4-oxopertanoic acid;
5-(7,7-Dimethyl-2-oxo-bicyclo[2.2.1]hept-1-ylmethanesulfonylamino)-3-{3-methyl-2-[(3-phenyl-propylcarbamoyl)-methyl]-butyrylamino}-4-oxo-pentanoic acid;
3-{3-Methyl-2-[(3-phenyl-propylcarbamoyl)-methyl]-butyrylamino}-4-oxo-5-(2-oxo-2H-chromen-6-yloxy)-pentanoic acid;
5-[3-(1H-Imidazol-2-yl)-naphthalen-2-yloxy]-3-{3-methyl-2-[(3-phenyl-propylcarbamoyl)-methyl]-butyrylamino}-4-oxo-pentanoic acid;

5-(7,7-Dimethyl-2-oxo-bicyclo[2.2.1]hept-1-ylmethanesulfonylamino)-3-(2-hydroxycarbamoylmethyl-3-methyl-butyrylamino)-4-oxo-pentanoic acid;
5-(7,7-Dimethyl-2-oxo-bicyclo[2.2.1]hept-1-ylmethanesulfonylamino)-3-(2-{[2-(1H-indol-3-yl)-ethylcarbamoyl]-methyl}-3-methyl-butyrylamino)-4-oxo-pentanoic acid; or
5-(7,7-Dimethyl-2-oxo-bicyclo[2.2.1]hept-1-ylmethanesulfonylamino)-3-{3-methyl-2-[[3-(4-hydroxyphenyl)-propylcubamoyl]-methyl]-butyrylamino}-4-oxo-pentanoic acid.

**11.** The compound:

5-(7,7-Dimethyl-2-oxo-bicyclo[2.2.1]hept-1-ylmethanesulfonylamino)-3-(2-{[2-(1H-indol-3-yl)-ethylcarbamoyl]-methyl}-3-methyl-butyryl amino)-4-oxo-pentanoic acid;
5-(7,7-Dimethyl-2-oxo-bicyclo[2.2.1]hept-1-ylmethanesulfonylamino)-3-(2-{[2-(1H-indol-3-yl)-ethylcarbamoyl]-methyl}-3-methyl-butyrylamino)-4-oxo-pentanoic acid;
5-(7,7-Dimethyl-2-oxo-bicyclo[2.2.1]hept-1-ylmethanesulfonylami no)-3-(3-methyl-2-{[2-(1-methyl-1H-indol-3-yl)-ethylcarbamoyl]-methyl}-butyrylamino)-4-oxo-pentanoic acid;
5-(7,7-Dimethyl-2-oxo-bicyclo[2.2.1]hept-1-ylmethanesulfonylamino)-3-(3-methyl-2-{[2-(7-methyl-1H-indol-3-yl)-ethylcarbamoyl]-methyl}-butyrylamino)-4-oxo-pentanoic acid;
5-(7,7-Dimethyl-2-oxo-bicyclo[2.2.1]hept-1-ylmethanesulfonylamino)-3-(2-{[2-(6-fluoro-1H-indol-3-yl)-ethylcarbamoyl]-methyl}-3-methyl-butyrylamino)-4-oxo-pentanoic acid;
5-(7,7-Dimethyl-2-oxo-bicyclo[2.2.1]hept-1-ylmethanesulfonylamino)-3-[3-methyl-2-({methyl-[2-(1-methyl-1H-indol-3-yl)-ethyl]-carbamoyl}-methyl)-butyrylamino]-4-oxo-pentanoic acid;
3-{2-[(2-Benzoimidazol-1-yl-ethylcarbamoyl)-methyl]-3-methyl-butyrylamino}-5-(7,7-dimethyl-2-oxo-bicyclo [2.2.1]hept-1-ylmethanesulfonylamino)-4-oxo-pentanoic acid;
5-(7,7-Dimethyl-2-oxo-bicyclo[2.2.1]hept-1-ylmethanesulfonylamino)-3-(3-methyl-2-{[2-(1H-tetrazol-5-yl)-ethylcarbamoyl]-methyl}-butyrylamino)-4-oxo-pentanoic acid;
5-(7,7-Dimethyl-2-oxo-bicyclo[2.2.1]hept-1-ylmethanesulfonylamino)-4-oxo-3-{2-[(3-phenyl-propylcarbamoyl)-methyl]-butyrylamino}-pentanoic acid;
5-(7,7-Dimethyl-2-oxo-bicyclo[2.2.1]hept-1-ylmethanesulfonylamino)-3-(2-{[2-(1-methyl-1H-indol-3-yl)-ethylcarbamoyl]-methyl}-butyrylamino)-4-oxo-pentanoic acid;
3-(3-Methyl-2-{[2-(1-methyl-1H-indol-3-yl)-ethylcarbamoyl]-methyl}-butyrylamino)-4-oxo-butyric acid;
3-[3-Methyl-2-({methyl-[2-(1-methyl-1H-indol-3-yl)-ethyl]-carbamoyl}-methyl)-butyrylamino]-4-oxo-butyric acid;
3-{2-[(2-Benzoimidazol-1-yl-ethylcarbamoyl)-methyl]-3-methyl-butyrylamino}-4-oxo-butyric acid;
5-(7,7-Dimethyl-2-oxo-bicyclo[2.2.1]hept-1-ylmethanesulfonylamino)-3-(2-{[3-(4-hydroxy-phenyl)-propylcarbamoyl]-methyl}-3-methyl-butyrylamino)-4-oxo-pentanoic acid;
5-(7,7-Dimethyl-2-oxo-bicyclo[2.2.1]hept-1-ylmethanesulfonylamino)-3-{3-methyl-2-[(2-pyridin-4-yl-ethylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(2-naphthalen-2-yl-ethylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(3-pyridin-4-yl-propylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(3-quinolin-2-yl-propylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(3-naphthalen-2-yl-propylcarbamoyl)-methyl]-butyrylamino}-4-oxo-pentanoic acid; or .
5-(7,7-Dimethyl-2-oxo-bicyclo[2.2.1]hept-1-ylmethanesulfonylamino)-3-{3-methyl-2-[(3-pyridin-3-yl-propylcarbamoyl)-methyl]-butyrylamino}-4-oxo-pentanoic acid.

**12.** The compound:

5-(7,7-Dimethyl-2-oxo-bicyclo[2.2.1]hept-1-lmethanesulfonylamino)-3-{3-methyl-2-[(2-naphthalen-2-yl-ethylcarbamoyl)-methyl]-butyrylamino}-4-oxo-pentanoic acid;
5-(7,7-Dimethyl-2-oxo-bicyclo[2.2.1]hept-1-lmethanesulfonylamino)-3-(3-methyl-2-{[2-(7-methyl-1H-indol-3-yl)-ethylcarbamoyl]-methyl}-butyrylamino)-4-oxo-pentanoic acid;
5-(7,7-Dimethyl-2-oxo-bicyclo[2.2.1]hept-1-lmethanesulfonylamino)-3-(2-{[2-(6-fluoro-1H-indol-3-yl)-ethylcarbamoyl]-methyl}-3-methyl-butyrylamino)-4-oxo-pentanoic acid;
3-[3-Methyl-2-({methyl-[2-(1-methyl-1H-indol-3-yl)-ethyl]-carbamoyl}-methyl)-butyrylamino]-4-oxo-butyric acid;

3-(3-Methyl-2-{[methyl-(2-phenoxy-ethyl)-carbamoyl]-methyl}-butyrylamino)-4-oxo-butyric acid;
3-(2-{[2-(5,6-Dimethyl-benzoimidazol-1-yl)-ethylcarbamoyl]-methyl}-3-methyl-butyrylamino)-4-oxo-butyric acid, trifluoroacetate salt;
5-(7,7-Dimethyl-bicyclo[2.2.1]hept-1-ylmethanesulfonylamino)-3-{3-methyl-2-[(3-phenyl-propylcarbamoyl)-methyl]-butyrylamino}-4-oxo-pentanoic acid;
5-(7,7-Dimethyl-bicyclo[2.2.1]hept-1-ylmethanesulfonylamino)-3-(2-{[2-(1H-indol-3-yl)-ethylcarbamoyl]-methyl}-3-methyl-butyrylamino)-4-oxo-pentanoic acid;
5-(7,7-Dimethyl-2-oxo-bicyclo[2.2.1]hept-1-lmethanesulfonylamino)-3-{3-methyl-2-[(3-pyridin-4-yl-propylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(3-quinolin-2-yl-propylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(3-naphthalen-1-yl-propylcarbamoyl)-methyl]-butyrylamino}-4-oxo-pentanoic acid;
5-(7,7-Dimethyl-2-oxo-bicyclo[2.2.1]hept-1-ylmethanesulfonylamino)-3-{3-methyl-2-[(3-pyridin-3-yl-propylcarbamoyl)-methyl]-bntyrylamino}-4-oxo-pentanoic acid; or
3-{2-[(2-Benzoimidazol-1-yl-ethylcarbamoyl)-methyl]-3-methyl-butyrylamino}-5-(7,7-dimethyl-2-oxo-bicyclo [2.2.1]hept-1-ylmethanesulfonylamino)-4-oxo-pentanoic acid.

**13.** The compound:

5-(7,7-Dimethyl-2-oxo-bicyclo[2.2.1]hept-1-ylmethanesulfonylamino)-3-(3-methyl-2-{[2-(1-methyl-1H-indol-3-yl)-ethylcarbamoyl]-methyl}-butyrylamino)-4-oxo-pentanoic acid;
5-(7,7-Dimethyl-2-oxo-bicyclo[2.2.1]hept-1-ylmethanesulfonylamino)-3-{3-methyl-2-[(2-pyridin-4-yl-ethylcarbamoyl)-methyl]-butyrylamino}-4-oxo-pentanoic acid;
3-(2-{[2-(5-Acetyl-1H-indol-3-yl)-ethylcarbamoyl]-methyl}-3-methyl-butyrylamino)-5-(7,7-dimethyl-2-oxo-bicyclo[2.2.1]hept-1-ylmethanesulfonylamino)-4-oxo-pentanoic acid;
5-(7,7-Dimethyl-2-oxo-bicyclo[2.2.1]hept-1-ylmethanesulfonylamino)-3-(3-methyl-2-{[2-(1H-tetrazol-5-yl)-ethylcarbamoyl]-methyl}-butyrylamino)-4-oxo-pentanoic acid;
$N^4$-(2-Benzoimidazol-1-yl-ethyl)-$N^1$-(2-ethoxy-5-oxo-tetrahydro-furan-3-yl)-2-isopropyl-succinamide;
$N^1$-(2-Ethoxy-5-oxo-tetrahydro-furan-3-yl)-$N^4$-[2-(1H-indol-3-yl)-ethyl]-2-isopropyl-succinamide;
$N^1$-(2-Ethoxy-5-oxo-tetrahydro-furan-3-yl)-2-isopropyl-$N^4$-[2-(1-methyl-1H-indol-3-yl)-ethyl]-succinamide;
$N^4$-[2-(5,6-Dichloro-benzoimidazol-1-yl)-ethyl]-$N^1$-(2-ethoxy-5-oxo-tetrahydro-furan-3-yl)-2-isopropyl-succinamide;
$N^1$-(2-Ethoxy-5-oxo-tetrahydro-furan-3-yl)-2-isopropyl-$N^4$-(2-phenoxy-ethyl)-succinamide; or
$N^1$-(2-Ethoxy-5-oxo-tetrahydro-furan-3-yl)-2-isopropyl-$N^4$-[2-(6-methoxy-1H-indol-3-yl)-ethyl]-succinamide.

**14.** The compound:

$N^4$-(2-Benzotriazol-1-yl-ethyl)-$N^1$-(2-ethoxy-5-oxo-tetrahydro-furan-3-yl)-2-isopropyl-succinamide;
$N^1$-(2-Ethoxy-5-oxo-tetrahydro-furan-3-yl)-$N^4$-(2-indazol-1-yl-ethyl)-2-isopropyl-succinamide;
$N^1$-(2-Ethoxy-5-oxo-tetrahydro-furan-3-yl)-2-isopropyl-$N^4$-(2-phenylamino-ethyl)-succinamide;
$N^1$-(2-Ethoxy-5-oxo-tetrahydro-furan-3-yl)-$N^4$-[2-(6-fluoro-1H-indol-3-yl)-ethyl]-2-isopropyl-succinamide;
$N^1$-(2-Ethoxy-5-oxo-tetrahydro-furan-3-yl)-$N^4$-[2-(7-methyl-1H-indol-3-yl)-ethyl]-2-isopropyl-succinamide;
$N^1$-(2-ethoxy-5-oxotetrahydro-furan-3-yl)-2-isopropyl-$N^4$-[2-(2-methyl-benzoimidazol-1-yl)-ethyl]-succinamide;
$N^1$-(2-Ethoxy-5-oxo-tetrahydro-furan-3-yl)-2-isopropyl-$N^4$-[3-(3,4,5-trimethoxy-phenyl)-propyl]-succinamide;
$N^1$-(2-Ethoxy-5-oxo-tetrahydro-furan-3-yl)-2-isopropyl-$N^4$-[2-(phenyl)-ethyl]-succinamide;
$N^1$-(2-Ethoxy-5-oxo-tetrahydro-furan-3-yl)-2-isopropyl-$N^4$-[4-(phenyl)-butyl]-succinamide; or
$N^1$-(2-Ethoxy-5-oxo-tetrahydro-furan-3-yl)-$N^4$-(2-indol-1-yl-ethyl)-2-isopropyl-succinamide.

**15.** The compound:

$N^1$-(2-Ethoxy-5-oxo-tetrahydro-furan-3-yl)-2-isopropyl-$N^4$-[4-(phenyl)-propyl]-succinamide;
$N^1$-(2-Ethoxy-5-oxo-tetrahydro-furan-3-yl)-$N^4$-[2-(5-fluoro-1H-indol-3-yl)-ethyl]-2-isopropyl-succinamide;
3-{2-[(2-Benzoimidazol-1-yl-ethylcarbamoyl)-methyl]-3-methyl-butyrylamino}-4-oxo-butyric acid;
3-(3-Methyl-2-{[2-(1-methyl-1H-indol-3-yl)-ethylcarbamoyl]-methyl}-butyrylamino)-4-oxo-butyric acid;
3-(2-{[2-(5-Fluoro-1-methyl-1H-indol-3-yl)-ethylcarbamoyl]-methyl}-3-methyl-butyrylamino)-4-oxo-butyric acid;

3-(2-{[2-(5,6-Dichloro-benzoimidazol-1-yl)-ethylcarbamoyl]-methyl}-3-methyl-butyrylamino)-4-oxo-butyric acid;
3-(2-{[(2-Benzoimidazol-1-yl-ethyl)-methyl-carbamoyl]-methyl}-3-methyl-butyrylamino)-4-oxo-butyric acid;
3-{2-[(2-Benzotriazol-1-yl-ethylcarbamoyl)-methyl]-3-methyl-butyrylamino}-4-oxo-butyric acid; .
3-{2-[(2-Indazol-1-yl-ethylcarbamoyl)-methyl]-3-methyl-butyrylamino)-4-oxo-butyric acid;
3-[2-({[2-(5,6-Dimethyl-benzoimidazal-1-yl)-ethyl]-methylcarbamoyl}-methyl)-3-methyl-butyrylamino]4-oxo-burytic acid;
3-[2-({[2-(2-Methyl-benzoimidazol-1-yl)-ethyl]-methylcarbamoyl}-methyl)-3-methyl-butyrylamino]4-oxo-butyric add;
3-(3-methyl-2-{[3-(3,4,5-trimethoxy-pheny)-propylcarbamoyl]-methyl}-butyrylamino)-4-oxo-butyric acid;
3-{3-Methyl-2-[(2-phenoxy-ethylcarbamoyl)-methyl]-butyrylamino}-4-oxo-butyric acid ethyl ester;
3-Cyano-3-{3-methyl-2-[(2-phenoxy-ethylcarbamoyl)-methyl]-butyrylamino}-propionic acid ethyl ester; or
3-Cyano-3-{3-methyl-2-[(2-phenoxy-ethylcarbamoyl)-methyl]-butyrylamino}-propionic acid.

**16.** A pharmaceutical composition that comprises a compound of any one of Claims 1 to 15.

**17.** Use of a compound of any one of Claims 1 to 15 for preparing a pharmaceutical composition for treating or preventing stroke.

**18.** Use of a compound of any one of Claims 1 to 15 for preparing a pharmaceutical composition for treating inflammatory diseases.

**19.** The use of Claim 18 wherein the inflammatory disease is rheumatoid arthritis or inflammatory bowel disease.

**20.** Use of a compound of any one of Claims 1 to 15 for preparing a pharmaceutical composition for treating septic shock.

**21.** Use of a compound of any one of Claims 1 to 15 for preparing a pharmaceutical composition for treating reperfusion injury.

**22.** Use of a compound of any one of Claims 1 to 15 for preparing a pharmaceutical composition for treating Alzheimer's disease.

**23.** Use of a compound of any one of Claims 1 to 15 for preparing a pharmaceutical composition for treating shigellosis.

**24.** Use of a compound of any one of Claims 1 to 15 for preparing a pharmaceutical composition for multiple sclerosis.

**Patentansprüche**

**1.** Verbindung der Formel I

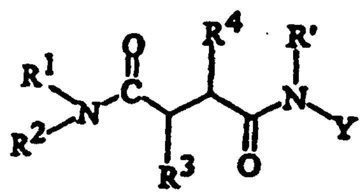

I

worin
Y für

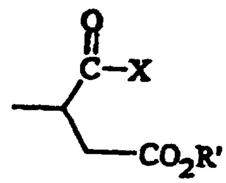

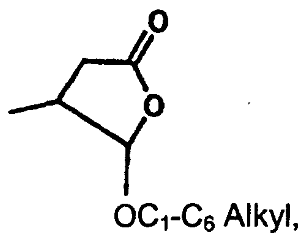

oder

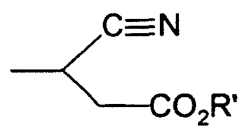

steht.

Jedes R' unabhängig für Wasserstoff oder $C_1$-$C_6$ Alkyl steht;

$R^1$ und $R^2$ unabhängig voneinander für Wasserstoff, $C_1$-$C_6$ Alkyl,

- -OH, $-(CH_2)_n$ Aryl,
- $-(CH_2)_n$ substituiertes Aryl,
- $-(CH_2)_n$-O-Aryl,
- $-(CH_2)_n$-O-substituiertes Aryl,
- $-(CH_2)_n$-S-Aryl,
- $-(CH_2)_n$-S-substituiertes Aryl,
- $-(CH_2)_n$-S-Heteroaryl
- $-(CH_2)_n$-S-substituiertes Heteroaryl,
- $-(CH_2)_n$-NR'-Aryl,
- $-(CH_2)_n$-NR'-substituiertes Aryl
- $-(CH_2)_n$-NR'-Heteroaryl,
- $-(CH_2)_n$-NR'-substituiertes Heteroaryl,
- $-(CH_2)_n$-Heteroaryl, oder
- $-(CH_2)_n$-substituiertes Heteroaryl stehen;

jedes n unabhängig für 0 bis 6 steht;

$R^3$ für Wasserstoff oder $C_1$-$C_6$ Alkyl steht;

$R^4$ für $C_1$-$C_6$ Alkyl oder Wasserstoff steht; und

X für Wasserstoff,

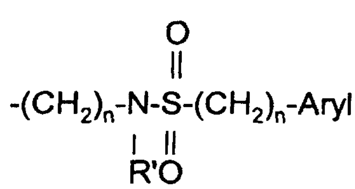

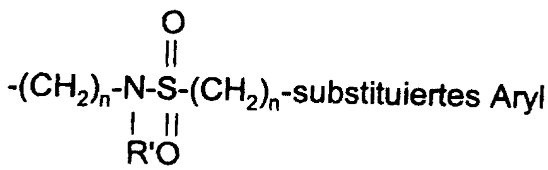

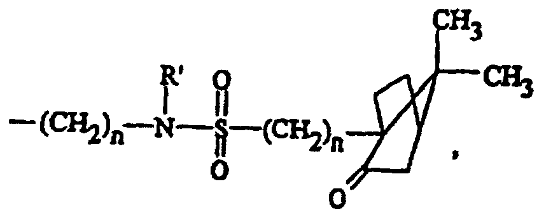

,

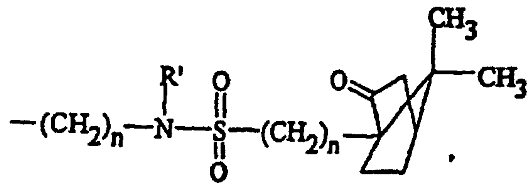

,

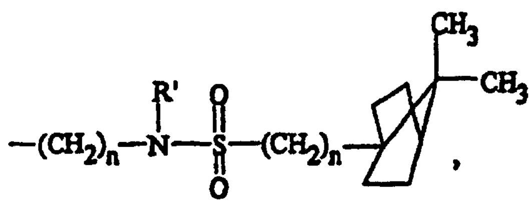

,

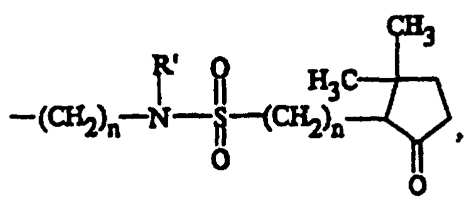

,

$-(CH_2)_n-S-(CH_2)_n-$Aryl,

$-(CH_2)_n-S-(CH_2)_n-$substituiertes Aryl

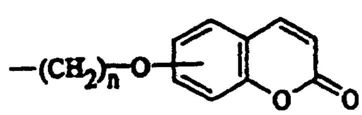

oder

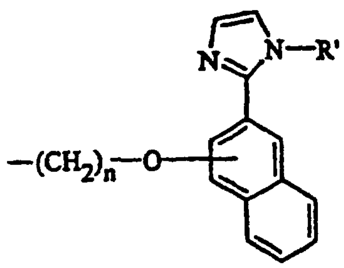

steht
und die pharmazeutisch akzeptablen Salze davon.

**2.** Verbindung nach Anspruch 1, worin R' Wasserstoff oder Methyl ist.

**3.** Verbindung nach Anspruch 1, worin jedes R' Wasserstoff ist.

**4.** Verbindung nach Anspruch 1, worin
$R^3$ für Wasserstoff und
$R^4$ für Methyl, Ethyl oder Isopropyl stehen.

**5.** Verbindung nach Anspruch 1, worin
$R^1$ für Wasserstoff oder Methyl steht und
$R^2$ für $-(CH_2)_n$-Phenyl,

Wasserstoff,
$-(CH_2)_n$-O-Phenyl,
-OH,
$-(CH_2)_n$-Benzimidazoyl,
$-(CH_2)_n$-Indolyl, oder
$-(CH_2)_n$-Phenol

steht.

**6.** Verbindung nach Anspruch 1, worin Y für

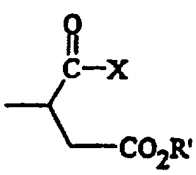

steht.

**7.** Verbindung nach Anspruch 1, worin Y für

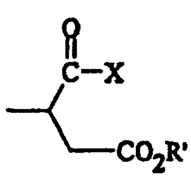

steht,

X für Wasserstoff,

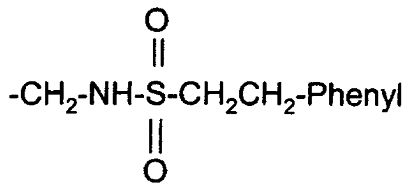

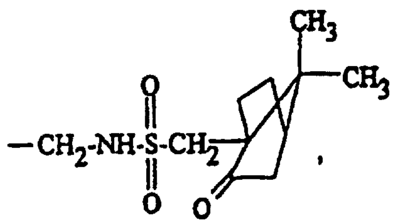
,

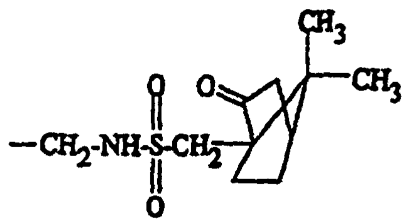
,

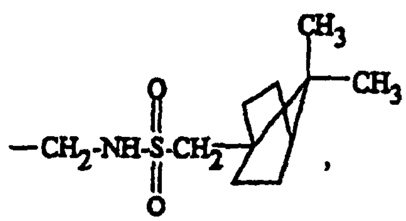
,

$-CH_2-S-CH_2CH_2CH_2$ -Phenyl

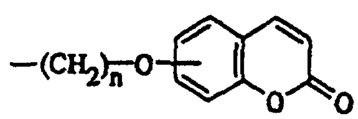

oder

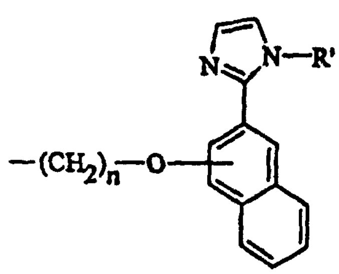

steht.

**8.** Verbindung der Formel I:

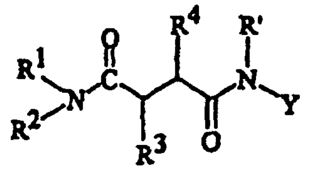

I

worin Y für

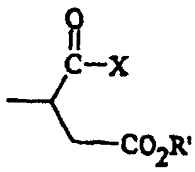

,

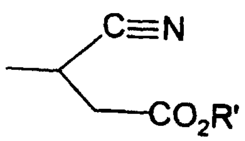

,

oder

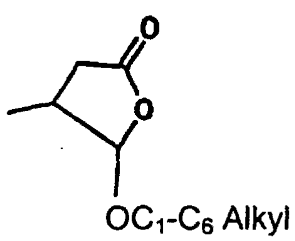

steht;
jedes R' unabhängig für Wasserstoff oder Methyl steht;
jedes n unabhängig für 1, 2 oder 3 steht;
$R^1$ und $R^2$ unabhängig für Wasserstoff,

$-(CH_2)_n$-Phenyl,

$-(CH_2)_n$-O-Phenyl

-OH,

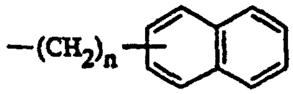,

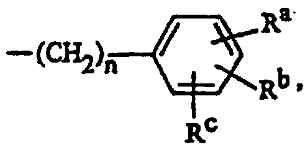,

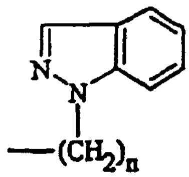,

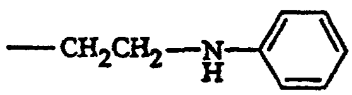,

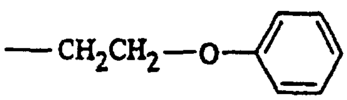,

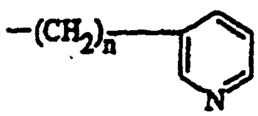,

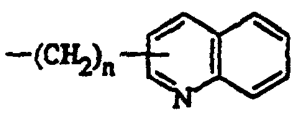,

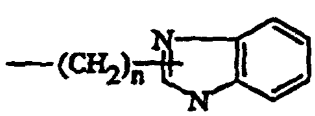,

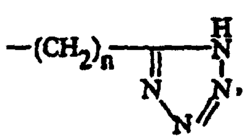,

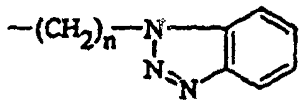,

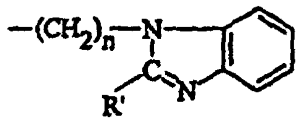,

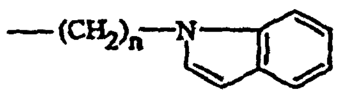,

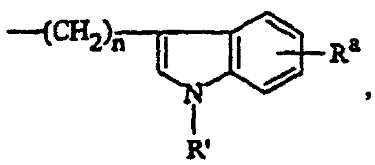,

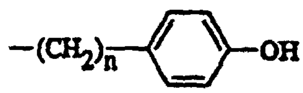;

stehen;
$R^a$, $R^b$ und $R^c$ unabhängig für Halogen, $-OC_1-C_6$ Alkyl oder Wasserstoff stehen;
$R^3$ für Wasserstoff steht;
$R^4$ für Methyl, Ethyl oder Isopropyl steht und
X für Wasserstoff,

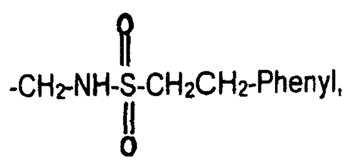,

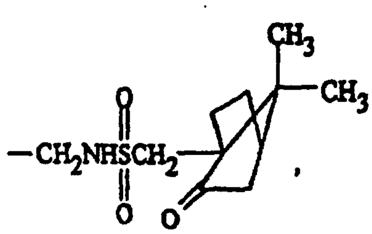,

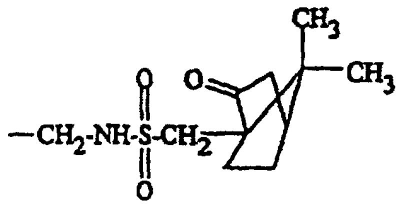

,

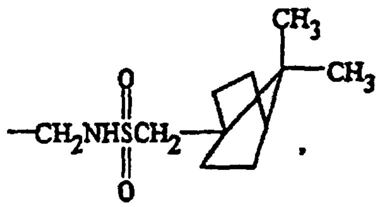

,

$-CH_2-S-CH_2CH_2CH_2$-Phenyl,

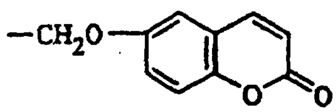

,

oder

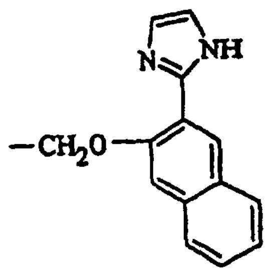

steht
und die pharmazeutisch akzeptablen Salze davon.

**9.** Verbindung nach Anspruch 1, worin
$R^1$ für Wasserstoff steht und
$R^2$ für

$-(CH_2)_n$-Indolyl,
$-(CH_2)_n$-NH-Phenyl,
$-(CH_2)_n$-O-Phenyl,
$-(CH_2)_n$-substituiertes Indolyl,
$-(CH_2)_n$-Tetrazolyl,
$-(CH_2)_n$-Phenyl,
$-(CH_2)_n$-substituiertes Phenyl,
$-(CH_2)_n$-substituiertes Benzimidazolyl,
$-(CH_2)_n$-Benztriazolyl,

$-(CH_2)_n$-Indazolyl,
$-(CH_2)_n$-Benzimidazolyl,
$-(CH_2)_n$-Pyridyl,
$-(CH_2)_n$-Naphthyl oder
$-(CH_2)_n$-Quinolinyl

steht.

**10.** Die Verbindung:

3-(2-Methyl-3-phenethylcarbamoyl-propionylamino)-4-oxo-5-(2-phenyl-ethansulfonylamino)-pentansäure;
3-(2-Carbamoylmethyl-3-methyl-butyrylamino)-5-(7,7-dimethyl-2-oxo-bicyclo[2.2.1]hept-1-ylmethansulfonylamino)-4-oxo-pentansäure;
5-(7,7-Dimethyl-2-oxo-bicyclo[2.2.1]hept-1-ylmethansulfonylamino)-3-[3-methyl-2-(phenethylcarbamoyl-methyl)-butyrylamino]-4-oxo-pentansäure;
3-(2-Carbamoylmethyl-3-methyl-butyrylamino)-4-oxo-5-(2-phenyl-ethansulfonyl-amino)-pentansäure;
3-[3-Methyl-2-(phenethylcarbamoyl-methyl)-butyrylamino-4-oxo-5-(2-phenyl-ethansulfonylamino)-pentansäure;
5-(7,7-Dimethyl-bicyclo[2.2.1]hept-1-ylmethansulfonylamino)-3-[3-methyl-2-(phenethylcarbamoyl-methyl)-butyrylamino]-4-oxo-pentansäure;
(S,S)-3-{3-Methyl-2-[(3-phenyl-propylcarbamoyl)-methyl]-butyrylamino}-4-oxo-buttersäure;
3-{3-Methyl-2-[(3-phenoxy-ethylcarbamoyl)-methyl]-butyrylamino}-4-oxo-buttersäure;
5-(7,7-Dimethyl-2-oxo-bicyclo[2.2.1]hept-1-ylmethansulfonylamino)-3-{3-methyl-2-[(2-phenoxy-ethylcarbamoyl)-methyl]-butyrylamino}-4-oxo-pentansäure;
3-[3-Methyl-2-(phenethylcarbamoyl-methyl)-butyrylamino]-4-oxo-5-(3-phenyl-propylsulfanyl)-pentansäure;
5-(7,7-Dimethyl-2-oxo-bicyclo[2.2.1]hept-1-ylmethansulfonylamino)-3-{3-methyl-2-[(methyl-phenethyl-carbamoyl)-methyl]-butyrylamino}-4-oxo-pentansäure;
5-(7,7-Dimethyl-2-oxo-bicyclo[2.2.1]hept-1-ylmethansulfonylamino)-3-{3-methyl-2-[(3-phenyl-propylcarbamoyl)-methyl]-butyrylamino}-4-oxo-pentansäure;
3-{3-Methyl-2-[(3-phenyl-propylcarbamoyl)-methyl]-butyrylamino}-4-oxo-5-(2-oxo-2H-chromen-6-yloxy)-pentansäure;
5-[3-( 1 H-Imidazol-2-yl)-naphthalin-2-yloxy]-3-{3-methyl-2-[(3-phenyl-propylcarbamoyl)-methyl]-butyrylamino}-4-oxo-pentansäure;
5-(7,7-Dimethyl-2-oxo-bicyclo[2.2.1]hept-1-ylmethansulfonylamino)-3-(2-hydroxycarbamoylmethyl-3-methyl-butyrylamino)-4-oxo-pentansäure;
5-(7,7-Dimethyl-2-oxo-bicyclo[2.2.1]hept-1-ylmethansulfonylamino)-3-(2-{[2-(1H-indol-3-yl)-ethylcarbamoyl]-methyl}-3-methyl-butyrylamino)-4-oxo-pentansäure; oder
5-(7,7-Dimethyl-2-oxo-bicyclo[2.2.1]hept-1-ylmethansulfonylamino)-3-{3-methyl-2-[[3-(4-hydroxyphenyl)-propylcarbamoyl)-methyl]-butyrylamino}-4-oxo-pentansäure.

**11.** Die Verbindung:

5-(7,7-Dimethyl-2-oxo-bicyclo[2.2.1]hept-1-ylmethansulfonylamino)-3-(2-{[2-(1H-indol-3-yl)-ethylcarbamoyl]-methyl}-3-methyl-butyrylamino)-4-oxo-pentansäure;
5-(7,7-Dimethyl-2-oxo-bicyclo[2.2.1]hept-1-ylmethansulfonylamino)-3-(2-{[2-(1H-indol-3-yl)-ethylcarbamoyl]-methyl}-3-methyl-butyrylamino)-4-oxo-pentansäure;
5-(7,7-Dimethyl-2-oxo-bicyclo[2.2.1]hept-1-ylmethansulfonylamino)-3-(3-methyl-2-{[2-(1-methyl-1H-indol-3-yl)-ethylcarbamoyl]-methyl}-butyrylamino)-4-oxo-pentansäure;
5-(7,7-Dimethyl-2-oxo-bicyclo[2.2.1]hept-1-ylmethansulfonylamino)-3-(3-methyl-2-{[2-(7-methyl-1H-indol-3-yl)-ethylcarbamoyl]-methyl}-butyrylamino)-4-oxo-pentansäure;
5-(7,7-Dimethyl-2-oxo-bicyclo[2.2.1]hept-1-ylmethansulfonylamino)-3-(2-{[2-(6-fluoro-1H-indol-3-yl)-ethylcarbamoyl]-methyl}-3-methyl-butyrylamino)-4-oxo-pentansäure;
5-(7,7-Dimethyl-2-oxo-bicyclo[2.2.1]hept-1-ylmethansulfonylamino)-3-[3-methyl-2-({methyl-[2-(1-methyl-1H-indol-3-yl)-ethyl]-carbamoyl}-methyl)-butyrylamino]-4-oxo-pentansäure;
3-{2-[(2-Benzoimidazol-1-yl-ethylcarbamoyl)-methyl-3-methyl-butyrylamino}-5-(7,7-dimethyl-2-oxo-bicyclo [2.2.1]hept-1-ylmethansulfonylamino)-4-oxo-pentansäure;
5-(7,7-Dimethyl-2-oxo-bicyclo[2.2.1]hept-1-ylmethansulfonylamino)-3-(3-methyl-2-{[2-(1H-tetrazol-5-yl)-ethylcarbamoyl]-methyl}-butyrylamino)-4-oxo-pentansäure;

5-(7,7-Dimethyl-2-oxo-bicyclo[2.2.1]hept-1-ylmethansulfonylamino)-4-oxo-3-{2-[(3-phenyl-propylcarbamoyl)-methyl]-butyrylamino}-pentansäure;
5-(7,7-Dimethyl-2-oxo-bicyclo[2.2.1]hept-1-ylmethansulfonylamino)3-(2-{[2-(1-methyl-1H-indol-3-yl)-ethylcarbamoyl]-methyl}-butyrylamino)-4-oxo-pentansäure;
3-(3-Methyl-2-{[2-(1-methyl-1H-indol-3-yl)-ethylcarbamoyl]-methyl}-butyrylamino)-4-oxo-buttersäure;
3-[3-Methyl-2-({methyl-[2-(1-methyl-1H-indol-3-yl)-ethyl]-carbamoyl}-methyl)-butyrylamino]-4-oxo-buttersäure;
3-{2-[(2-Benzimidazol-1-yl-ethylcarbamoyl)-methyl]-3-methyl-butyrylamino}-4-oxo-buttersäure;
5-(7,7-Dimethyl-2-oxo-bicyclo[2.2.1]hept-1-ylmethansulfonylamino)-3-(2-{[3-(4-hydroxy-phenyl)-propylcarbamoyl]-methyl}-3-methyl-butyrylamino)-4-oxo-pentansäure;
5-(7,7-Dimethyl-2-oxo-bicyclo[2.2.1]hept-1-ylmethansulfonylamino)-3-{3-methyl-2-[(2-pyridin-4-yl-ethylcarbamoyl)-methyl]-butyrylamino}-4-oxo-pentansäure;
5-(7,7-Dimethyl-2-oxo-bicyclo[2.2.1]hept-1-ylmethansulfonylamino)-3-{3-methyl-2-[(2-naphthalin-2-yl-ethylcarbamoyl)-methyl]-butyrylamino}-4-oxo-pentansäure;
5-(7,7-Dimethyl-2-oxo-bicyclo[2.2.1]hept-1-ylmethansulfonylamino)-3-{3-methyl-2-[(3-pyridin-4-yl-propylcarbamoyl)-methyl]-butyrylamino}-4-oxo-pentansäure;
5-(7,7-Dimethyl-2-oxo-bicyclo[2.2.1]hept-1-ylmethansulfonylamino)-3-{3-methyl-2-[(3-chinolin-2-yl-propylcarbamoyl)-methyl]-butyrylamino}-4-oxo-pentansäure;
5-(7,7-Dimethyl-2-oxo-bicyclo[2.2.1]hept-1-ylmethansulfonylamino)-3-{3-methyl-2-[(3-naphthalin-2-yl-propylcarbamoyl)-methyl]-butyrylamino}-4-oxo-pentansäure, oder
5-(7,7-Dimethyl-2-oxo-bicyclo[2.2.1]hept-1-ylmethansulfonylamino)-3-{3-methyl-2-[(3-pyridin-3-yl-propylcarbamoyl)-methyl]-butyrylamino}-4-oxo-pentansäure;

**12.** Die Verbindung:

5-(7,7-Dimethyl-2-oxo-bicyclo[2.2.1]hept-1-ylmethansulfonylamino)-3-{3-methyl-2-[(2-naphthalin-2-yl-ethylcarbamoyl)-methyl]-butyrylamino}-4-oxo-pentansäure;
5-(7,7-Dimethyl-2-oxo-bicyclo[2.2.1]hept-1-ylmethansulfonylamino)-3-{3-methyl-2-{[2-(7-methyl-1H-indol-3-yl)-ethylcarbamoyl]-methyl}-butyrylamino)-4-oxo-pentansäure;
5-(7,7-Dimethyl-2-oxo-bicyclo[2.2.1]hept-1-ylmethansulfonylamino)-3-(2-{[2-(6-fluoro-1H-indol-3-yl)-ethylcarbamoyl]-methyl}-3-methyl-butyrylamino)-4-oxo-pentansäure;
3-[3-Methyl-2-({methyl-[2-(1-methyl-1H-indol-3-yl)-ethyl]-carbamoyl}-methyl)-butyrylamino]-4-oxo-buttersäure;
3-(3-Methyl-2-{[methyl-(2-phenoxy-ethyl)-carbamoyl]-methyl}-butyrylamino)-4-oxo-buttersäure;
3-(2-{[2-(5,6-Dimethyl-benzimidazol-1-yl)-ethylcarbamoyl]-methyl}-3-methyl-butyrylamino)-4-oxo-buttersäure, Trifluoracetatsalz;
5-(7,7-Dimethyl-bicyclo[2.2.1]hept-1-ylmethansulfonylamino)-3-{3-methyl-2-[(3-phenyl-propylcarbamoyl)-methyl]-butyrylamino}-4-oxo-pentansäure;
5-(7,7-Dimethyl-bicyclo[2.2.1]hept-1-ylmethansulfonylamino)-3-(2-{[2-(1H-indol-3-yl)-ethylcarbamoyl)-methyl}-3-methyl-butyrylamino)-4-oxo-pentansäure;
5-(7,7-Dimethyl-2-oxo-bicyclo[2.2.1]hept-1-ylmethansulfonylamino)-3-{3-methyl-2-[(3-pyridin-4-yl-propylcarbamoyl)-methyl]-butyrylamino}-4-oxo-pentansäure;
5-(7,7-Dimethyl-2-oxo-bicyclo[2.2.1]hept-1-ylmethansulfonylamino)-3-{3-methyl-2-[(3-chinolin-2-yl-propylcarbamoyl)-methyl]-butyrylamino}-4-oxo-pentansäure;
5-(7,7-Dimethyl-2-oxo-bicyclo[2.2.1]hept-1-ylmethansulfonylamino)-3-{3-methyl-2-[(3-naphthalin-1-yl-propylcarbamoyl)-methyl]-butyrylamino}-4-oxo-pentansäure;
5-(7,7-Dimethyl-2-oxo-bicyclo[2.2.1]hept-1-ylmethansulfonylamino)-3-{3-methyl-2-[(3-pyridin-3-yl-propylcarbamoyl)-methyl]-butyrylamino}-4-oxo-pentansäure oder
3-{2-[(2-Benzimidazol-1-yl-ethylcarbamoyl)-methyl]-3-methyl-butyrylamino}-5-(7,7-dimethyl-2-oxo-bicyclo [2.2.1]hept-1-ylmethansulfonylamino)-4-oxo-pentansäure.

**13.** Die Verbindung:

5-(7,7-Dimethyl-2-oxo-bicyclo[2.2.1]hept-1-ylmethansulfonylamino)-3-(3-methyl-2-{[2-(1-methyl-1H-indol-3-yl)-ethylcarbamoyl]-methyl}-butyrylamino}-4-oxo-pentansäure;
5-(7,7-Dimethyl-2-oxo-bicyclo[2.2.1]hept-1-ylmethansulfonylamino)-3-{3-methyl-2-[(2-pyridin-4-yl-ethylcarbamoyl)-methyl]-butyrylamino}-4-oxo-pentansäure;
3-(2-{[2-(5-Acetyl-1H-indol-3-yl)-ethylcarbamoyl]-methyl}-3-methyl-butyrylamino)-5-(7,7-dimethyl-2-oxo-bicyclo[2.2.1]hept-1-ylmethansulfonylamino)-4-oxo-pentansäure;
5-(7,7-Dimethyl-2-oxo-bicyclo[2.2.1]hept-1-ylmethansulfonylamino)-3-(3-methyl-2-{[2-(1H-tetrazol-5-yl)-ethylcarbamoyl]-methyl}-butyrylamino)-4-oxo-pentansäure;
$N^4$-(2-Benzimidazol-1-yl-ethyl)-$N^1$-(2-ethoxy-5-oxo-tetrahydrofuran-3-yl)-2-isopropyl-succinamid;
$N^1$-(2-Ethoxy-5-oxo-tetrahydrofuran-3-yl)-$N^4$-[2-(1H-indol-3-yl)-ethyl]-2-isopropyl-succinamid;
$N^1$-(2-Ethoxy-5-oxo-tetrahydrofuran-3-yl)-2-isopropyl-$N^4$-[2-{1-methyl-1 H-indol-3-yl)-ethyl]-succinamid;
$N^4$-[2-(5,6-Dichloro-benzimidazol-1-yl)-ethyl]-$N^1$-(2-ethoxy-5-oxo-tetrahydrofuran-3-yl)-2-isopropyl-succinamid;
$N^1$-(2-Ethoxy-5-oxo-tetrahydrofuran-3-yl)-2-isopropyl-$N^4$-(2-phenoxy-ethyl)-succinamid oder
$N^1$-(2-Ethoxy-5-oxo-tetrahydrofuran-3-yl)-2-isopropyl-$N^4$-[2-(6-methoxy-1H-indol-3-yl)-ethyl]succinamid.

**14.** Die Verbindung:

$N^4$-(2-Benzotriazol-1-yl-ethyl)-$N^1$-(2-ethoxy-5-oxo-tetrahydrofuran-3-yl)-2-isopropyl-succinamid;
$N^1$-(2-Ethoxy-5-oxo-tetrahydrofuran-3-yl)-$N^4$-(2-indazol-1-yl-ethyl)-2-isopropyl-succinamid;
$N^1$-(2-Ethoxy-5-oxo-tetrahydrofuran-3-yl)-2-isopropyl-$N^4$-(2-phenyl-amino-ethyl)-succinamid;
$N^1$-(2-Ethoxy-5-oxo-tetrahydrofuran-3-yl)-$N^4$-[2-(6-fluoro-1H-indol-3-yl)-ethyl]-2-isopropyl-succinamid;
$N^1$-(2-Ethoxy-5-oxo-tetrahydrofuran-3-yl)-$N^4$-[2-(7-methyl-1H-indol-3-yl)-ethyl]-2-isopropyl-succinamid;
$N^1$-(2-Ethoxy-5-oxo-tetrahydrofuran-3-yl)-2-isopropyl-$N^4$-[2-(2-methyl-benzimidazol-1-yl)-ethyl]-succinamid;
$N^1$-(2-Ethoxy-5-oxo-tetrahydrofuran-3-yl)-2-isopropyl-$N^4$-[3-(3,4,5-trimethoxy-phenyl)-propyl]-succinamid;
$N^1$-(2-Ethoxy-5-oxo-tetrahydrofuran-3-yl)-2-isopropyl-$N^4$-[2-(phenyl)-ethyl]-succinamid;
$N^1$-(2-Ethoxy-5-oxo-tetrahydrofuran-3-yl)-2-isopropyl-$N^4$-[4-(phenyl)-butyl]-succinamid oder
$N^1$-(2-Ethoxy-5-oxo-tetrahydrofuran-3-yl)-$N^4$-(2-indol-1-yl)-ethyl)-2-isopropyl-succinamid.

**15.** Die Verbindung:

$N^1$-(2-Ethoxy-5-oxo-tetrahydrofuran-3-yl)-2-isopropyl-$N^4$-[4-(phenyl)-propyl]-succinamid.
$N^1$-(2-Ethoxy-5-oxo-tetrahydrofuran-3-yl)-$N^4$-[2-(5-fluoro-1H-indol-3-yl)-ethyl]-2-isopropyl-succinamid.
3-{2-[(2-Benzimidazol-1-yl-ethylcarbamoyl)-methyl]-3-methyl-butyrylamino-4-oxo-butyric acid;
3-(3-Methyl-2-{[2-(1-methyl-1H-indol-3-yl)-ethylcarbamoyl]-methyl}-butyrylamino)-4-oxo-buttersäure;
3-(2-{[2-(5-Fluoro-1-methyl-1H-indol-3-yl)-ethylcarbamoyl]-methyl-3-methyl-butyrylamino)-4-oxo-buttersäure;
3-(2-{[2-(5,6-Dichloro-benzimidazol-1-yl)-ethylcarbamoyl]-methyl}-3-methyl-butyrylamino)-4-oxo-buttersäure;
3-(2-{[(2-Benzimidazol-1-yl-ethyl)-methyl-carbamoyl]-methyl}-3-methyl-butyrylamino)-4-oxo-buttersäure;
3-{2-(2-Benzotriazol-1-yl-ethylcarbamoyl)-methyl]-3-methyl-butyrylamino}-4-oxo-buttersäure;
3-{2-[(2-Indazol-1-yl-ethylcarbamoyl)-methyl]-3-methyl-butyrylamino}-4-oxo-buttersäure;
3-[2-({[2-(5,6-Dimethyl-benzimidazol-1-yl)-ethyl]-methylcarbamoyl}-methyl)-3-methyl-butyrylamino]-4-oxo-buttersäure;
3-[2-({[2-(2-Methyl-benzimidazol-1-yl)-ethyl]-methylcarbamoyl}-methyl)-3-methyl-butyrylamino]-4-oxo-buttersäure
3-(3-Methyl-2-{[3-(3,4,5-trimethoxy-phenyl)-propylcarbamoyl]-methyl}-butyrylamino)-4-oxo-buttersäure;
3-{3-Methyl-2-[(2-phenoxy-ethylcarbamoyl)-methyl]-butyrylamino)-4-oxo-buttersäureethylester;
3-Cyano-3-(3-methyl-2-[(2-phenoxy-ethylcarbamoyl)-methyl]-butyrylamino}-propionsäureethylester oder
3-Cyano-3-(3-methyl-2-[(2-phenoxy-ethylcarbamoyl)-methyl]-butyrylamino)-propionsäure.

**16.** Pharmazeutische Zusammensetzung, welche eine Verbindung nach einem der Ansprüche 1 bis 15 umfasst.

**17.** Verwendung einer Verbindung nach einem der Ansprüche 1 bis 15 zur Herstellung einer pharmazeutischen Zusammensetzung zur Behandlung oder Prävention von Schlaganfall.

**18.** Verwendung einer Verbindung nach einem der Ansprüche 1 bis 15 zur Herstellung einer pharmazeutischen Zusammensetzung zur Behandlung inflammatorischer Erkrankungen.

**19.** Verwendung nach Anspruch 18, wobei die inflammatorische Erkrankung rheumatoide Arthritis oder inflammatory bowel disease ist.

**20.** Verwendung einer Verbindung nach einem der Ansprüche 1 bis 15 zur Herstellung einer pharmazeutischen Zusammensetzung zur Behandlung eines septischen Schocks.

**21.** Verwendung einer Verbindung nach einem der Ansprüche 1 bis 15 zur Herstellung einer pharmazeutischen Zusammensetzung zur Behandlung einer Reperfusionsverletzung.

**22.** Verwendung einer Verbindung nach einem der Ansprüche 1 bis 15 zur Herstellung einer pharmazeutischen Zusammensetzung zur Behandlung der Alzheimer Erkrankung.

**23.** Verwendung einer Verbindung nach einem der Ansprüche 1 bis 15 zur Herstellung einer pharmazeutischen Zusammensetzung zur Behandlung von Shigellose.

**24.** Verwendung einer Verbindung nach einem der Ansprüche 1 bis 15 zur Herstellung einer pharmazeutischen Zusammensetzung zur Behandlung multipler Sklerose.

**Revendications**

**1.** Composé de formule I

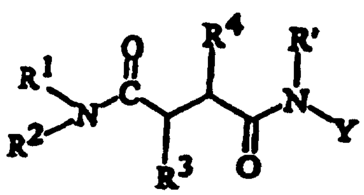

I

dans laquelle

Y est

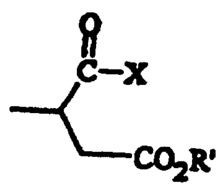

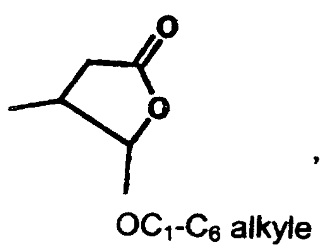

,

ou

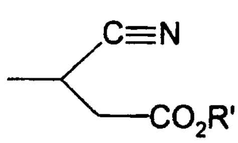

;

chaque R' est indépendamment un hydrogène ou un alkyle en $C_1$ à $C_6$ ;
$R^1$ et $R^2$ sont indépendamment un hydrogène, un alkyle en $C_1$ à $C_6$,

-OH, - $(CH_2)_n$aryle,
un -$(CH_2)_n$-aryle substitué,
-$(CH_2)_n$-O-aryle,
un-$(CH_2)_n$-O-aryle substitué,
-$(CH_2)_n$-S-aryle,
un-$(CH_2)_n$-S-aryle substitué,
-$(CH_2)_n$-S-hétéroaryle,
un -$(CH_2)_n$-S-hétéroaryle substitué,
- $(CH_2)_n$-NR'-aryle,
un-$(CH_2)_n$-NR'-aryle substitué,
-$(CH_2)_n$-NR'-hétéroaryle,
un-$(CH_2)_n$-NR'-hétéroaryle substitué,
-$(CH_2)_n$-hétéroaryle, ou
un-$(CH_2)_n$-hétéroaryle substitué ;

chaque n vaut indépendamment 0 à 6 ;
$R^3$ est un hydrogène ou un alkyle en $C_1$ à $C_6$ ;
$R^4$ est un alkyle en $C_1$ à $C_6$ ou un hydrogène ; et
X est un hydrogène,

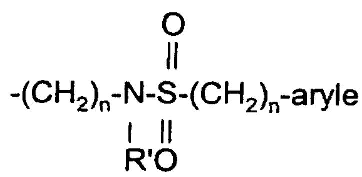

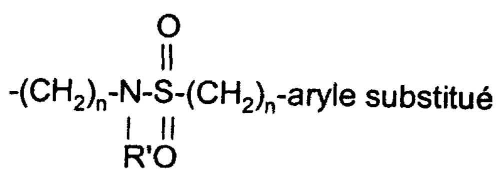

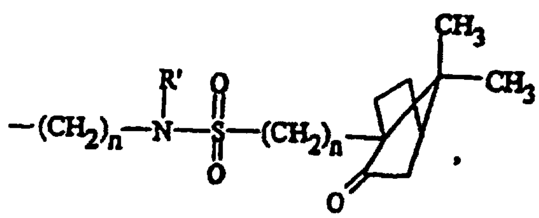
,

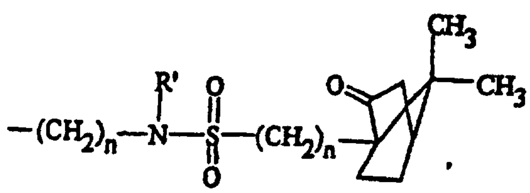
,

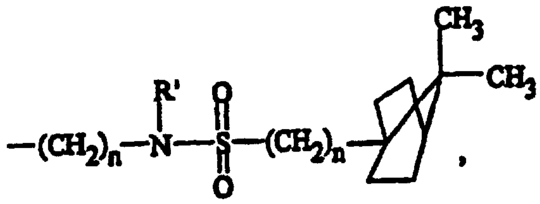,

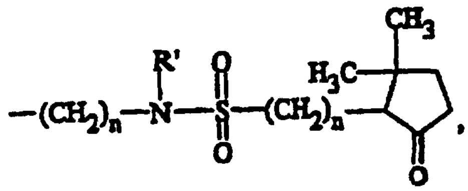,

- $(CH_2)_n$-S-$(CH_2)_n$-aryle,
Un -$(CH_2)_n$-S-$(CH_2)_n$-aryle substitué,

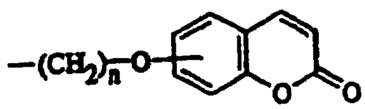,

ou

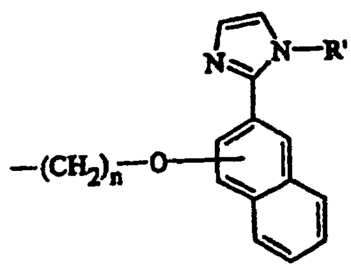;

et les sels pharmaceutiquement acceptables de ceux-ci.

**2.** Composé selon la revendication 1 dans lequel $R^1$ est un hydrogène ou un méthyle.

**3.** Composé selon la revendication 1 dans lequel chaque R' est un hydrogène.

**4.** Composé selon la revendication 1 dans lequel
$R^3$ est un hydrogène, et
$R^4$ est un méthyle, un éthyle, ou un isopropyle.

**5.** Composé selon la revendication 1 dans lequel
$R^1$ est un hydrogène ou un méthyle ; et
$R^2$ est -$(CH_2)_n$-phényle,
un hydrogène,
-$(CH_2)_n$-O-phényle,
-OH,
-$(CH_2)_n$-benzimidazoyle,
-$(CH_2)_n$-indolyle, ou

$-(CH_2)_n$-phénol.

**6.** Composé selon la revendication 1 dans lequel Y est

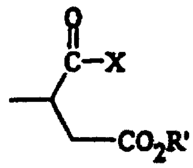

**7.** Composé selon la revendication 1 dans lequel Y est

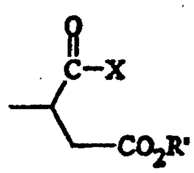

X est un hydrogène,

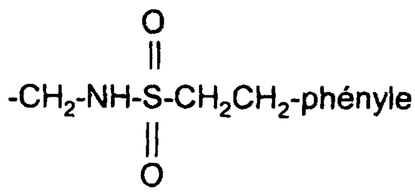

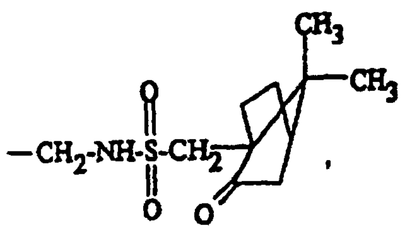

,

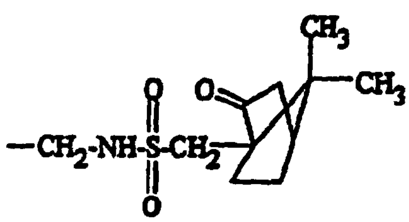

,

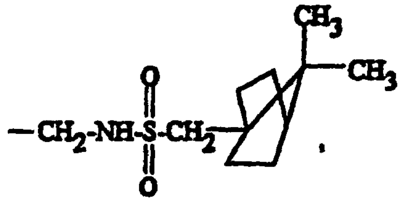

,

$-CH_2-S-CH_2CH_2CH_2$ -phényle

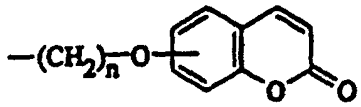

,

ou

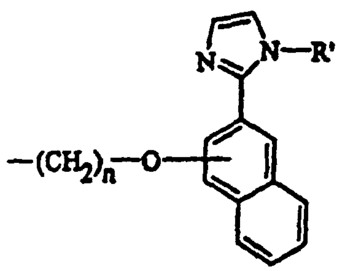

**8.** Composé de formule I :

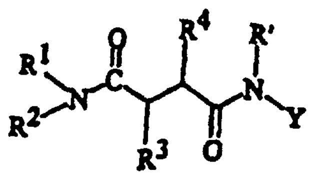

I

dans laquelle

Y est

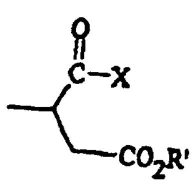

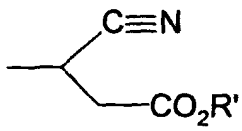

,

ou

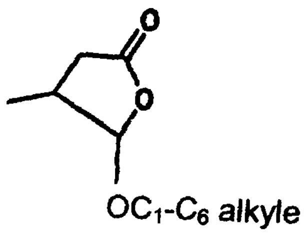

;

chaque R' est indépendamment un hydrogène ou un méthyle ;
chaque n vaut indépendamment 1, 2 ou 3 ;
$R^1$ et $R^2$ sont indépendamment un hydrogène,
- $(CH_2)_n$-phényle,
-$(CH_2)_n$-O-phényle,
-OH,

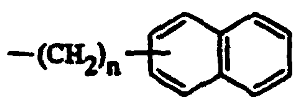

,

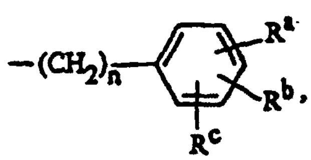

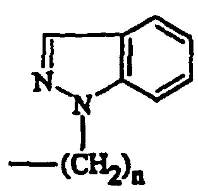

,

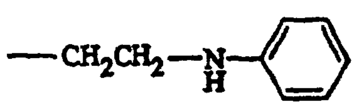

,

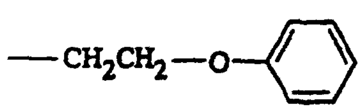

,

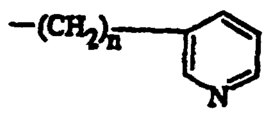

,

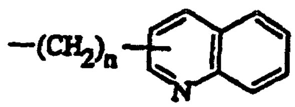

,

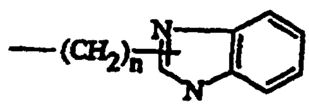

,

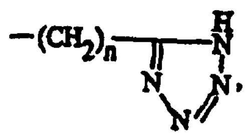

,

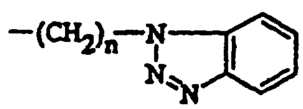

,

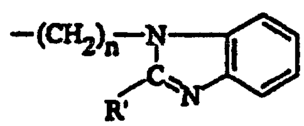

,

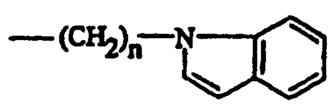

,

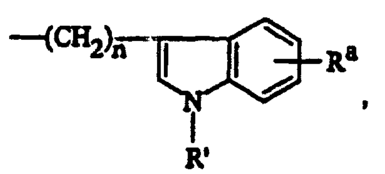

,

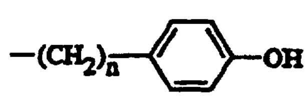

;

$R^a$, $R^b$ et $R^c$ sont indépendamment un halogène, un (alkyle en $C_1$ à $C_6$)-O- ou un hydrogène ;
$R^3$ est un hydrogène ;
$R^4$ est un méthyle, un éthyle, ou un isopropyle ; et
X est un hydrogène,

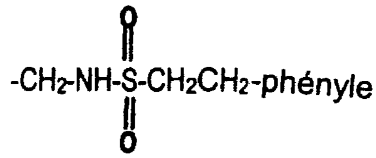

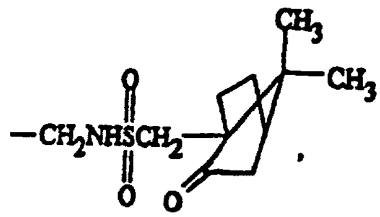
,

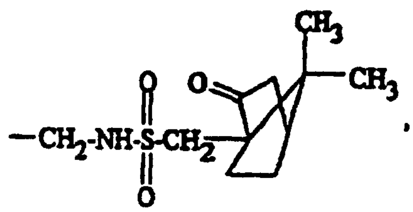
,

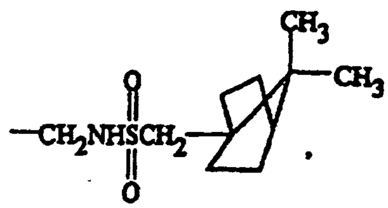
,

$-CH_2-S-CH_2CH_2CH_2-$phényle,

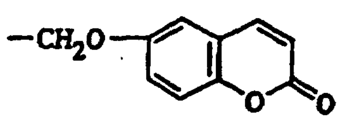
,

ou

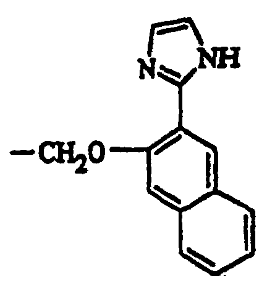
;

et les sels pharmaceutiquement acceptables de ceux-ci.

**9.** Composé selon la revendication 1 dans lequel
$R^1$ est un hydrogène et
$R^2$ est $-(CH_2)_n$-indolyle,
$-(CH_2)_n$-NH-phényle,
$-(CH_2)_n$-O-phényle,
un-$(CH_2)_n$-indolyle substitué,
$-(CH_2)_n$-tétrazolyle,
$-(CH_2)_n$-phényle,
Un $-(CH_2)_n$-phényle substitué,
un $-(CH_2)_n$-benzimidazolyle substitué,
$-(CH_2)_n$-benzotriazolyle,
$-(CH_2)_n$-indazolyle,
$-(CH_2)_n$-benzimidazolyle,
$-(CH_2)_n$-pyridyle,
$-(CH_2)_n$-naphtyle, ou
$-(CH_2)_n$-quinolinyle.

**10.** Composé :

acide 3-(2-méthyl-3-phénéthylcarbamoyl-propionyl amino)-4-oxo-5-(2-phényl-éthanesulfonylamino)-pentanoïque ;
acide 3-(2-carbamoylméthyl-3-méthyl-butyrylamino)-5-(7,7-diméthyl-2-oxo-bicyclo[2.2.1]hept-1-ylméthane sulfonylamino)-4-oxo-pentanoïque ;
acide 5-(7,7-diméthyl-2-oxo-bicyclo[2.2.1]hept-1-ylméthanesulfonylamino)-3-[3-méthyl-2(phénéthylcarbamoyl-méthyl)-butyrylamino]-4-oxo-pentanoïque ;
acide 3-(2-carbamoylméthyl-3-méthyl-butyrylamino-4-oxo-5-(2-phényl-éthanesulfonylamino)-pentanoïque ;
acide 3-(3-méthyl-2-(phénéthylcarbamoyl-méthyl)-butyrylamino)-4-oxo-5-(2-phényl-éthanesulfonylamino)-pentanoique ;
acide 5-(7,7-diméthyl-bicyclo[2.2.1]hept-1-ylméthane sulfonylamino)-3-[3-méthyl-2-(phénéthylcarbamoyl-méthyl)-butyrylamino]-4-oxo-pentanoïque ;
acide (*S,S*)-3-{3-méthyl-2-[(3-phényl-propylcarbamoyl) -méthyl]-butyrylamino}-4-oxo-butyrique ;
acide 3-{3-méthyl-2-[(3-phénoxy-éthylcarbamoyl)-méthyl]-butyrylamino}-4-oxo-butyrique ;
acide 5-(7,7-diméthyl-2-oxo-bicyclo[2.2.1]hept-1-ylméthanesulfonylamino)-3-{3-méthyl-2-[(2-phénoxy-éthylcarbamoyl)-méthyl]-butyrylamino}-4-oxo-pentanoïque ;
acide 3-[3-méthyl-2-(phénéthylcarbamoyl-méthyl)-butyrylamino]-4-oxo-5-(3-phényl-propylsulfanyl)-pentanoique ;
acide 5-(7,7-diméthyl-2-oxo-bicyclo[2.2.1]hept-1-ylméthanesulfonylamino)-3-{3-méthyl-2-[(méthyl-phénéthyl-carbamoyl)-méthyl]-butyrylamino}-4-oxo-pentanoïque ;
acide 5-(7,7-diméthyl-2-oxo-bicyclo[2.2.1]hept-1-ylméthanesulfonylamino)-3-{3-méthyl-2-[(3-phényl-propylcarbamoyl)-mêthyl]-butyrylamino}-4-oxo-pentanoïque ;
acide 3-{3-méthyl-2-[(3-phényl-propylcarbamoyl)-méthyl]-butyrylamino}-4-oxo-5-(2-oxo-2*H*-chromèn-6-yloxy)-pentanoïque ;
acide 5-[3-(1*H*-imidazol-2-yl)-naphtalèn-2-yloxy-3-(3-méthyl-2-[(3-phényl-propylcarbamoyl)-méthyl]-butyrylamino]-4-oxo-pentanoïque ;
acide 5-(7,7-diméthyl-2-oxo-bicyclo[2.2.1]hept-1-ylméthanesulfonylamino)-3-(2-hydroxycarbamoylméthyl-3-méthyl-butyrylamino}-4-oxo-pentanoïque ;
acide 5-(7,7-diméthyl-2-oxo-bicyclo[2.2.1]hept-1-ylméthanesulfonylamino)-3-(2-{[2-(1*H*-indol-3-yl)-éthylcarbamoyl]-méthyl}-3-méthyl-butyrylamino}-4-oxo-pentanoïque ; ou
acide 5-(7,7-diméthyl-2-oxo-bicyclo[2.2.1]hept-1-ylméthanesulfonylamino)-3-{3-méthyl-2-[[3-(4-hydroxyphényl)-propylcarbamoyl]-méthyl]-butyrylamino}-4-oxo-pentanoïque .

**11.** Composé :

acide 5-(7,7-diméthyl-2-oxo-bicyclo[2.2.1]hept-1-ylméthanesulfonylamino)-3-(2-{[2-(1*H*-indol-3-yl)-éthylcarbamoyl]-méthyl}-3-méthyl-butyrylamino}-4-oxo-pentanoïque ;
acide 5-(7,7-diméthyl-2-oxo-bicyclo[2.2.1]hept-1-ylméthanesulfonylamino)-3-(2-{[2-(1*H*-indol-3-yl)-éthylcarbamoyl]-méthyl}-3-méthyl-butyrylamino}-4-oxo-pentanoïque ;
acide 5-(7,7-diméthyl-2-oxo-bicyclo[2.2.1]hept-1-ylméthanesulfonylamino)-3-(3-méthyl-2-{[2-(1-méthyl-1*H*-indol-3-yl)-éthylcarbamoyl]-méthyl}-butyrylamino)-4-oxo-pentanoïque ;
acide 5-(7,7-diméthyl-2-oxo-bicyclo[2.2.1]hept-1-ylméthanesulfonylamino)-3-(3-méthyl-2-{[2-(7-méthyl-1*H*-indol-3-yl)-éthylcarbamoyl]-méthyl}-butyrylamino)-4-oxo-pentanoïque ;
acide 5-(7,7-diméthyl-2-oxo-bicyclo[2.2.1]hept-1-ylméthanesulfonylamino)-3-(2-{[2-(6-fluoro-1*H*-indol-3-yl)-éthylcarbamoyl]-méthyl}-3-méthyl-butyrylamino)-4-oxo-pentanoïque ;
acide 5-(7,7-diméthyl-2-oxo-bicyclo[2.2.1]hept-1-ylméthanesulfonylamino)-3-[3-méthyl-2-({méthyl-[2-(1-méthyl-1*H*-indol-3-yl)-éthyl]-carbamoyl}-méthyl)-butyrylamino]-4-oxo-pentanoïque ;
acide 3-{2-[(2-benzoimidazol-1-yl-éthylcarbamoyl)-méthyl]-3-méthyl-butyrylamino}-5-(7,7-diméthyl-2-oxo-bicyclo[2.2.1]hept-1-ylméthanesulfonylamino)-4-oxo-pentanoïque ;
acide 5-(7,7-diméthyl-2-oxo-bicyclo[2.2.1]hept-1-ylméthanesulfonylamino)-3-(3-méthyl-2-{[2-(1*H*-tétrazol-5-yl)-éthylcarbamoyl]-méthyl}-butyrylamino)-4-oxo-pentanoïque ;
acide 5-(7,7-diméthyl-2-oxo-bicyclo[2.2.1]hept-1-ylméthanesulfonylamino)-4-oxo-3-{2-[(3-phényl-propylcarbamoyl)-méthyl]-butyrylamino}-pentanoïque ;
acide 5-(7,7-diméthyl-2-oxo-bicyclo[2.2.1]hept-1-ylméthanesulfonylamino)-3-(2-{[2-(1-méthyl-1*H*-indol-3-yl)-éthylcarbamoyl]-méthyl}-butyrylamino)-4-oxo-pentanoique ;
acide 3-(3-méthyl-2-{[2-(1-méthyl-1*H*-indol-3-yl)-éthylcarbamoyl]-méthyl}-butyrylamino)-4-oxo-butyrique ;
acide 3-[3-méthyl-2-({méthyl-[2-(1-méthyl-1*H*-indol-3-yl)-éthyl]carbamoyl}-méthyl}-butyrylamino]-4-oxo-butyrique ;
acide 3-{2-[(2-benzoimidazol-1-yl-éthylcarbamoyl)-méthyl]-3-méthyl-butyrylamino}-4-oxo-butyrique ;
acide 5-(7,7-diméthyl-2-oxo-bicyclo[2.2.1]hept-1-ylméthanesulfonylamino)-3-(2-{[3-(4-hydroxyphényl)-propylcarbamoyl]-méthyl}-3-méthyl-butyrylamino)-4-oxo-pentanoïque ;
acide 5-(7,7-diméthyl-2-oxo-bicyclo[2.2.1]hept-1-ylméthanesulfonylamino)-3-{3-méthyl-2-[(2-pyridin-4-yl-éthylcarbamoyl)-méthyl]-butyrylamino}-4-oxo-pentanoïque ;
acide 5-(7,7-diméthyl-2-oxo-bicyclo[2.2.1]hept-1-ylméthanesulfonylamino)-3-{3-méthyl-2-[(2-naphtalèn-2-yl-éthylcarbamoyl)-méthyl]-butyrylamino}-4-oxo-pentanoïque ;
acide 5-(7,7-diméthyl-2-oxo-bicyclo[2.2.1]hept-1-ylméthanesulfonylamino)-3-{3-méthyl-2-[(3-pyridin-4-yl-propylcarbamoyl)-méthyl]-butyrylamino}-4-oxo-pentanoïque ;
acide 5-(7,7-diméthyl-2-oxo-bicyclo[2.2.1]hept-1-ylméthanesulfonylamino)-3-{3-méthyl-2-[(3-quinolin-2-yl-propylcarbamoyl)-méthyl]-butyrylamino}-4-oxo-pentanoïque ;
acide 5-(7,7-diméthyl-2-oxo-bicyclo[2.2.1]hept-1-ylméthanesulfonylamino)-3-{3-méthyl-2-[(3-naphtalèn-2-yl-propylcarbamoyl)-méthyl]-butyrylamino}-4-oxo-pentanoîque ; ou
acide 5-(7,7-diméthyl-2-oxo-bicyclo[2.2.1]hept-1-ylméthanesulfonylamino)-3-{3-méthyl-2-[(3-pyridin-3-yl-propylcarbamoyl)-méthyl]-butyrylamino}-4-oxo-pentanoïque.

**12.** Composé :

acide 5-(7,7-diméthyl-2-oxo-bicyclo[2.2.1]hept-1-ylméthanesulfonylamino)-3-{3-méthyl-2-[(2-naphtalèn-2-yl-éthylcarbamoyl)-méthyl]-butyrylamino}-4-oxo-pentanoïque ;
acide 5-(7,7-diméthyl-2-oxo-bicyclo[2.2.1]hept-1-ylméthanesulfonylamino)-3-(3-méthyl-2-{[2-(7-méthyl-1*H*-indol-3-yl)-éthylcarbamoyl]-méthyl}-butyrylamino)-4-oxo-pentanoïque ;
acide 5-(7,7-diméthyl-2-oxo-bicyclo[2.2.1]hept-1-ylméthanesulfonylamino)-3-(2-{[2-(6-fluoro-1H-indol-3-yl)-éthylcarbamoyl]-méthyl}-3-méthyl-butyrylamino)-4-oxo-pentanoïque ;
acide 3-[3-méthyl-2-({méthyl-[2-(1-méthyl-1*H*-indol-3-yl)-éthyl]-carbamoyl}-méthyl)-butyrylamino]-4-oxo-butyrique ;
acide 3-(3-méthyl-2-{[méthyl-(2-phénoxy-éthyl)-carbamoyl]-méthyl}-butyrylamino)-4-oxo-butyrique ;
trifluroacétate de l'acide 3-(2-{[2-(5,6-diméthyl-benzoimidazol-1-yl)-éthylcarbamoyl]-méthyl}-3-méthyl-butyrylamino)-4-oxo-butyrique ;
acide 5-(7,7-diméthyl-bicyclo[2.2.1]hept-1-ylméthane sulfonylamino)-3-{3-méthyl-2-[(3-phényl-propylcarbamoyl)-méthyl]-butyrylamino}-4-oxo-pentanoïque ;
acide 5-(7,7-diméthyl-bicyclo[2.2.1]hept-1-ylméthane sulfonylamino)-3-(2-{[2-(1*H*-indol-3-yl)-éthylcarbamoyl]-méthyl}-3-méthyl-butyrylamino)-4-oxo-pentanoïque ;
acide 5-(7,7-diméthyl-2-oxo-bicyclo[2.2.1]hept-1-ylméthanesulfonylamino)-3-{3-méthyl-2-[(3-pyridin-4-yl-propylcarbamoyl)-méthyl]-butyrylamino}-4-oxo-pentanoique ;
acide 5-(7,7-diméthyl-2-oxo-bicyclo[2.2.1]hept-1-ylméthanesulfonylamino)-3-{3-méthyl-2-[(3-quinolin-2-yl-propylcarbamoyl)-méthyl]-butyrylamino}-4-oxo-pentanoique ;
acide 5-(7,7-diméthyl-2-oxo-bicyclo[2.2.1]hept-1-ylméthanesulfonylamino)-3-{3-méthyl-2-[(3-naphtalèn-1-yl-propylcarbamoyl)-méthyl]-butyrylamino}-4-oxo-pentanoique ;
acide 5-(7,7-diméthyl-2-oxo-bicyclo[2.2.1]hept-1-ylméthanesulfonylamino)-3-{3-méthyl-2-[(3-pyridin-3-yl-propylcarbamoyl)-méthyl]-butyrylamino}-4-oxo-pentanoïque ; ou
acide 3-{2-[(2-benzoimidazol-1-yl-éthylcarbamoyl)-méthyl]-3-méthyl-butyrylamino}-5-(7,7-diméthyl-2-oxo-bicyclo[2.2.1]hept-1-ylméthanesulfonylamino)-4-oxo-pentanoïque .

**13.** Composé

acide 5-(7,7-diméthyl-2-oxo-bicyclo[2.2.1]hept-1-ylméthanesulfonylamino)-3-(3-méthyl-2-{[2-(1-méthyl-1*H*-indol-3-yl)-éthylcarbamoyl]-méthyl}-butyrylamino)-4-oxo-pentanoïque ;
acide 5-(7,7-diméthyl-2-oxo-bicyclo[2.2.1]hept-1-ylméthanesulfonylamino)-3-{3-méthyl-2-[(2-pyridin-4-yl-éthylcarbamoyl)-méthyl]-butyrylamino}-4-oxo-pentanoïque ;
acide 3-(2-{[2-(5-acétyl-1*H*-indol-3-yl)-éthyl carbamoyl]-méthyl}-3-méthyl-butyrylamino}-5-(7,7-diméthyl -2-oxo-bicyclo[2.2.1]hept-1-ylméthanesulfonylamino)-4-oxo-pentanoique ;
acide 5-(7,7-diméthyl-2-oxo-bicyclo[2.2.1]hept-1-ylméthanesulfonylamino)-3-(3-méthyl-2-{[2-(1*H*-tétrazol-5-yl)-éthylcarbamoyl]-méthyl}-butyrylamino)-4-oxo-pentanoique ;
$N^4$-(2-benzoimidazol-1-yl-éthyl)-$N^1$-(2-éthoxy-5-oxo-tétrahydrofuran-3-yl)-2-isopropyl-succinamide ;
$N^1$-(2-éthoxy-5-oxo-tétrahydrofuran-3-yl)-$N^4$-[2-(1*H*-indol-3-yl)-éthyl]-2-isopropyl-succinamide ;
$N^1$-(2-éthoxy-5-oxo-tétrahydrofuran-3-yl)-2-isopropyl -$N^4$-[2-(1-méthyl-1*H*-indol-3-yl)-éthyl]-succinamide ;
$N^4$-[2-(5,6-dichloro-benzoimidazol-1-yl)-éthyl]-$N^1$-(2-éthoxy-5-oxo-tétrahydrofuran-3-yl)-2-isopropyl-succinamide ;
$N^1$-(2-éthoxy-5-oxo-tétrahydrofuran-3-yl)-2-isopropyl -$N^4$-[2-phénoxy-éthyl]-succinamide ; ou
$N^1$-(2-éthoxy-5-oxo-tétrahydrofuran-3-yl)-2-isopropyl -$N^4$-[2-(6-méthoxy-1*H*-indol-3-yl)-éthyl]-succinamide.

**14.** Composé :

$N^4$-(2-benzotriazol-1-yl-éthyl)-$N^1$-(2-éthoxy-5-oxo-tétrahydrofuran-3-yl)-2-isopropyl-succinamide ;
$N^1$-(2-éthoxy-5-oxo-tétrahydrofuran-3-yl) -$N^4$-(2-indazol-1-yl-éthyl)-2-isopropyl-succinamide ;
$N^1$-(2-éthoxy-5-oxo-tétrahydrofuran-3-yl)-2-isopropyl -$N^4$-(2-phénylamino-éthyl]-succinamide ;
$N^1$-(2-éthoxy-5-oxo-tétrahydrofuran-3-yl)-$N^4$-[2-(6-fluoro-1*H*-indol-3-yl)-éthyl]-2-isopropyl-succinamide ;
$N^1$-(2-éthoxy-5-oxo-tétrahydrofuran-3-yl)-$N^4$-[2-(7-méthyl-1*H*-indol-3-yl)-éthyl]-2-isopropyl-succinamide ;
$N^1$-(2-éthoxy-5-oxo-tétrahydrofuran-3-yl)-2-isopropyl-$N^4$-[2-(2-méthyl-benzoimidazol-1-yl)-éthyl]-succinamide ;
$N^1$-(2-éthoxy-5-oxo-tétrahydrofuran-3-yl)-2-isopropyl -$N^4$-[3-(3,4,5-triméthoxyphényl)-propyl]-succinamide ;
$N^1$-(2-éthoxy-5-oxo-tétrahydrofuran-3-yl)-2-isopropyl -$N^4$-[2-(phényl)-éthyl]-succinamide ;
$N^1$-(2-éthoxy-5-oxo-tétrahydrofuran-3-yl)-2-isopropyl -$N^4$-[4-(phényl)-butyl]-succinamide ; ou
$N^1$-(2-éthoxy-5-oxo-tétrahydrofuran-3-yl)-$N^4$-[2-indol -1-yl-éthyl)-2-isopropyl-succinamide.

**15.** Composé :

$N^1$-(2-éthoxy-5-oxo-tétrahydrofuran-3-yl)-2-isopropyl -$N^4$-[4-(phényl)-propyl]-succinamide ;
$N^1$-(2-éthoxy-5-oxo-tétrahydrofuran-3-yl)-$N^4$-[2- (5-fluoro-1*H*-indol-3-yl)-éthyl]-2-isopropyl-succinamide ;
acide 3-{2-[(2-benzoimidazol-1-yl-éthylcarbamoyl)-méthyl]-3-méthyl-butyrylamino}-4-oxo-butyrique ;
acide 3-(3-méthyl-2-{[2-(1-méthyl-1*H*-indol-3-yl)-éthylcarbamoyl]-méthyl}-butyrylamino)-4-oxo-butyrique ;
acide 3-(2-{[2-(5-fluoro-1-méthyl-1*H*-indol-3-yl)-éthylcarbamoyl]-méthyl}-3-méthyl-butyrylamino)-4-oxo-butyrique ;
acide 3-(2-{[2-(5,6-dichloro-benzoimidazol-1-yl)-éthylcarbamoyl]-méthyl}-3-méthyl-butyrylamino)-4-oxo-butyrique ;
acide 3-(2-{[(2-benzoimidazol-1-yl-éthyl)-méthyl-carbamoyl)-méthyl}-3-méthyl-butyrylamino)-4-oxo-butyrique ;
acide 3-{2-[(2-benzotriazol-1-yl-éthylcarbamoyl)-méthyl]-3-méthyl-butyrylamino}-4-oxo-butyrique ;
acide 3-{2-[(2-indazol-1-yl-éthylcarbamoyl)-méthyl]-3-méthyl-butyrylamino}-4-oxo-butyrique ;
acide 3-[2-({[2-(5,6-diméthyl-benzoimidazol-1-yl)-éthyl]-méthyl-carbamoyl}-méthyl)-3-méthyl-butyrylamino]-4-oxo-butyrique ;
acide 3-[2-({[2-(2-méthyl-benzoimidazol-1-yl)-éthyl]-méthyl-carbamoyl}-méthyl)-3-méthyl-butyrylamino]-4-oxo-butyrique ;
acide 3-(3-méthyl-2-{[3-(3,4,5-triméthoxy-phényl)-propylcarbamoyl]-méthyl}-butyrylamino)-4-oxo-butyrique ;
ester d'éthyle de l'acide 3-{3-méthyl-2-[(2-phénoxy-éthylcarbamoyl)-méthyl]-butyrylamino}-4-oxo-butyrique ;
ester d'éthyle de l'acide 3-cyano-3-{3-méthyl-2-[(2-phénoxy-éthylcarbamoyl)-méthyl]-butyrylamino}-propionique ; ou
acide 3-cyano-3-{3-méthyl-2-[(2-phénoxy-éthyl carbamoyl)-méthyl]-butyrylamino}-propionique.

**16.** Composition pharmaceutique comprenant un composé selon l'une quelconque des revendications 1 à 15.

**17.** Utilisation d'un composé selon l'une quelconque des revendications 1 à 15 pour la préparation d'une composition pharmaceutique destinée à traiter ou à prévenir l'accident vasculaire cérébral.

**18.** Utilisation d'un composé selon l'une quelconque des revendications 1 à 15 pour la préparation d'une composition pharmaceutique destinée à traiter les maladies inflammatoires.

**19.** Utilisation selon la revendication 18 dans laquelle la maladie inflammatoire est la polyarthrite rhumatoïde ou l'affection abdominale inflammatoire.

**20.** Utilisation d'un composé selon l'une quelconque des revendications 1 à 15 pour la préparation d'une composition pharmaceutique destinée à traiter les chocs infectieux.

**21.** Utilisation d'un composé selon l'une quelconque des revendications 1 à 15 pour la préparation d'une composition pharmaceutique destinée à traiter les blessures dues aux perfusions.

**22.** Utilisation d'un composé selon l'une quelconque des revendications 1 à 15 pour la préparation d'une composition pharmaceutique destinée à traiter la maladie d'Alzheimer.

**23.** Utilisation d'un composé selon l'une quelconque des revendications 1 à 15 pour la préparation d'une composition pharmaceutique destinée à traiter la shigellose.

**24.** Utilisation d'un composé selon l'une quelconque des revendications 1 à 15 pour la préparation d'une composition pharmaceutique destinée à traiter la sclérose en plaques.